(19) Europäisches Patentamt
European Patent Office
Office européen des brevets

(11) Veröffentlichungsnummer : **0 457 023 B1**

(12) **EUROPÄISCHE PATENTSCHRIFT**

(45) Veröffentlichungstag der Patentschrift : **13.07.94 Patentblatt 94/28**

(21) Anmeldenummer : **91105674.5**

(22) Anmeldetag : **10.04.91**

(51) Int. Cl.$^5$ : **C07D 417/12,** C07D 409/12, C07D 413/12, C07D 285/12, C07D 271/10, C07D 261/08, A01N 43/72, A01N 43/48, A01N 43/00

# (54) Hydrochinondiether, ihre Herstellung und ihre Verwendung.

(30) Priorität : **19.04.90 DE 4012409**

(43) Veröffentlichungstag der Anmeldung : **21.11.91 Patentblatt 91/47**

(45) Bekanntmachung des Hinweises auf die Patenterteilung : **13.07.94 Patentblatt 94/28**

(84) Benannte Vertragsstaaten : **AT BE CH DE DK ES FR GB IT LI NL SE**

(56) Entgegenhaltungen :
**EP-A- 0 353 571**
**EP-A- 0 353 674**
**US-A- 4 171 365**
**CHEMICAL ABSTRACTS, Band 77, Nr 5, 31. Juli 1972, Seite 101, Nr. 29615c, Columbus, Ohio, US; A.K. MURTHY et al.: "Synthesis of 3-phenyl-5-aryloxy-methylisoxazoles and their uv spectra and physiological activity" & INDIAN J. CHEM. 1972,10(1), 38-40 & Chemical Abstracts, 9th Collective substance index, 1972-1976, Seite 21233cs, mittlere Spalte, "Isoxatole, 5,5'-1,4-phenylenebis (oxymethylene)bis-3-phenyl-" * Abstract ***

(73) Patentinhaber : **BASF Aktiengesellschaft**
**Carl-Bosch-Strasse 38**
**D-67063 Ludwigshafen (DE)**

(72) Erfinder : **Kardorff, Uwe, Dr.**
**D 3,4**
**W-6800 Mannheim 1 (DE)**
Erfinder : **Nuebling, Christoph, Dr.**
**Wilhelmstrasse 13**
**W-6733 Hassloch (DE)**
Erfinder : **Theobald, Hans, Dr.**
**Queichstrasse 6**
**W-6703 Limburgerhof (DE)**
Erfinder : **Kuekenhoehner, Thomas, Dr.**
**Seidelstrasse 2**
**W-6710 Frankenthal (DE)**
Erfinder : **Neubauer, Hans-Juergen, Dr.**
**Mozartstrasse 6**
**W-4400 Muenster-Hiltrup (DE)**
Erfinder : **Leyendecker, Joachim, Dr.**
**Stahlbuehlring 79**
**W-6802 Ladenburg (DE)**
Erfinder : **Kuenast, Christoph, Dr.**
**Salierstrasse 2**
**W-6701 Otterstadt (DE)**
Erfinder : **Hofmeister, Peter, Dr.**
**Bernard-Humblot-Strasse 12**
**W-6730 Neustadt (DE)**
Erfinder : **Krieg, Wolfgang, Dr.**
**Saarstrasse 17**
**W-6721 Weingarten (DE)**

**EP 0 457 023 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft Hydrochinondiether der allgemeinen Formel I

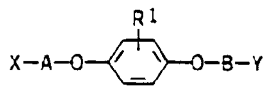

I,

in der die Substituenten folgende Bedeutung haben:

$R^1$ Wasserstoff, Halogen oder $C_1$-$C_6$-Alkyl;

A,B Methylen, Ethylen oder Propylen wobei diese Gruppen ein oder zwei $C_1$-$C_3$-Alkylreste tragen können;

X,Y ein 5-gliedriger Heteroaromat enthaltend neben Kohlenstoffatomen 1 bis 3 Heteroatome aus der Gruppe Stickstoff, Sauerstoff und Schwefel, welcher ein bis drei der folgenden Gruppen tragen kann: Halogen, $C_1$-$C_6$-Alkyl, $C_1$-$C_4$-Halogenalkyl, $C_1$-$C_6$-Alkoxy, $C_1$-$C_4$-Halogenalkoxy, $C_1$-$C_3$-Alkoxy-$C_1$-$C_4$-alkyl, $C_3$-$C_8$-Cycloalkyl, $C_2$-$C_8$-Alkenyl, Aryl oder Aryl-$C_1$-$C_{10}$-alkyl, wobei diese aromatischen Reste ihrerseits ein bis fünf Halogenatome und ein bis drei der folgenden Gruppen tragen können: $C_1$-$C_4$-Alkyl, $C_1$-$C_4$-Halogenalkyl, $C_1$-$C_4$-Alkoxy, $C_1$-$C_4$-Halogenalkoxy oder $C_1$-$C_4$-Alkylthio,

wobei X und Y nicht gleichzeitig für 3-Phenylisoxazol-5-yl stehen, wenn A und B Methylen und $R^1$ Wasserstoff bedeuten und wobei X und Y nicht gleichzeitig für 3,5-Diethylpyrazol-4-yl oder 1-Methyl-3,5-diethylpyrazol-4-yl stehen, wenn A und B Propylen und $R^1$ Wasserstoff bedeuten.

Außerdem betrifft die Erfindung ein Verfahren zur Herstellung der Verbindungen I und Schädlingsbekämpfungsmittel, welche die Verbindungen I enthalten.

Aus der Literatur sind 4-Aryloxyphenolhetarylmethylether mit insektizider Wirkung bekannt (EP-A 239 047, EP-A 287 959, EP-A 289 919, EP-A 353 571, EP-A 353 674).

Außerdem wird in J. Indian. Chem. 10(1), 38-40 (1972) 1,4-bis-[(3-Phenylisoxazol-5-yl)-methoxy]-phenyl als fungizid und antibakteriell wirksam und werden in US-A 4,171,365 1,4-bis-[3-(3,5-Diethylpyrazol-4-yl)-propyloxy]-phenyl und 1,4-bis-[3-(1-Methyl-3,5-diethylpyrazol-4-yl)-propyloxy]-phenyl als antiviral wirksam beschrieben.

Der Erfindung lagen neue Hydrochinondiether mit verbesserten insektiziden Eigenschaften als Aufgabe zugrunde.

Demgemäß wurden die eingangs definierten Hydrochinondiether der Formel I und ein Verfahren zu ihrer Herstellung gefunden. Außerdem wurden Schädlingsbekämpfungsmittel und Verfahren zur Bekämpfung von Schädlingen mit Hydrochinondiethern der allgemeinen Formel IA

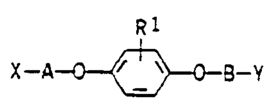

IA ,

in der die Substituenten folgende Bedeutung haben:

$R^1$ Wasserstoff, Halogen oder $C_1$-$C_6$-Alkyl;

A,B Methylen, Ethylen oder Propylen, wobei diese Gruppen ein oder zwei $C_1$-$C_3$-Alkylreste tragen können;

X,Y ein 5-gliedriger Heteroaromat, enthaltend neben Kohlenstoffatomen 1 bis 3 Heteroatome aus der Gruppe Stickstoff, Sauerstoff und Schwefel, welcher eine bis drei der folgenden Gruppen tragen kann: Halogen, $C_1$-$C_6$-Alkyl, $C_1$-$C_4$-Halogenalkyl, $C_1$-$C_6$-Alkoxy, $C_1$-$C_4$-Halogenalkoxy, $C_1$-$C_3$-Alkoxy-$C_1$-$C_4$-alkyl, $C_3$-$C_8$-Cycloalkyl, $C_2$-$C_8$-Alkenyl, Aryl oder Aryl-$C_1$-$C_{10}$-alkyl, wobei diese aromatischen Reste ihrerseits ein bis fünf Halogenatome und ein bis drei der folgenden Gruppen tragen können: $C_1$-$C_4$-Alkyl, $C_1$-$C_4$-Halogenalkyl, $C_1$-$C_4$-Alkoxy, $C_1$-$C_4$-Halogenalkoxy oder $C_1$-$C_4$-Alkylthio,

gefunden.

Die Hydrochinondiether der Formel I sind auf verschiedenen Wegen herstellbar. Man erhält sie beispielsweise nach dem folgenden Verfahren:

Man verethert einen 4-Hydroxyphenolether der allgemeinen Formel II in an sich bekannter Weise in einem inerten organischen Lösungsmittel in Gegenwart einer Base mit einem Hetarylalkylderivat der Formel IIIa oder IIIb, überführt das so erhaltene Dietherderivat IVa bzw. IVb in an sich bekannter Weise durch Abspaltung der Schutzgruppe R in den entsprechenden Monoether Va bzw. Vb, welcher anschließend in an sich bekannter Weise mit einem Hetarylalkylderivat IIIa oder IIIb zu I verethert wird.

Die Reaktionsschritte sind im folgenden Schema dargestellt:

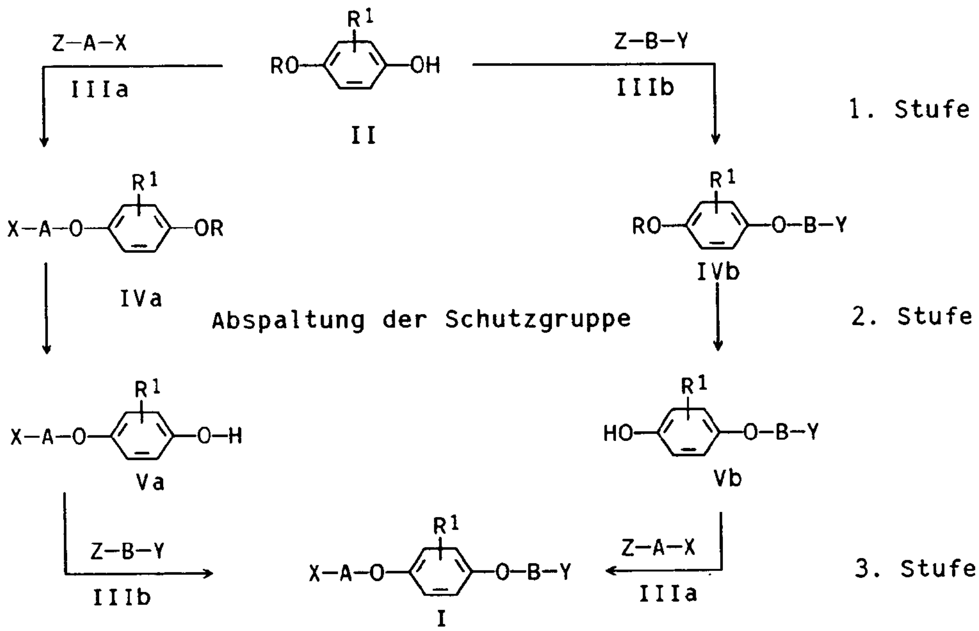


R in Formel II steht für eine unter den Reaktionsbedingungen inerte Schutzgruppe wie verzweigtes oder unverzweigtes $C_1$-$C_6$-Alkyl, wie tert.-Butyl, Benzyl oder Silyl, welches dreifach durch $C_1$-$C_4$-Alkyl oder Aryl substituiert ist, wie Trimethylsilyl, tert.-Butyl-dimethylsilyl, Phenyl-dimethylsilyl und tert.-Butyldiphenylsilyl, insbesondere tert.-Butyl-dimethylsilyl.

Z in den Formeln IIIa und IIIb bedeutet eine nucleofuge Abgangsgruppe, beispielsweise Sulfonylreste wie Methylsulfonyl, Trifluormethylsulfonyl, Phenylsulfonyl und p-Tolylsulfonyl oder Halogen wie Chlor, Brom und Iod, insbesondere Chlor und Brom.

Die Veretherungen von II mit IIIa oder IIIb (1. Stufe) bzw. von Va oder Vb mit IIIb bzw. IIIa (3. Stufe) werden im allgemeinen in einem inerten organischen Lösungsmittel in Gegenwart einer Base bei Temperaturen von -50°C bis 150°C, insbesondere -20°C bis 120°C durchgeführt.

Die Umsetzungen verlaufen gewöhnlich oberhalb von -20°C mit ausreichenden Geschwindigkeiten. 120°C müssen im allgemeinen nicht überschritten werden. Da die Umsetzungen in einigen Fällen unter Wärmeentwicklung verlaufen, kann es von Vorteil sein, Kühlmöglichkeiten vorzusehen.

Als Lösungsmittel eignen sich beispielsweise Kohlenwasserstoffe wie Pentan, Hexan, Benzol, Toluol und Xylole; halogenierte Kohlenwasserstoffe wie Methylenchlorid, Ethylenchlorid, Chloroform und Chlorbenzol; Ether wie Diethyl-, Di-n-butylether, Methyl-tert.-butylether, Tetrahydrofuran und Dioxan; Ketone wie Aceton und Methylethylketon; Nitrile wie Acetonitril; Alkohole wie Methanol und Ethanol; aprotische dipoloare Lösungsmittel wie Dimethylformamid, Dimethylsulfoxid und Pyridin, insbesondere Acetonitril, Ethanol, Dimethylformamid und entsprechende Gemische.

Man verwendet normalerweise mindestens äquivalente Mengen einer Base, kann diese aber auch im überschuß oder gegebenenfalls als Lösungsmittel einsetzen. Als Basen eignen sich beispielsweise Hydroxide von Alkali- und Erdalkalimetallen wie Natriumhydroxid, Kaliumhydroxid und Calciumhydroxid. Alkoholate von Alkali- und Erdalkalimetallen wie Natriummethylat, Natriumethylat, Calciummethanolat oder Kalium-tert.-butylat; Alkali- oder Erdalkalimetallhydride wie Natriumhydrid, Kaliumhydrid oder Calciumhydrid; Alkali- oder Erdalkalicarbonate wie Natriumcarbonat, Kaliumcaronat oder Calciumcarbonat; aliphatische Amine wie Dimethylamin, Triethylamin oder Diisopropylamin; heterocyclische Amine wie Piperidin, Piperazin oder Pyrrolidin; aromatische Amine wie Pyridin oder Pyrrol und gegebenenfalls auch Alkyllithium-Verbindungen wie n-Butyllithium, insbesondere Kaliumhydroxid, Kaliumcarbonat, Natriummethylat, Natriumethylat, Kalium-tert.-butylat und Natriumhydrid.

Die Ausgangsstoffe werden üblicherweise in stöchiometrischen Mengen miteinander umgesetzt, zur Steigerung der Ausbeute kann es jedoch vorteilhaft sein, einen der Ausgangsstoffe in einem Überschuß von 0,1 bis 10 mol-Äq., insbesondere 0,2 bis 1,5 mol-Äq. einzusetzen. Außerdem kann es von Vorteil sein, 5 bis 20 mol.-% Kaliumjodid zuzugeben.

Die Reaktionsgemische werden in üblicher Weise aufgearbeitet, z.B. durch Versetzen mit Wasser, Trennen der Phasen und Reinigung der Rohprodukte mittels Säulenchromatographie. Die neuen Verbindungen der Formel I fallen teilweise in Form farbloser oder schwach bräunlich gefärbter, zäher Öle an, die sich durch längeres Erwärmen unter vermindertem Druck auf mäßig erhöhte Temperatur ("Andestillieren") von den letzten flüchtigen Anteilen befreien und auf diese Weise reinigen lassen. Erhält man die Verbindungen der Formel IVa, IVb bzw. I in kristalliner Form, so kann ihre Reinigung durch Umkristallisieren erfolgen.

Die Abspaltung der Schutzgruppe R aus den Diethern IVa bzw. IVb erfolgt ebenfalls in an sich bekannter Weise in einem inerten organischen Lösungsmittel in Gegenwart einer Säure bei Temperaturen von -20°C bis 120°C, vorzugsweise 20 bis 100°C.

Im allgemeinen eignen sich hierfür Lösungsmittel wie vorstehend genannt, insbesondere Methanol, Ethanol, Chloroform und Dioxan und entsprechende Gemische.

Als Säuren eignen sich anorganische Säuren wie Fluorwasserstoffsäure, Salzsäure, Bromwasserstoffsäure, Schwefelsäure, Perchlorsäure und organische Säuren wie Ameisensäure, Essigsäure, Propionsäure, Oxalsäure, Zitronensäure und Trifluoressigsäure, insbesondere Salzsäure, Bromwasserstoffsäure und Trifluoressigsäure.

Die Säuren werden hierbei im allgemeinen in katalytischen Mengen zugesetzt, sie können aber auch äquimolar, im Überschuß oder ggf. als Lösungsmittel eingesetzt werden.

Die Reaktionsgemische werden in üblicher Weise, beispielsweise wie vorstehend für die Veretherung beschrieben, aufgearbeitet.

Symmetrische Hydrochinondiether I (A-X=B-Y) sind besonders vorteilhaft in einstufiger Reaktion durch Veretherung von gegebenenfalls substituierten Hydrochinonen gemäß den vorstehend beschriebenen Veretherungsmethoden zugänglich.

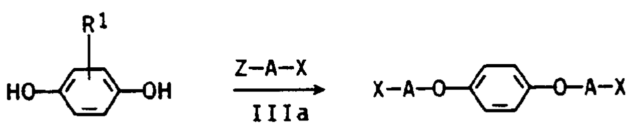


I (A–X=B–Y)

Die für die Umsetzung benötigten Hetarylalkylderivate der Formeln IIIa und IIIb sind entweder bekannt, kommerziell erhältlich oder lassen sich nach allgemein bekannten chemischen Verfahren herstellen.

Verfahren zur Herstellung von Thiophenderivaten finden sich beispielsweise in: Comprehensive Heterocyclic Chemistry, R. Katritzky und W. Rees, Vol. 4, S. 863ff, Pergamon Press 1984; Thiazolderivate, Oxazolderivate; Isothiazolderivate, Thiadiazolderivate und Oxadiazolderivate z.B. in: Comprehensive Heterocyclic Chemistry, R. Katritzky und W. Rees, Vol. 6, S. 131, 177, 235, 365, 427, 545ff, Pergamon Press 1984;
Imidazolderivate z.B. in: Advances in Heterocyclic Chemistry, Vol. 27, S. 242 ff, 1980, Pyrazol- und Triazolderivate z.B. in Comprehensive Heterocyclic Chemistry, R. Katritzky und W. Rees, Vol. 5, S. 167, 733 ff, Pergamon Press 1984;
Isoxazolderivate z.B. in DE-A-25 49 962 und DE-A-27 54 832;
N-Methylazole sind zum Teil aus Heterocycles 24, 2233-2237 (1986) bekannt oder können nach der dort beschriebenen Methode durch Umsetzung des Azols mit para-Formaldehyd erhalten werden.

Im Hinblick auf die biologische Wirkung der Hydrochinondiether der Formel I kommen als Substituenten folgende Reste in Betracht:

$R^1$ Wasserstoff;
Halogen wie Fluor, Chlor, Brom und Iod, insbesondere Fluor;
Alkyl wie Methyl, Ethyl, Propyl, 1-Methylethyl, Butyl, 1-Methylpropyl, 2-Methylpropyl, 1, 1-Dimethylethyl, Pentyl, 1-Methylbutyl, 2-Methylbutyl, 3-Methylbutyl, 1, 1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1, 1-Dimethylbutyl, 1, 2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, vorzugsweise $C_1$-$C_4$-Alkyl, insbesondere Methyl, Ethyl und Isopropyl;

A,B unabhängig voneinander Methylen, Ethylen oder Propylen, wobei diese Gruppen ein oder zwei Alkylreste mit 1 bis 3 Kohlenstoffatomen wie vorstehend bei $R^1$ genannt, insbesondere einen Methyl- oder Ethylrest tragen können;

X,Y unabhängig voneinander 5-gliedrige Heteroaromaten, enthaltend neben Kohlenstoffatomen 1 bis 3 Heteroatome aus der Gruppe Stickstoff, Sauerstoff und Schwefel wie Furanyl, Thienyl, Pyrrolyl, Isoxazolyl, Isothiazolyl, Pyrazolyl, Oxazolyl, Thiazolyl, Imidazolyl, Oxa-2,4-diazolyl, Oxa-3,4-diazolyl, Thia-2,4-diazolyl, Thia-3,4-diazolyl und

Triazolyl, wobei diese Ringe über ein Kohlenstoffringglied oder sofern vorhanden ein Stickstoffringglied an den Rest A bzw. B gebunden sein können; diese Heteroaromaten können außerdem an den Kohlenstoff- und soweit vorhanden valenten Stickstoffringgliedern eine bis drei der folgenden Gruppen tragen:
Alkyl wie bei $R^1$ genannt, bevorzugt $C_1$-$C_4$-Alkyl, insbesondere Methyl, Ethyl und 1-Methylethyl;
Halogenalkyl wie Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl, insbesondere Trifluormethyl und Trichlormethyl;
Alkoxy wie Methoxy, Ethoxy, n-Propoxy, 2-Methylethoxy, n-Butoxy, 1-Methylpropoxy, 2-Methylpropoxy, 1,1-Dimethylethoxy, Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexyloxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethlbutoxy, 2,3-Dimethylbutoxy, 3,3-Dimethylbutoxy, 1-Ethylbutoxy, 1,1,2-Trimethylpropoxy, 1,2,2-Trimethylpropoxy, 1-Ethyl-1-methylpropoxy und 1-Ethyl-2-methylpropoxy, vorzugsweise $C_1$-$C_4$-Alkoxy, insbesondere Methoxy, Ethoxy und 1-Methylethoxy;
Halogenalkoxy wie Difluormethoxy, Trifluormethoxy, Chlor-difluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy und Pentafluorethoxy;
Alkoxyalkyl wie Methoxymethyl, Methoxyethyl, 2-Methoxyethyl, 1-Methoxypropyl, 2-Methoxypropyl, 1-Methoxy-1-methylethyl, 2-Methoxy-1-methylethyl, 1-Methoxybutyl, 2-Methoxybutyl, 1-Methoxy-1-methylpropyl, 1-Methoxy-2-methylpropyl, 2-Methoxy-1-methylpropyl, 2-Methoxy-2-methylpropyl, Ethoxymethyl, 1-Ethoxyethyl, 2-Ethoxyethyl, 1-Ethoxypropyl, 2-Ethoxypropyl, 1-Ethoxy-1-methylethyl, 2-Ethoxy-1-methylethyl, 1-Ethoxybutyl, 2-Ethoxybutyl, 1-Ethoxy-1-methylpropyl, 1-Ethoxy-2-methylpropyl, 2-Ethoxy-1-methylpropyl und 2-Ethoxy-2-methylpropyl, insbesondere Methoxymethyl und Ethoxymethyl;
Cycloalkyl wie Cyclopropyl, Cyclobutyl, Cyclopentyl, Cyclohexyl, Cycloheptyl und Cyclooctyl, vorzugsweise Cyclopropyl, Cyclopentyl und Cyclohexyl und insbesondere Cyclopropyl;
Alkenyl wie insbesondere Ethenyl, 1-Propenyl, 2-Propenyl, 2-Butenyl, 1-Methyl-2-butenyl, 2-Methyl-2-butenyl, 3-Methyl-2-butenyl, 2-Pentenyl, 3-Pentenyl und 1,2-Dimethyl-2-propenyl, besonders bevorzugt Ethenyl, 2-Propenyl, 2-Butenyl und 3-Methyl-2-butenyl;
Aryl wie Phenyl, 1-Naphthyl und 2-Naphthyl, vorzugsweise Phenyl;
Arylalkyl wie durch Phenyl, 1-Naphthyl oder 2-Naphthyl substituiertes $C_1$-$C_{10}$-Alkyl, vorzugsweise $C_1$-$C_6$-Alkyl wie bei $R^1$ genannt, insbesondere durch Phenyl substituiertes $C_1$-$C_4$-Alkyl wie Benzyl, 1-Phenylethyl, 2-Phenylethyl, 1-Phenylpropyl, 2-Phenylpropyl, 1-Methyl-1-phenylethyl, 1-Methyl-2-phenylethyl und 1,1-Dimethyl-2-phenylethyl, wobei die aromatischen Reste ihrerseits ein bis fünf Halogenatome wie bei $R^1$ genannt, insbesondere Fluor und/oder Chlor und/oder ein bis drei der folgenden Gruppen tragen können:
$C_1$-$C_4$-Alkyl wei bei $R^1$ genannt, insbesondere Methyl, Ethyl, 1-Methylethyl und 1,1-Dimethylethyl;
$C_1$-$C_4$-Halogenalkyl wie im allgemeinen und im besonderen vorstehend genannt;
$C_1$-$C_4$-Alkoxy wie vorstehend genannt, insbesondere Methoxy, Ethoxy und 1-Methylethoxy;
$C_1$-$C_4$-Halogenalkoxy wie im allgemeinen und im besonderen vorstehend genannt
$C_2$-$C_4$-Alkenyl wie im allgemeinen und im besonderen vorstehend genannt und/oder
Alkylthio wie Methylthio, Ethylthio, Propylthio, 1-Methylethylthio, Butylthio, 1-Methylpropylthio, 2-Methylpropylthio und 1,1-Dimethylethylthio, insbesondere Methylthio;
neben den vorstehend genannten Gruppen können die 5-gliedrigen Heteroarylreste (X,Y) an den Kohlenstoffringgliedern auch ein bis drei Halogenatome wie Fluor, Chlor, Brom und Iod, insbesondere Fluor, Chlor und Brom tragen.

Als Substituent X bzw. Y insbesondere bevorzugte 5-gliedrige Heteroaromaten sind nachfolgend zusammengestellt, wobei die möglichen Substituenten der Übersichtlichkeit wegen nicht eingezeichnet sind; die Bindung zu A bzw. B ist als -- bezeichnet.

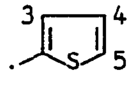

X/Y-1

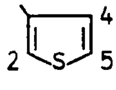

X/Y-2

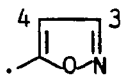
X/Y-3

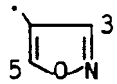
X/Y-4

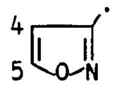
X/Y-5

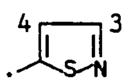
X/Y-6

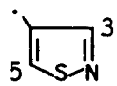
X/Y-7

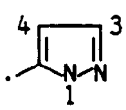
X/Y-8

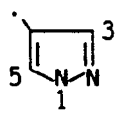
X/Y-9

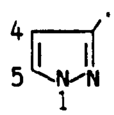
X/Y-10

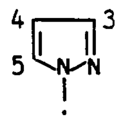
X/Y-11

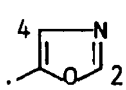
X/Y-12

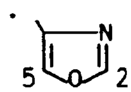
X/Y-13

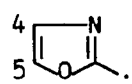
X/Y-14

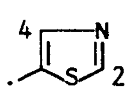
X/Y-15

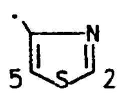
X/Y-16

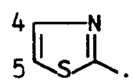
X/Y-17

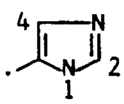
X/Y-18

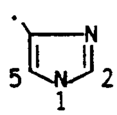
X/Y-19

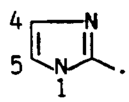
X/Y-20

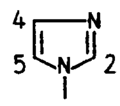
X/Y-21

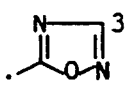
X/Y-22

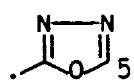
X/Y-23

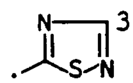
X/Y-24

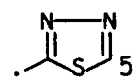
X/Y-25

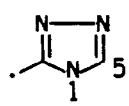
X/Y-26

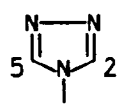
X/Y-27

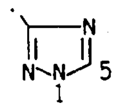
X/Y-28

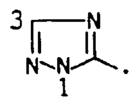
X/Y-29

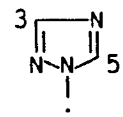
X/Y-30

Beispiele für die insbesondere bevorzugten Verbindungen der allgemeinen Formeln I.A, I.B, I.C, I.D, I.E, I.F und I.G sind in den nachstehenden Tabellen A bis G wiedergegeben.

I.A

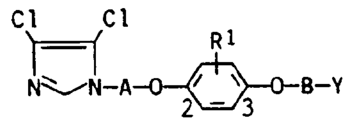

I.B

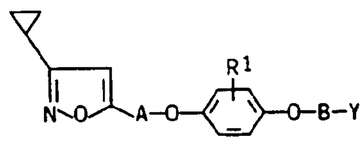

I.C

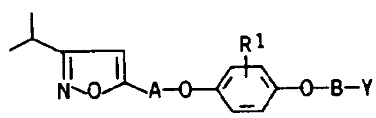

I.D

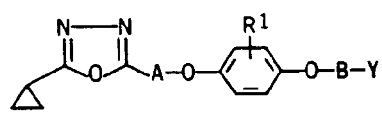

I.E

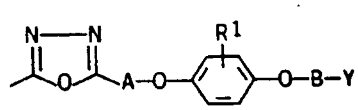

I.F

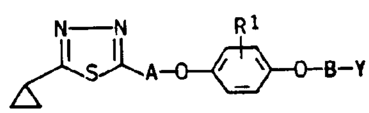

I.G

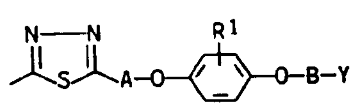

Tabelle A (zu Struktur I.A).

| A | R1 | B | Y | Substituent an Y |
|---|---|---|---|---|
| $CH_2$ | H | $CH_2$ | X/Y-1 | |
| $CH(CH_3)$ | H | $CH_2$ | X/Y-1 | |
| $CH(CH_2CH_3)$ | H | $CH_2$ | X/Y-1 | |
| $CH_2$ | H | $CH(CH_3)$ | X/Y-1 | |
| $CH_2$ | H | $CH(CH_2CH_3)$ | X/Y-1 | |
| $CH_2$ | 3-$CH_3$ | $CH_2$ | X/Y-1 | |
| $CH_2$ | 3-F | $CH_2$ | X/Y-1 | |
| $CH_2$ | H | $CH_2$ | X/Y-1 | 5-$CH_3$ |
| $CH_2$ | H | $CH_2$ | X/Y-1 | 4-$CH_3$ |
| $CH_2$ | H | $CH_2$ | X/Y-1 | 3-$CH_3$ |
| $CH_2$ | H | $CH_2$ | X/Y-1 | 5-Cyclopropyl |
| $CH_2$ | H | $CH_2$ | X/Y-1 | 4-Cyclopropyl |
| $CH_2$ | H | $CH_2$ | X/Y-1 | 3-Cyclopropyl |
| $CH_2$ | H | $CH_2$ | X/Y-1 | 5-Cl |
| $CH_2$ | H | $CH_2$ | X/Y-1 | 5-Br |
| $CH_2$ | H | $CH_2$ | X/Y-1 | 4-Cl |
| $CH_2$ | H | $CH_2$ | X/Y-1 | 4-Br |
| $CH_2$ | H | $CH_2$ | X/Y-1 | 4,5-Cl,Cl |
| $CH_2$ | H | $CH_2$ | X/Y-1 | 4-$OCH_2CH_3$ |
| $CH(CH_3)$ | H | $CH_2$ | X/Y-1 | 5-Br |
| $CH(CH_3)$ | H | $CH_2$ | X/Y-1 | 4,5-Cl,Cl |
| $CH(CH_3)$ | H | $CH_2$ | X/Y-1 | 4-Cl |
| $CH_2$ | H | $CH(CH_3)$ | X/Y-1 | 5-Br |
| $CH_2$ | H | $CH(CH_3)$ | X/Y-1 | 4,5-Cl,Cl |
| $CH(CH_3)$ | H | $CH(CH_3)$ | X/Y-1 | – |
| $CH(CH_3$ | H | $CH(CH_3)$ | X/Y-1 | 4,5-Cl,Cl |
| $CH(CH_3)$ | 3-F | $CH(CH_3)$ | X/Y-1 | – |
| $CH(CH_3)$ | 3-F | $CH(CH_3)$ | X/Y-1 | 5-Br |
| $CH_2CH_2$ | H | $CH_2$ | X/Y-1 | 5-Br |
| $CH_2CH_2$ | H | $CH_2CH_2$ | X/Y-1 | 4,5-Cl,Cl |
| $CH_2CH_2CH_2$ | H | $CH_2$ | X/Y-1 | 5-Br |
| $CH_2CH_2$ | H | $CH_2CH_2CH_2$ | X/Y-1 | 4,5-Cl,Cl |
| $CH_2CH(CH_3)$ | H | $CH_2CH_2$ | X/Y-1 | 4,5-Cl,Cl |
| $CH_2CH_2$ | 3-F | $CH_2CH(CH_3)$ | X/Y-1 | 5-Br |

Tabelle A (Forts.)

| A | $R^1$ | B | Y | Substituent an Y |
|---|---|---|---|---|
| $CH_2$ | H | $CH_2$ | X/Y-2 | - |
| $CH_2$ | H | $CH_2$ | X/Y-2 | 4-Cyclopropyl |
| $CH_2$ | H | $CH_2$ | X/Y-2 | 5-Cyclopropyl |
| $CH_2$ | H | $CH_2$ | X/Y-2 | 5-Cl |
| $CH_2$ | H | $CH_2$ | X/Y-2 | 4,5-Cl,Cl |
| $CH_2$ | H | $CH_2$ | X/Y-2 | 4,5-Br,Br |
| $CH_2$ | H | $CH_2$ | X/Y-2 | 4-$CH_3$ |
| $CH_2$ | H | $CH_2$ | X/Y-2 | 5-$CH_3$ |
| $CH_2$ | H | $CH_2$ | X/Y-2 | 4-$OCH_3$ |
| $CH_2$ | H | $CH_2$ | X/Y-2 | 5-$OCH_3$ |
| $CH(CH_3)$ | H | $CH_2$ | X/Y-2 | 4,5-Cl,Cl |
| $CH(CH_3)$ | H | $CH_2$ | X/Y-2 | 4,5-Br,Br |
| $CH_2$ | H | $CH(CH_3)$ | X/Y-2 | 4,5-Cl,Cl |
| $CH_2$ | H | $CH(CH_3)$ | X/Y-2 | 4,5-Br,Br |
| $CH(CH_3)$ | H | $CH(CH_3)$ | X/Y-2 | 4,5-Cl,Cl |
| $CH(CH_3)$ | H | $CH(CH_3)$ | X/Y-2 | 4,5-Br,Br |
| $CH(CH_3)$ | 3-F | $CH(CH_3)$ | X/Y-2 | 4,5-Cl,Cl |
| $CH(CH_3)$ | 3-F | $CH(CH_3)$ | X/Y-2 | 4,5-Br,Br |
| $CH_2CH_2$ | H | $CH_2$ | X/Y-2 | 4,5-Cl,Cl |
| $CH_2CH_2$ | H | $CH_2CH_2$ | X/Y-2 | 4,5-Br,Br |
| $CH_2CH_2CH_2$ | H | $CH_2$ | X/Y-2 | 4,5-Cl,Cl |
| $CH_2CH_2$ | H | $CH_2CH_2CH_2$ | X/Y-2 | 4,5-Br,Br |
| $CH_2CH(CH_3)$ | H | $CH_2CH_2$ | X/Y-2 | 4,5-Br,Br |
| $CH_2CH_2$ | 3-F | $CH_2CH(CH_3)$ | X/Y-2 | 4,5-Cl,Cl |
| $CH_2$ | H | $CH_2$ | X/Y-15 | - |
| $CH_2$ | H | $CH_2$ | X/Y-15 | 2-$CH_3$ |
| $CH_2$ | H | $CH_2$ | X/Y-15 | 2-Cyclopropyl |
| $CH_2$ | H | $CH_2$ | X/Y-15 | 2-$OCH_2CH_3$ |
| $CH_2$ | H | $CH_2$ | X/Y-15 | 2-Cl |
| $CH(CH_3)$ | H | $CH_2$ | X/Y-15 | 2-Cyclopropyl |
| $CH(CH_3)$ | H | $CH_2$ | X/Y-15 | 2-$OCH_2CH_3$ |
| $CH_2$ | H | $CH(CH_3)$ | X/Y-15 | 2-Cyclopropyl |
| $CH_2$ | H | $CH(CH_3)$ | X/Y-15 | 2-$OCH_2CH_3$ |
| $CH(CH_3)$ | H | $CH(CH_3)$ | X/Y-15 | 2-Cyclopropyl |
| $CH(CH_3)$ | H | $CH(CH_3)$ | X/Y-15 | 2-$OCH_2CH_3$ |

Tabelle A (Forts.)

| A | $R^1$ | B | Y | Substituent an Y |
|---|---|---|---|---|
| $CH(CH_3)$ | 3-F | $CH(CH_3)$ | X/Y-15 | 2-Cyclopropyl |
| $CH(CH_3)$ | 3-F | $CH(CH_3)$ | X/Y-15 | 2-$OCH_2CH_3$ |
| $CH_2CH_2$ | H | $CH_2$ | X/Y-15 | 2-Cyclopropyl |
| $CH_2CH_2$ | H | $CH_2CH_2$ | X/Y-15 | 2-$OCH_2CH_3$ |
| $CH_2CH_2CH_2$ | H | $CH_2$ | X/Y-15 | 2-Cyclopropyl |
| $CH_2CH_2$ | H | $CH_2CH_2CH_2$ | X/Y-15 | 2-$OCH_2CH_3$ |
| $CH_2CH(CH_3)$ | H | $CH_2CH_2$ | X/Y-15 | 2-Cyclopropyl |
| $CH_2CH_2$ | 3-F | $CH_2CH(CH_3)$ | X/Y-15 | 2-$OCH_2CH_3$ |
| $CH_2$ | H | $CH_2$ | X/Y-12 | - |
| $CH_2$ | H | $CH_2$ | X/Y-12 | 2-$CH_3$ |
| $CH_2$ | H | $CH_2$ | X/Y-12 | 2-Cyclopropyl |
| $CH_2$ | H | $CH_2$ | X/Y-12 | 2-$OCH_2CH_3$ |
| $CH(CH_3)$ | H | $CH_2$ | X/Y-12 | 2-Cyclopropyl |
| $CH(CH_3)$ | H | $CH_2$ | X/Y-12 | 2-$OCH_2CH_3$ |
| $CH_2$ | H | $CH(CH_3)$ | X/Y-12 | 2-Cyclopropyl |
| $CH_2$ | H | $CH(CH_3)$ | X/Y-12 | 2-$OCH_2CH_3$ |
| $CH(CH_3)$ | H | $CH(CH_3)$ | X/Y-12 | 2-Cyclopropyl |
| $CH(CH_3)$ | 3-F | $CH(CH_3)$ | X/Y-12 | 2-$OCH_2CH_3$ |
| $CH_2CH_2$ | H | $CH_2$ | X/Y-12 | 2-Cyclopropyl |
| $CH_2CH_2$ | H | $CH_2CH_2$ | X/Y-12 | 2-$OCH_2CH_3$ |
| $CH_2CH_2CH_2$ | H | $CH_2$ | X/Y-12 | 2-Cyclopropyl |
| $CH_2CH_2$ | H | $CH_2CH_2CH_2$ | X/Y-12 | 2-$OCH_2CH_3$ |
| $CH_2CH(CH_3)$ | H | $CH_2CH_2$ | X/Y-12 | 2-Cyclopropyl |
| $CH_2CH_2$ | 3-F | $CH_2CH(CH_3)$ | X/Y-12 | 2-Cyclopropyl |
| $CH_2$ | H | $CH_2$ | X/Y-18 | 1-$CH_3$ |
| $CH_2$ | H | $CH_2$ | X/Y-18 | 1,2-$(CH_3)_2$ |
| $CH_2$ | H | $CH_2$ | X/Y-18 | 1,4-$(CH_3)_2$ |
| $CH_2$ | H | $CH_2$ | X/Y-18 | 1,2,4-$(CH_3)_2$ |
| $CH_2$ | H | $CH_2$ | X/Y-18 | 1-$CH_3$, 2-Cyclopropyl |
| $CH(CH_3)$ | H | $CH_2$ | X/Y-18 | 1-$CH_3$, 2-Cyclopropyl |
| $CH(CH_3)$ | H | $CH_2$ | X/Y-18 | 1,2,4-$(CH_3)_3$ |
| $CH_2$ | H | $CH(CH_3)$ | X/Y-18 | 1-$CH_3$, 2-Cyclopropyl |
| $CH_2$ | H | $CH(CH_3)$ | X/Y-18 | 1,2,4-$(CH_3)_3$ |
| $CH(CH_3)$ | H | $CH(CH_3)$ | X/Y-18 | 1-$CH_3$, 2-Cyclopropyl |
| $CH(CH_3)$ | 3-F | $CH(CH_3)$ | X/Y-18 | 1,2,4-$(CH_3)_3$ |

Tabelle A (Forts.)

| A | $R^1$ | B | Y | Substituent an Y |
|---|---|---|---|---|
| $CH_2CH_2$ | H | $CH_2$ | X/Y-18 | 1-$CH_3$, 2-Cyclopropyl |
| $CH_2CH_2$ | H | $CH_2CH_2$ | X/Y-18 | 1,2,4-$(CH_3)_2$ |
| $CH_2CH_2CH_2$ | H | $CH_2$ | X/Y-18 | 1-$CH_3$, 2-Cyclopropyl |
| $CH_2CH_2$ | H | $CH_2CH_2CH_2$ | X/Y-18 | 1,2,4-$(CH_3)_2$ |
| $CH_2CH(CH_3)$ | H | $CH_2CH_2$ | X/Y-18 | 1-$CH_3$, 2-Cyclopropyl |
| $CH_2CH_2$ | 3-F | $CH_2CH(CH_3)$ | X/Y-18 | 1,2,4-$(CH_3)_2$ |
| $CH_2$ | H | $CH_2$ | X/Y-19 | 1-$CH_3$ |
| $CH_2$ | H | $CH_2$ | X/Y-19 | 1,2-$(CH_3)_2$ |
| $CH_2$ | H | $CH_2$ | X/Y-19 | 1,5-$(CH_3)_2$ |
| $CH_2$ | H | $CH_2$ | X/Y-19 | 1-$CH_3$, 2-Cyclopropyl |
| $CH(CH_3)$ | H | $CH_2$ | X/Y-19 | 1,2-$(CH_3)_2$ |
| $CH(CH_3)$ | H | $CH_2$ | X/Y-19 | 1-$CH_3$, 2-Cyclopropyl |
| $CH_2$ | H | $CH(CH_3)$ | X/Y-19 | 1,2-$(CH_3)_2$ |
| $CH_2$ | H | $CH(CH_3)$ | X/Y-19 | 1-$CH_3$, 2-Cyclopropyl |
| $CH(CH_3)$ | H | $CH(CH_3)$ | X/Y-19 | 1,2-$(CH_3)_2$ |
| $CH(CH_3)$ | H | $CH(CH_3)$ | X/Y-19 | 1-$CH_3$, 2-Cyclopropyl |
| $CH(CH_3)$ | 3-F | $CH_2$ | X/Y-19 | 1-$CH_3$, 2-Cyclopropyl |
| $CH_2CH_2$ | H | $CH_2$ | X/Y-19 | 1-$CH_3$, 2-Cyclopropyl |
| $CH_2CH_2$ | H | $CH_2CH_2$ | X/Y-19 | 1,2-$(CH_3)_2$ |
| $CH_2CH_2CH_2$ | H | $CH_2$ | X/Y-19 | 1-$CH_3$, 2-Cyclopropyl |
| $CH_2CH_2$ | H | $CH_2CH_2CH_2$ | X/Y-19 | 1,2-$(CH_3)_2$ |
| $CH_2CH(CH_3)$ | H | $CH_2CH_2$ | X/Y-19 | 1-$CH_3$, 2-Cyclopropyl |
| $CH_2CH_2$ | 3-F | $CH_2CH(CH_3)$ | X/Y-19 | 1,2-$(CH_3)_2$ |
| $CH_2$ | H | $CH_2$ | X/Y-16 | – |
| $CH_2$ | H | $CH_2$ | X/Y-16 | 2-$CH_3$ |
| $CH_2$ | H | $CH_2$ | X/Y-16 | 2-Cyclopropyl |
| $CH_2$ | H | $CH_2$ | X/Y-16 | 2-Cl |
| $CH_2$ | H | $CH_2$ | X/Y-16 | 2-$OCH_2CH_3$ |
| $CH(CH_3)$ | H | $CH_2$ | X/Y-16 | 2-$CH_3$ |
| $CH(CH_3)$ | H | $CH_2$ | X/Y-16 | 2-Cyclopropyl |
| $CH_2$ | H | $CH(CH_3)$ | X/Y-16 | 2-$CH_3$ |
| $CH_2$ | H | $CH(CH_3)$ | X/Y-16 | 2-Cyclopropyl |
| $CH_2(CH_3)$ | H | $CH(CH_3)$ | X/Y-16 | 2-$CH_3$ |
| $CH_2(CH_3)$ | H | $CH(CH_3)$ | X/Y-16 | 2-Cyclopropyl |
| $CH_2(CH_3)$ | 3-F | $CH(CH_3)$ | X/Y-16 | 2-Cyclopropyl |

Tabelle A (Forts.)

| A | $R^1$ | B | Y | Substituent an Y |
|---|---|---|---|---|
| $CH_2CH_2$ | H | $CH_2$ | X/Y-16 | 2-Cyclopropyl |
| $CH_2CH_2$ | H | $CH_2CH_2$ | X/Y-16 | 2-$CH_3$ |
| $CH_2CH_2CH_2$ | H | $CH_2$ | X/Y-16 | 2-Cyclopropyl |
| $CH_2CH_2$ | H | $CH_2CH_2CH_2$ | X/Y-16 | 2-$CH_3$ |
| $CH_2CH(CH_3)$ | H | $CH_2CH_2$ | X/Y-16 | 2-Cyclopropyl |
| $CH_2CH_2$ | 3-F | $CH_2CH(CH_3)$ | X/Y-16 | 2-$CH_3$ |
| $CH_2$ | H | $CH_2$ | X/Y-13 | – |
| $CH_2$ | H | $CH_2$ | X/Y-13 | 2-$CH_3$ |
| $CH_2$ | H | $CH_2$ | X/Y-13 | 2-Cyclopropyl |
| $CH_2$ | H | $CH_2$ | X/Y-13 | 2-$OCH_2CH_3$ |
| $CH(CH_3)$ | H | $CH_2$ | X/Y-13 | 2-$OCH_2CH_3$ |
| $CH(CH_3)$ | H | $CH_2$ | X/Y-13 | 2-Cyclopropyl |
| $CH_2$ | H | $CH(CH_3)$ | X/Y-13 | 2-$OCH_2CH_3$ |
| $CH_2$ | H | $CH(CH_3)$ | X/Y-13 | 2-Cyclopropyl |
| $CH(CH_3)$ | H | $CH(CH_3)$ | X/Y-13 | 2-$OCH_2CH_3$ |
| $CH(CH_3)$ | 3-F | $CH(CH_3)$ | X/Y-13 | 2-Cyclopropyl |
| $CH_2CH_2$ | H | $CH_2$ | X/Y-13 | 2-Cyclopropyl |
| $CH_2CH_2$ | H | $CH_2CH_2$ | X/Y-13 | 2-$CH_3$ |
| $CH_2CH_2CH_2$ | H | $CH_2$ | X/Y-13 | 2-Cyclopropyl |
| $CH_2CH_2$ | H | $CH_2CH_2CH_2$ | X/Y-13 | 2-$CH_3$ |
| $CH(CH_3)CH_2$ | 3-F | $CH_2CH_2$ | X/Y-13 | 2-Cyclopropyl |
| $CH_2CH_2$ | H | $CH(CH_3)CH_2$ | X/Y-13 | 2-$CH_3$ |
| $CH_2$ | H | $CH_2$ | X/Y-6 | – |
| $CH_2$ | H | $CH_2$ | X/Y-6 | 3-$CH_3$ |
| $CH_2$ | H | $CH_2$ | X/Y-6 | 3-Cyclopropyl |
| $CH_2$ | H | $CH_2$ | X/Y-6 | 3-$OCH_2CH_3$ |
| $CH(CH_3)$ | H | $CH(CH_3)$ | X/Y-6 | 3-Cyclopropyl |
| $CH(CH_3)$ | 3-F | $CH(CH_3)$ | X/Y-6 | 3-Cyclopropyl |
| $CH_2CH_2$ | H | $CH_2CH_2$ | X/Y-6 | 3-Cyclopropyl |
| $CH_2CH_2$ | H | $CH_2CH_2CH_2$ | X/Y-6 | 3-$CH_3$ |
| $CH(CH_3)CH_2$ | 3-F | $CH_2CH_2$ | X/Y-6 | 3-Cyclopropyl |
| $CH_2CH_2$ | H | $CH(CH_3)CH_2$ | X/Y-6 | 3-Cyclopropyl |
| $CH_2$ | H | $CH_2$ | X/Y-8 | 1-$CH_3$ |
| $CH_2$ | H | $CH_2$ | X/Y-8 | 1,3-$(CH_3)_2$ |
| $CH_2$ | H | $CH_2$ | X/Y-8 | 1,4-$(CH_3)_2$ |

Tabelle A (Forts.)

| A | $R^1$ | B | Y | Substituent an Y |
|---|---|---|---|---|
| $CH_2$ | H | $CH_2$ | X/Y-8 | 1-$CH_3$, 3-Cyclopropyl |
| $CH(CH_3)$ | H | $CH(CH_3)$ | X/Y-8 | 1-$CH_3$, 3-Cyclopropyl |
| $CH(CH_3)$ | 3-F | $CH(CH_3)$ | X/Y-8 | 1-$CH_3$, 3-Cyclopropyl |
| $CH_2CH_2$ | H | $CH_2CH_2$ | X/Y-8 | 1-$CH_3$, 3-Cyclopropyl |
| $CH_2CH_2$ | H | $CH_2CH_2CH_2$ | X/Y-8 | 1,3-$(CH_3)_2$ |
| $CH_2CH(CH_3)$ | H | $CH_2CH_2$ | X/Y-8 | 1-$CH_3$, 3-Cyclopropyl |
| $CH(CH_3)CH_2$ | 3-F | $CH_2CH_2$ | X/Y-8 | 1-$CH_3$, 3-Cyclopropyl |
| $CH_2$ | H | $CH_2$ | X/Y-10 | 1-$CH_3$ |
| $CH_2$ | H | $CH_2$ | X/Y-10 | 1,4-$(CH_3)_2$ |
| $CH_2$ | H | $CH_2$ | X/Y-10 | 1-$CH_3$, 4-$OCH_3$ |
| $CH_2$ | H | $CH_2$ | X/Y-10 | 1-$CH_3$, 5-$CH(CH_3)_2$ |
| $CH_2$ | H | $CH_2$ | X/Y-10 | 1-$CH_3$, 5-Cyclopropyl |
| $CH(CH_3)$ | H | $CH(CH_3)$ | X/Y-10 | 1-$CH_3$, 5-Cyclopropyl |
| $CH(CH_3)$ | 3-F | $CH(CH_3)$ | X/Y-10 | 1,4-$(CH_3)_2$ |
| $CH_2CH_2$ | H | $CH_2CH_2$ | X/Y-10 | 1-$CH_3$, 5-Cyclopropyl |
| $CH_2CH_2$ | H | $CH_2CH_2CH_2$ | X/Y-10 | 1,5-$(CH_3)_2$ |
| $CH_2CH(CH_3)$ | H | $CH_2CH_2$ | X/Y-10 | 1-$CH_3$, 5-Cyclopropyl |
| $CH(CH_3)CH_2$ | 3-F | $CH_2CH_2$ | X/Y-10 | 1-$CH_3$, 5-Cyclopropyl |
| $CH_2$ | H | $CH_2$ | X/Y-7 | - |
| $CH_2$ | H | $CH_2$ | X/Y-7 | 3-$CH_3$ |
| $CH(CH_3)$ | H | $CH(CH_3)$ | X/Y-7 | 3-$CH_3$ |
| $CH(CH_3)$ | 3-F | $CH(CH_3)$ | X/Y-7 | 3-$CH_3$ |
| $CH_2CH_2$ | H | $CH_2CH_2$ | X/Y-7 | 3-$CH_3$ |
| $CH_2CH_2$ | 3-F | $CH(CH_3)CH_2CH_2$ | X/Y-7 | 3-$CH_3$ |
| $CH_2$ | H | $CH_2$ | X/Y-9 | 1-$CH_3$ |
| $CH_2$ | H | $CH_2$ | X/Y-9 | 1,3-$(CH_3)_2$ |
| $CH_2$ | H | $CH_2$ | X/Y-9 | 1,5-$(CH_3)_2$ |
| $CH(CH_3)$ | H | $CH(CH_3)$ | X/Y-9 | 1,3-$(CH_3)_2$ |
| $CH(CH_3)$ | 3-F | $CH(CH_3)$ | X/Y-9 | 1,5-$(CH_3)_2$ |
| $CH_2CH_2$ | H | $CH_2CH_2$ | X/Y-9 | 1,3-$(CH_3)_2$ |
| $CH_2CH_2$ | H | $CH_2CH_2CH_2$ | X/Y-9 | 1,5-$(CH_3)_2$ |
| $CH_2CH(CH_3)$ | H | $CH_2CH_2$ | X/Y-9 | 1,3-$(CH_3)_2$ |
| $CH(CH_3)CH_2$ | 3-F | $CH_2CH_2$ | X/Y-9 | 1,5-$(CH_3)_2$ |
| $CH_2$ | H | $CH_2$ | X/Y-25 | 5-Cyclopropyl |
| $CH_2$ | H | $CH_2$ | X/Y-25 | 5-$CH_3$ |

Tabelle A (Forts.)

| A | $R^1$ | B | Y | Substituent an Y |
|---|---|---|---|---|
| $CH_2$ | H | $CH_2$ | X/Y-25 | 5-$CH_2CH_3$ |
| $CH_2$ | H | $CH_2$ | X/Y-25 | 5-$CH(CH_3)_2$ |
| $CH_2$ | H | $CH_2$ | X/Y-25 | 5-$OCH_3$ |
| $CH(CH_3)$ | H | $CH(CH_3)$ | X/Y-25 | 5-Cyclopropyl |
| $CH_2(CH_3)$ | H | $CH(CH_3)$ | X/Y-25 | 5-$CH_3$ |
| $CH(CH_3)$ | H | $CH(CH_3)$ | X/Y-25 | 5-$OCH_2CH_3$ |
| $CH(CH_3)$ | 3-F | $CH(CH_3)$ | X/Y-25 | 5-Cyclopropyl |
| $CH_2CH_2$ | H | $CH_2$ | X/Y-25 | 5-Cyclopropyl |
| $CH_2CH_2$ | H | $CH_2CH_2$ | X/Y-25 | 5-$CH_3$ |
| $CH_2CH_2CH_2$ | H | $CH_2$ | X/Y-25 | 5-$OCH_2CH_3$ |
| $CH_2CH_2$ | 3-F | $CH_2CH(CH_3)$ | X/Y-25 | 5-Cyclopropyl |
| $CH_2$ | H | $CH(CH_3)$ | X/Y-25 | 5-Cyclopropyl |
| $CH_2$ | H | $CH_2$ | X/Y-23 | – |
| $CH_2$ | H | $CH_2$ | X/Y-23 | 5-Cyclopropyl |
| $CH_2$ | H | $CH_2$ | X/Y-23 | 5-$CH_3$ |
| $CH_2$ | H | $CH_2$ | X/Y-23 | 5-$CH(CH_3)_2$ |
| $CH_2$ | H | $CH_2$ | X/Y-23 | 5-Cl |
| $CH_2$ | H | $CH_2$ | X/Y-23 | 5-$OCH_2CH_3$ |
| $CH_2$ | H | $CH(CH_3)$ | X/Y-23 | 5-Cyclopropyl |
| $CH_2$ | 3-F | $CH(CH_3)$ | X/Y-23 | 5-$CH_3$ |
| $CH_2$ | H | $CH(CH_3)$ | X/Y-23 | 5-$CH(CH_3)_2$ |
| $CH(CH_3)$ | H | $CH(CH_3)$ | X/Y-23 | 5-Cyclopropyl |
| $CH_2$ | H | $CH_2CH_2$ | X/Y-23 | 5-Cyclopropyl |
| $CH_2CH_2CH_2$ | H | $CH_2$ | X/Y-23 | 5-$CH_3$ |
| $CH_2CH_2$ | H | $CH_2CH_2$ | X/Y-23 | 5-$CH(CH_3)_2$ |
| $CH_2CH_2$ | H | $CH_2CH_2$ | X/Y-23 | 5-Cyclopropyl |
| $CH_2$ | H | $CH_2$ | X/Y-26 | 1-$CH_3$ |
| $CH_2$ | H | $CH_2$ | X/Y-26 | 1,5-$(CH_3)_2$ |
| $CH_2$ | H | $CH_2$ | X/Y-26 | 1-$CH_3$, 5-Cyclopropyl |
| $CH(CH_3)$ | H | $CH(CH_3)$ | X/Y-26 | 1-$CH_3$, 5-Cyclopropyl |
| $CH(CH_3)$ | H | $CH(CH_3)$ | X/Y-26 | 1,5-$(CH_3)_2$ |
| $CH(CH_3)$ | 3-F | $CH_2$ | X/Y-26 | 1-$CH_3$, 5-Cyclopropyl |
| $CH_2CH_2$ | H | $CH_2CH_2$ | X/Y-26 | 1-$CH_3$, 5-Cyclopropyl |
| $CH_2$ | H | $CH_2CH_2$ | X/Y-26 | 1,5-$(CH_3)_2$ |
| $CH_2$ | H | $CH_2$ | X/Y-28 | 1-$CH_3$ |
| $CH_2$ | H | $CH_2$ | X/Y-28 | 1,5-$(CH_3)_2$ |
| $CH_2$ | H | $CH_2$ | X/Y-28 | 1-$CH_3$, 5-Cyclopropyl |

Tabelle A (Forts.)

| A | $R^1$ | B | Y | Substituent an Y |
|---|---|---|---|---|
| $CH(CH_3)$ | H | $CH(CH_3)$ | X/Y-28 | $1-CH_3$, 5-Cyclopropyl |
| $CH(CH_3)$ | H | $CH(CH_3)$ | X/Y-28 | $1,5-(CH_3)_2$ |
| $CH(CH_3)$ | 3-F | $CH_2$ | X/Y-28 | $1-CH_3$, 5-Cyclopropyl |
| $CH_2CH_2$ | H | $CH_2CH_2$ | X/Y-28 | $1-CH_3$, 5-Cyclopropyl |
| $CH_2$ | H | $CH_2CH_2$ | X/Y-28 | $1,5-(CH_3)_2$ |
| $CH_2$ | H | $CH_2$ | X/Y-22 | – |
| $CH_2$ | H | $CH_2$ | X/Y-22 | $3-CH_3$ |
| $CH_2$ | H | $CH_2$ | X/Y-22 | 3-Cyclopropyl |
| $CH_2$ | H | $CH_2$ | X/Y-22 | $3-OCH_3$ |
| $CH(CH_3)$ | H | $CH_2$ | X/Y-22 | $3-CH_3$ |
| $CH_2$ | H | $CH(CH_3)$ | X/Y-22 | 3-Cyclopropyl |
| $CH_2$ | 3-F | $CH(CH_3)$ | X/Y-22 | $3-OCH_3$ |
| $CH_2CH_2$ | H | $CH_2CH_2$ | X/Y-22 | 3-Cyclopropyl |
| $CH_2$ | H | $CH_2CH_2$ | X/Y-22 | $3-CH_3$ |
| $CH_2$ | H | $CH_2$ | X/Y-29 | $1-CH_3$ |
| $CH_2$ | H | $CH_2$ | X/Y-29 | $1,3-(CH_3)_2$ |
| $CH_2$ | H | $CH_2$ | X/Y-29 | $1-CH_3$, 3-Cyclopropyl |
| $CH(CH_3)$ | H | $CH(CH_3)$ | X/Y-29 | $1,3-(CH_3)_2$ |
| $CH(CH_3)$ | 3-F | $CH(CH_3)$ | X/Y-29 | $1-CH_3$, 3-Cyclopropyl |
| $CH_2CH_2$ | H | $CH_2CH_2CH_2$ | X/Y-29 | $1,3-(CH_3)_2$ |
| $CH_2CH_2$ | H | $CH_2CH_2$ | X/Y-29 | $1-CH_3$, 3-Cyclopropyl |
| $CH_2$ | H | $CH_2$ | X/Y-17 | – |
| $CH_2$ | H | $CH_2$ | X/Y-17 | 5-Cyclopropyl |
| $CH_2$ | H | $CH_2$ | X/Y-17 | $5-CH_3$ |
| $CH_2$ | H | $CH_2$ | X/Y-17 | $5-OCH_2CH_3$ |
| $CH_2$ | H | $CH_2$ | X/Y-17 | 5-Cl |
| $CH_2$ | H | $CH_2$ | X/Y-17 | $4-CH_3$ |
| $CH_2$ | H | $CH_2$ | X/Y-17 | 4-Cyclopropyl |
| $CH_2$ | H | $CH_2$ | X/Y-17 | $4-OCH_2CH_3$ |
| $CH_2$ | H | $CH_2$ | X/Y-17 | 4-Cl |
| $CH(CH_3)$ | H | $CH(CH_3)$ | X/Y-17 | 5-Cyclopropyl |
| $CH(CH_3)$ | H | $CH(CH_3)$ | X/Y-17 | 4-Cyclopropyl |
| $CH(CH_3)$ | 3-F | $CH(CH_3)$ | X/Y-17 | $5-OCH_2CH_3$ |
| $CH_2CH_2$ | H | $CH_2CH_2$ | X/Y-17 | 5-Cyclopropyl |
| $CH_2CH_2$ | H | $CH_2CH_2$ | X/Y-17 | 4-Cyclopropyl |

Tabelle A (Forts.)

| A | $R^1$ | B | Y | Substituent an Y |
|---|---|---|---|---|
| $CH_2CH_2$ | H | $CH_2CH_2CH_2$ | X/Y-17 | 5-$CH_3$ |
| $CH_2$ | H | $CH_2$ | X/Y-14 | – |
| $CH_2$ | H | $CH_2$ | X/Y-14 | 5-$CH_3$ |
| $CH_2$ | H | $CH_2$ | X/Y-14 | 5-Cyclopropyl |
| $CH_2$ | H | $CH_2$ | X/Y-14 | 4-$CH_3$ |
| $CH_2$ | H | $CH_2$ | X/Y-14 | 4-Cyclopropyl |
| $CH_2$ | H | $CH_2$ | X/Y-14 | 4-$OCH_2CH_3$ |
| $CH_2$ | H | $CH_2$ | X/Y-14 | 5-$OCH_2CH_3$ |
| $CH_2$ | H | $CH_2$ | X/Y-14 | 5-Cl |
| $CH(CH_3)$ | H | $CH(CH_3)$ | X/Y-14 | 4-Cyclopropyl |
| $CH(CH_3)$ | H | $CH(CH_3)$ | X/Y-14 | 5-Cyclopropyl |
| $CH(CH_3)$ | H | $CH(CH_3)$ | X/Y-14 | 4-$CH_3$ |
| $CH(CH_3)$ | 3-F | $CH(CH_3)$ | X/Y-14 | 4-Cyclopropyl |
| $CH_2$ | 3-F | $CH_2$ | X/Y-14 | 5-$OCH_2CH_3$ |
| $CH_2CH_2$ | H | $CH_2CH_2$ | X/Y-14 | 4-Cyclopropyl |
| $CH_2CH_2$ | H | $CH_2CH_2$ | X/Y-14 | 5-Cyclopropyl |
| $CH_2CH_2$ | H | $CH_2CH_2CH_2$ | X/Y-14 | 5-Cl |
| $CH_2CH_2$ | H | $CH_2CH_2$ | X/Y-14 | 4-$CH_3$ |
| $CH_2$ | H | $CH_2$ | X/Y-20 | 1-$CH_3$ |
| $CH_2$ | H | $CH_2$ | X/Y-20 | 1,5-$(CH_3)_2$ |
| $CH(CH_3)$ | H | $CH(CH_3)$ | X/Y-20 | 1-$CH_3$ |
| $CH(CH_3)$ | 3-F | $CH(CH_3)$ | X/Y-20 | 1,5-$(CH_3)_2$ |
| $CH_2CH_2$ | H | $CH_2CH_2$ | X/Y-20 | 1-$CH_3$ |
| $CH_2CH_2$ | H | $CH_2CH_2$ | X/Y-20 | 1,5-$(CH_3)_2$ |
| $CH(CH_3)CH_2$ | 3-F | $CH(CH_3)CH_2$ | X/Y-20 | 1,5-$(CH_3)_2$ |
| $CH_2$ | H | $CH_2$ | X/Y-3 | – |
| $CH_2$ | H | $CH_2$ | X/Y-3 | 3-$CH_3$ |
| $CH_2$ | H | $CH_2$ | X/Y-3 | 3-$CH_2CH_3$ |
| $CH_2$ | H | $CH_2$ | X/Y-3 | 3-$CH(CH_3)_2$ |
| $CH_2$ | H | $CH_2$ | X/Y-3 | 3-Cyclopropyl |
| $CH_2$ | H | $CH_2$ | X/Y-3 | 3-$OCH_2CH_3$ |
| $CH_2$ | H | $CH_2$ | X/Y-3 | 3-$CH_2OCH_3$ |
| $CH_2$ | H | $CH_2$ | X/Y-3 | 3-$CF_3$ |
| $CH(CH_3)$ | H | $CH_2$ | X/Y-3 | 3-$CH_3$ |
| $CH(CH_3)$ | H | $CH_2$ | X/Y-3 | 3-$CH(CH_3)_2$ |

Tabelle A (Forts.)

| A | $R^1$ | B | Y | Substituent an Y |
|---|---|---|---|---|
| $CH(CH_3)$ | H | $CH_2$ | X/Y-3 | 3-Cyclopropyl |
| $CH_2$ | H | $CH(CH_3)$ | X/Y-3 | $3-CH_3$ |
| $CH_2$ | H | $CH(CH_3)$ | X/Y-3 | $3-CH(CH_3)_2$ |
| $CH_2$ | H | $CH(CH_3)$ | X/Y-3 | 3-Cyclopropyl |
| $CH(CH_3)$ | H | $CH(CH_3)$ | X/Y-3 | $3-CH_3$ |
| $CH(CH_3)$ | 3-F | $CH(CH_3)$ | X/Y-3 | $3-CH(CH_3)_2$ |
| $CH(CH_3)$ | H | $CH(CH_3)$ | X/Y-3 | 3-Cyclopropyl |
| $CH_2$ | H | $CH_2CH_2$ | X/Y-3 | $3-CH_3$ |
| $CH_2CH_2$ | H | $CH_2CH_2CH_2$ | X/Y-3 | $3-CH(CH_3)_2$ |
| $CH_2$ | H | $CH_2CH_2$ | X/Y-3 | 3-Cyclopropyl |
| $CH_2CH_2$ | H | $CH_2CH(CH_3)$ | X/Y-3 | 3-Cyclopropyl |
| $CH_2CH_2$ | H | $CH_2CH(CH_3)$ | X/Y-3 | $3-CH(CH_3)_2$ |
| $CH_2CH_2CH_2$ | 3-F | $CH(CH_3)CH_2$ | X/Y-3 | $3-CH_3$ |
| $CH_2$ | H | $CH_2$ | X/Y-5 | - |
| $CH_2$ | H | $CH_2$ | X/Y-5 | $5-OCH_2CH_3$ |
| $CH_2$ | H | $CH_2$ | X/Y-5 | 5-Cyclopropyl |
| $CH_2$ | H | $CH_2$ | X/Y-5 | $5-CH_3$ |
| $CH(CH_3)$ | 3-F | $CH(CH_3)$ | X/Y-5 | $5-CH_3$ |
| $CH_2$ | H | $CH(CH_3)$ | X/Y-5 | 5-Cyclopropyl |
| $CH(CH_3)$ | H | $CH_2$ | X/Y-5 | 5-Cyclopropyl |
| $CH_2$ | H | $CH_2CH_2$ | X/Y-5 | $5-OCH_2CH_3$ |
| $CH_2CH_2$ | H | $CH_2CH_2CH_2$ | X/Y-5 | $5-CH_3$ |
| $CH_2$ | H | $CH_2$ | X/Y-4 | - |
| $CH_2$ | H | $CH_2$ | X/Y-4 | $3-OCH_2CH_3$ |
| $CH_2$ | H | $CH_2$ | X/Y-4 | $5-OCH_2CH_3$ |
| $CH_2$ | H | $CH_2$ | X/Y-4 | $3-CH(CH_3)_2$ |
| $CH_2$ | H | $CH_2$ | X/Y-4 | $5-CH_3$ |
| $CH(CH_3)$ | 3-F | $CH(CH_3)$ | X/Y-4 | $3-CH(CH_3)_2$ |
| $CH_2$ | H | $CH_2$ | X/Y-11 | - |
| $CH_2$ | H | $CH_2$ | X/Y-11 | $3-CH_3$ |
| $CH_2$ | H | $CH_2$ | X/Y-11 | $5-CH_3$ |
| $CH_2$ | H | $CH(CH_3)$ | X/Y-11 | $3-CH_3$ |
| $CH_2$ | 3-F | $CH(CH_3)$ | X/Y-11 | $5-CH_3$ |
| $CH(CH_3)$ | H | $CH(CH_3)$ | X/Y-11 | $5-CH_3$ |
| $CH_2$ | H | $CH_2$ | X/Y-21 | - |

Tabelle A (Forts.)

| A | $R^1$ | B | Y | Substituent an Y |
|---|---|---|---|---|
| $CH_2$ | H | $CH_2$ | X/Y-21 | 4,5-$Cl_2$ |
| $CH_2$ | H | $CH_2$ | X/Y-21 | 4,5-$(CH_3)_2$ |
| $CH_2$ | H | $CH_2$ | X/Y-21 | 4,5-$Br_2$ |
| $CH(CH_3)$ | H | $CH(CH_3)$ | X/Y-21 | 4,5-$Cl_2$ |
| $CH(CH_3)$ | H | $CH(CH_3)$ | X/Y-21 | 4,5-$(CH_3)_2$ |
| $CH(CH_3)$ | H | $CH(CH_3)$ | X/Y-21 | 4,5-$Br_2$ |
| $CH_2$ | H | $CH_2$ | X/Y-30 | – |
| $CH_2$ | H | $CH_2$ | X/Y-30 | 3,5-$(CH_3)_2$ |
| $CH(CH_3)$ | H | $CH(CH_3)$ | X/Y-30 | – |
| $CH(CH_3)$ | H | $CH(CH_3)$ | X/Y-30 | 3,5-$(CH_3)_2$ |
| $CH_2$ | H | $CH_2$ | X/Y-27 | – |
| $CH_2$ | H | $CH_2$ | X/Y-27 | 2-Cl |
| $CH_2$ | H | $CH_2$ | X/Y-27 | 2,5-$(CH_3)_2$ |
| $CH(CH_3)$ | H | $CH(CH_3)$ | X/Y-27 | – |
| $CH(CH_3)$ | H | $CH(CH_3)$ | X/Y-27 | 2-Cl |
| $CH(CH_3)$ | H | $CH(CH_3)$ | X/Y-27 | 2,5-$(CH_3)_2$ |

Tabelle B (zu Struktur I.B).

| A | $R^1$ | B | Y | Substituent an Y |
|---|---|---|---|---|
| $CH_2$ | H | $CH_2$ | X/Y-1 | |
| $CH(CH_3)$ | H | $CH_2$ | X/Y-1 | |
| $CH(CH_2CH_3)$ | H | $CH_2$ | X/Y-1 | |
| $CH_2$ | H | $CH(CH_3)$ | X/Y-1 | |
| $CH_2$ | H | $CH(CH_2CH_3)$ | X/Y-1 | |
| $CH_2$ | 3-$CH_3$ | $CH_2$ | X/Y-1 | |
| $CH_2$ | 3-F | $CH_2$ | X/Y-1 | |
| $CH_2$ | H | $CH_2$ | X/Y-1 | 5-$CH_3$ |
| $CH_2$ | H | $CH_2$ | X/Y-1 | 4-$CH_3$ |
| $CH_2$ | H | $CH_2$ | X/Y-1 | 3-$CH_3$ |
| $CH_2$ | H | $CH_2$ | X/Y-1 | 5-Cyclopropyl |
| $CH_2$ | H | $CH_2$ | X/Y-1 | 4-Cyclopropyl |
| $CH_2$ | H | $CH_2$ | X/Y-1 | 3-Cyclopropyl |
| $CH_2$ | H | $CH_2$ | X/Y-1 | 5-Cl |
| $CH_2$ | H | $CH_2$ | X/Y-1 | 5-Br |
| $CH_2$ | H | $CH_2$ | X/Y-1 | 4-Cl |
| $CH_2$ | H | $CH_2$ | X/Y-1 | 4-Br |
| $CH_2$ | H | $CH_2$ | X/Y-1 | 4,5-Cl,Cl |
| $CH_2$ | H | $CH_2$ | X/Y-1 | 4-$OCH_2CH_3$ |
| $CH(CH_3)$ | H | $CH_2$ | X/Y-1 | 5-Br |
| $CH(CH_3)$ | H | $CH_2$ | X/Y-1 | 4,5-Cl,Cl |
| $CH(CH_3)$ | H | $CH_2$ | X/Y-1 | 4-Cl |
| $CH_2$ | H | $CH(CH_3)$ | X/Y-1 | 5-Br |
| $CH_2$ | H | $CH(CH_3)$ | X/Y-1 | 4,5-Cl,Cl |
| $CH(CH_3)$ | H | $CH(CH_3)$ | X/Y-1 | - |
| $CH(CH_3$ | H | $CH(CH_3)$ | X/Y-1 | 4,5-Cl,Cl |
| $CH(CH_3)$ | 3-F | $CH(CH_3)$ | X/Y-1 | - |
| $CH(CH_3)$ | 3-F | $CH(CH_3)$ | X/Y-1 | 5-Br |
| $CH_2CH_2$ | H | $CH_2$ | X/Y-1 | 5-Br |
| $CH_2CH_2$ | H | $CH_2CH_2$ | X/Y-1 | 4,5-Cl,Cl |
| $CH_2CH_2CH_2$ | H | $CH_2$ | X/Y-1 | 5-Br |
| $CH_2CH_2$ | H | $CH_2CH_2CH_2$ | X/Y-1 | 4,5-Cl,Cl |
| $CH_2CH(CH_3)$ | H | $CH_2CH_2$ | X/Y-1 | 4,5-Cl,Cl |
| $CH_2CH_2$ | 3-F | $CH_2CH(CH_3)$ | X/Y-1 | 5-Br |

Tabelle B (Forts.)

| A | $R^1$ | B | Y | Substituent an Y |
|---|---|---|---|---|
| $CH_2$ | H | $CH_2$ | X/Y-2 | – |
| $CH_2$ | H | $CH_2$ | X/Y-2 | 4-Cyclopropyl |
| $CH_2$ | H | $CH_2$ | X/Y-2 | 5-Cyclopropyl |
| $CH_2$ | H | $CH_2$ | X/Y-2 | 5-Cl |
| $CH_2$ | H | $CH_2$ | X/Y-2 | 4,5-Cl,Cl |
| $CH_2$ | H | $CH_2$ | X/Y-2 | 4,5-Br,Br |
| $CH_2$ | H | $CH_2$ | X/Y-2 | 4-$CH_3$ |
| $CH_2$ | H | $CH_2$ | X/Y-2 | 5-$CH_3$ |
| $CH_2$ | H | $CH_2$ | X/Y-2 | 4-$OCH_3$ |
| $CH_2$ | H | $CH_2$ | X/Y-2 | 5-$OCH_3$ |
| $CH(CH_3)$ | H | $CH_2$ | X/Y-2 | 4,5-Cl,Cl |
| $CH(CH_3)$ | H | $CH_2$ | X/Y-2 | 4,5-Br,Br |
| $CH_2$ | H | $CH(CH_3)$ | X/Y-2 | 4,5-Cl,Cl |
| $CH_2$ | H | $CH(CH_3)$ | X/Y-2 | 4,5-Br,Br |
| $CH(CH_3)$ | H | $CH(CH_3)$ | X/Y-2 | 4,5-Cl,Cl |
| $CH(CH_3)$ | H | $CH(CH_3)$ | X/Y-2 | 4,5-Br,Br |
| $CH(CH_3)$ | 3-F | $CH(CH_3)$ | X/Y-2 | 4,5-Cl,Cl |
| $CH(CH_3)$ | 3-F | $CH(CH_3)$ | X/Y-2 | 4,5-Br,Br |
| $CH_2CH_2$ | H | $CH_2$ | X/Y-2 | 4,5-Cl,Cl |
| $CH_2CH_2$ | H | $CH_2CH_2$ | X/Y-2 | 4,5-Br,Br |
| $CH_2CH_2CH_2$ | H | $CH_2$ | X/Y-2 | 4,5-Cl,Cl |
| $CH_2CH_2$ | H | $CH_2CH_2CH_2$ | X/Y-2 | 4,5-Br,Br |
| $CH_2CH(CH_3)$ | H | $CH_2CH_2$ | X/Y-2 | 4,5-Br,Br |
| $CH_2CH_2$ | 3-F | $CH_2CH(CH_3)$ | X/Y-2 | 4,5-Cl,Cl |
| $CH_2$ | H | $CH_2$ | X/Y-15 | – |
| $CH_2$ | H | $CH_2$ | X/Y-15 | 2-$CH_3$ |
| $CH_2$ | H | $CH_2$ | X/Y-15 | 2-Cyclopropyl |
| $CH_2$ | H | $CH_2$ | X/Y-15 | 2-$OCH_2CH_3$ |
| $CH_2$ | H | $CH_2$ | X/Y-15 | 2-Cl |
| $CH(CH_3)$ | H | $CH_2$ | X/Y-15 | 2-Cyclopropyl |
| $CH(CH_3)$ | H | $CH_2$ | X/Y-15 | 2-$OCH_2CH_3$ |
| $CH_2$ | H | $CH(CH_3)$ | X/Y-15 | 2-Cyclopropyl |
| $CH_2$ | H | $CH(CH_3)$ | X/Y-15 | 2-$OCH_2CH_3$ |
| $CH(CH_3)$ | H | $CH(CH_3)$ | X/Y-15 | 2-Cyclopropyl |
| $CH(CH_3)$ | H | $CH(CH_3)$ | X/Y-15 | 2-$OCH_2CH_3$ |

Tabelle B (Forts.)

| A | $R^1$ | B | Y | Substituent an Y |
|---|---|---|---|---|
| $CH(CH_3)$ | 3-F | $CH(CH_3)$ | X/Y-15 | 2-Cyclopropyl |
| $CH(CH_3)$ | 3-F | $CH(CH_3)$ | X/Y-15 | 2-$OCH_2CH_3$ |
| $CH_2CH_2$ | H | $CH_2$ | X/Y-15 | 2-Cyclopropyl |
| $CH_2CH_2$ | H | $CH_2CH_2$ | X/Y-15 | 2-$OCH_2CH_3$ |
| $CH_2CH_2CH_2$ | H | $CH_2$ | X/Y-15 | 2-Cyclopropyl |
| $CH_2CH_2$ | H | $CH_2CH_2CH_2$ | X/Y-15 | 2-$OCH_2CH_3$ |
| $CH_2CH(CH_3)$ | H | $CH_2CH_2$ | X/Y-15 | 2-Cyclopropyl |
| $CH_2CH_2$ | 3-F | $CH_2CH(CH_3)$ | X/Y-15 | 2-$OCH_2CH_3$ |
| $CH_2$ | H | $CH_2$ | X/Y-12 | - |
| $CH_2$ | H | $CH_2$ | X/Y-12 | 2-$CH_3$ |
| $CH_2$ | H | $CH_2$ | X/Y-12 | 2-Cyclopropyl |
| $CH_2$ | H | $CH_2$ | X/Y-12 | 2-$OCH_2CH_3$ |
| $CH(CH_3)$ | H | $CH_2$ | X/Y-12 | 2-Cyclopropyl |
| $CH(CH_3)$ | H | $CH_2$ | X/Y-12 | 2-$OCH_2CH_3$ |
| $CH_2$ | H | $CH(CH_3)$ | X/Y-12 | 2-Cyclopropyl |
| $CH_2$ | H | $CH(CH_3)$ | X/Y-12 | 2-$OCH_2CH_3$ |
| $CH(CH_3)$ | H | $CH(CH_3)$ | X/Y-12 | 2-Cyclopropyl |
| $CH(CH_3)$ | 3-F | $CH(CH_3)$ | X/Y-12 | 2-$OCH_2CH_3$ |
| $CH_2CH_2$ | H | $CH_2$ | X/Y-12 | 2-Cyclopropyl |
| $CH_2CH_2$ | H | $CH_2CH_2$ | X/Y-12 | 2-$OCH_2CH_3$ |
| $CH_2CH_2CH_2$ | H | $CH_2$ | X/Y-12 | 2-Cyclopropyl |
| $CH_2CH_2$ | H | $CH_2CH_2CH_2$ | X/Y-12 | 2-$OCH_2CH_3$ |
| $CH_2CH(CH_3)$ | H | $CH_2CH_2$ | X/Y-12 | 2-Cyclopropyl |
| $CH_2CH_2$ | 3-F | $CH_2CH(CH_3)$ | X/Y-12 | 2-Cyclopropyl |
| $CH_2$ | H | $CH_2$ | X/Y-18 | 1-$CH_3$ |
| $CH_2$ | H | $CH_2$ | X/Y-18 | 1,2-$(CH_3)_2$ |
| $CH_2$ | H | $CH_2$ | X/Y-18 | 1,4-$(CH_3)_2$ |
| $CH_2$ | H | $CH_2$ | X/Y-18 | 1,2,4-$(CH_3)_2$ |
| $CH_2$ | H | $CH_2$ | X/Y-18 | 1-$CH_3$, 2-Cyclopropyl |
| $CH(CH_3)$ | H | $CH_2$ | X/Y-18 | 1-$CH_3$, 2-Cyclopropyl |
| $CH(CH_3)$ | H | $CH_2$ | X/Y-18 | 1,2,4-$(CH_3)_3$ |
| $CH_2$ | H | $CH(CH_3)$ | X/Y-18 | 1-$CH_3$, 2-Cyclopropyl |
| $CH_2$ | H | $CH(CH_3)$ | X/Y-18 | 1,2,4-$(CH_3)_3$ |
| $CH(CH_3)$ | H | $CH(CH_3)$ | X/Y-18 | 1-$CH_3$, 2-Cyclopropyl |
| $CH(CH_3)$ | 3-F | $CH(CH_3)$ | X/Y-18 | 1,2,4-$(CH_3)_3$ |

Tabelle B (Forts.)

| A | $R^1$ | B | Y | Substituent an Y |
|---|---|---|---|---|
| $CH_2CH_2$ | H | $CH_2$ | X/Y-18 | 1-$CH_3$, 2-Cyclopropyl |
| $CH_2CH_2$ | H | $CH_2CH_2$ | X/Y-18 | 1,2,4-$(CH_3)_2$ |
| $CH_2CH_2CH_2$ | H | $CH_2$ | X/Y-18 | 1-$CH_3$, 2-Cyclopropyl |
| $CH_2CH_2$ | H | $CH_2CH_2CH_2$ | X/Y-18 | 1,2,4-$(CH_3)_2$ |
| $CH_2CH(CH_3)$ | H | $CH_2CH_2$ | X/Y-18 | 1-$CH_3$, 2-Cyclopropyl |
| $CH_2CH_2$ | 3-F | $CH_2CH(CH_3)$ | X/Y-18 | 1,2,4-$(CH_3)_2$ |
| $CH_2$ | H | $CH_2$ | X/Y-19 | 1-$CH_3$ |
| $CH_2$ | H | $CH_2$ | X/Y-19 | 1,2-$(CH_3)_2$ |
| $CH_2$ | H | $CH_2$ | X/Y-19 | 1,5-$(CH_3)_2$ |
| $CH_2$ | H | $CH_2$ | X/Y-19 | 1-$CH_3$, 2-Cyclopropyl |
| $CH(CH_3)$ | H | $CH_2$ | X/Y-19 | 1,2-$(CH_3)_2$ |
| $CH(CH_3)$ | H | $CH_2$ | X/Y-19 | 1-$CH_3$, 2-Cyclopropyl |
| $CH_2$ | H | $CH(CH_3)$ | X/Y-19 | 1,2-$(CH_3)_2$ |
| $CH_2$ | H | $CH(CH_3)$ | X/Y-19 | 1-$CH_3$, 2-Cyclopropyl |
| $CH(CH_3)$ | H | $CH(CH_3)$ | X/Y-19 | 1,2-$(CH_3)_2$ |
| $CH(CH_3)$ | H | $CH(CH_3)$ | X/Y-19 | 1-$CH_3$, 2-Cyclopropyl |
| $CH(CH_3)$ | 3-F | $CH_2$ | X/Y-19 | 1-$CH_3$, 2-Cyclopropyl |
| $CH_2CH_2$ | H | $CH_2$ | X/Y-19 | 1-$CH_3$, 2-Cyclopropyl |
| $CH_2CH_2$ | H | $CH_2CH_2$ | X/Y-19 | 1,2-$(CH_3)_2$ |
| $CH_2CH_2CH_2$ | H | $CH_2$ | X/Y-19 | 1-$CH_3$, 2-Cyclopropyl |
| $CH_2CH_2$ | H | $CH_2CH_2CH_2$ | X/Y-19 | 1,2-$(CH_3)_2$ |
| $CH_2CH(CH_3)$ | H | $CH_2CH_2$ | X/Y-19 | 1-$CH_3$, 2-Cyclopropyl |
| $CH_2CH_2$ | 3-F | $CH_2CH(CH_3)$ | X/Y-19 | 1,2-$(CH_3)_2$ |
| $CH_2$ | H | $CH_2$ | X/Y-16 | - |
| $CH_2$ | H | $CH_2$ | X/Y-16 | 2-$CH_3$ |
| $CH_2$ | H | $CH_2$ | X/Y-16 | 2-Cyclopropyl |
| $CH_2$ | H | $CH_2$ | X/Y-16 | 2-Cl |
| $CH_2$ | H | $CH_2$ | X/Y-16 | 2-$OCH_2CH_3$ |
| $CH(CH_3)$ | H | $CH_2$ | X/Y-16 | 2-$CH_3$ |
| $CH(CH_3)$ | H | $CH_2$ | X/Y-16 | 2-Cyclopropyl |
| $CH_2$ | H | $CH(CH_3)$ | X/Y-16 | 2-$CH_3$ |
| $CH_2$ | H | $CH(CH_3)$ | X/Y-16 | 2-Cyclopropyl |
| $CH_2(CH_3)$ | H | $CH(CH_3)$ | X/Y-16 | 2-$CH_3$ |
| $CH_2(CH_3)$ | H | $CH(CH_3)$ | X/Y-16 | 2-Cyclopropyl |
| $CH_2(CH_3)$ | 3-F | $CH(CH_3)$ | X/Y-16 | 2-Cyclopropyl |

Tabelle B (Forts.)

| A | $R^1$ | B | Y | Substituent an Y |
|---|---|---|---|---|
| $CH_2CH_2$ | H | $CH_2$ | X/Y-16 | 2-Cyclopropyl |
| $CH_2CH_2$ | H | $CH_2CH_2$ | X/Y-16 | 2-$CH_3$ |
| $CH_2CH_2CH_2$ | H | $CH_2$ | X/Y-16 | 2-Cyclopropyl |
| $CH_2CH_2$ | H | $CH_2CH_2CH_2$ | X/Y-16 | 2-$CH_3$ |
| $CH_2CH(CH_3)$ | H | $CH_2CH_2$ | X/Y-16 | 2-Cyclopropyl |
| $CH_2CH_2$ | 3-F | $CH_2CH(CH_3)$ | X/Y-16 | 2-$CH_3$ |
| $CH_2$ | H | $CH_2$ | X/Y-13 | - |
| $CH_2$ | H | $CH_2$ | X/Y-13 | 2-$CH_3$ |
| $CH_2$ | H | $CH_2$ | X/Y-13 | 2-Cyclopropyl |
| $CH_2$ | H | $CH_2$ | X/Y-13 | 2-$OCH_2CH_3$ |
| $CH(CH_3)$ | H | $CH_2$ | X/Y-13 | 2-$OCH_2CH_3$ |
| $CH(CH_3)$ | H | $CH_2$ | X/Y-13 | 2-Cyclopropyl |
| $CH_2$ | H | $CH(CH_3)$ | X/Y-13 | 2-$OCH_2CH_3$ |
| $CH_2$ | H | $CH(CH_3)$ | X/Y-13 | 2-Cyclopropyl |
| $CH(CH_3)$ | H | $CH(CH_3)$ | X/Y-13 | 2-$OCH_2CH_3$ |
| $CH(CH_3)$ | 3-F | $CH(CH_3)$ | X/Y-13 | 2-Cyclopropyl |
| $CH_2CH_2$ | H | $CH_2$ | X/Y-13 | 2-Cyclopropyl |
| $CH_2CH_2$ | H | $CH_2CH_2$ | X/Y-13 | 2-$CH_3$ |
| $CH_2CH_2CH_2$ | H | $CH_2$ | X/Y-13 | 2-Cyclopropyl |
| $CH_2CH_2$ | H | $CH_2CH_2CH_2$ | X/Y-13 | 2-$CH_3$ |
| $CH(CH_3)CH_2$ | 3-F | $CH_2CH_2$ | X/Y-13 | 2-Cyclopropyl |
| $CH_2CH_2$ | H | $CH(CH_3)CH_2$ | X/Y-13 | 2-$CH_3$ |
| $CH_2$ | H | $CH_2$ | X/Y-6 | - |
| $CH_2$ | H | $CH_2$ | X/Y-6 | 3-$CH_3$ |
| $CH_2$ | H | $CH_2$ | X/Y-6 | 3-Cyclopropyl |
| $CH_2$ | H | $CH_2$ | X/Y-6 | 3-$OCH_2CH_3$ |
| $CH(CH_3)$ | H | $CH(CH_3)$ | X/Y-6 | 3-Cyclopropyl |
| $CH(CH_3)$ | 3-F | $CH(CH_3)$ | X/Y-6 | 3-Cyclopropyl |
| $CH_2CH_2$ | H | $CH_2CH_2$ | X/Y-6 | 3-Cyclopropyl |
| $CH_2CH_2$ | H | $CH_2CH_2CH_2$ | X/Y-6 | 3-$CH_3$ |
| $CH(CH_3)CH_2$ | 3-F | $CH_2CH_2$ | X/Y-6 | 3-Cyclopropyl |
| $CH_2CH_2$ | H | $CH(CH_3)CH_2$ | X/Y-6 | 3-Cyclopropyl |
| $CH_2$ | H | $CH_2$ | X/Y-8 | 1-$CH_3$ |
| $CH_2$ | H | $CH_2$ | X/Y-8 | 1,3-$(CH_3)_2$ |
| $CH_2$ | H | $CH_2$ | X/Y-8 | 1,4-$(CH_3)_2$ |

Tabelle B (Forts.)

| A | $R^1$ | B | Y | Substituent an Y |
|---|---|---|---|---|
| $CH_2$ | H | $CH_2$ | X/Y-8 | 1-$CH_3$, 3-Cyclopropyl |
| $CH(CH_3)$ | H | $CH(CH_3)$ | X/Y-8 | 1-$CH_3$, 3-Cyclopropyl |
| $CH(CH_3)$ | 3-F | $CH(CH_3)$ | X/Y-8 | 1-$CH_3$, 3-Cyclopropyl |
| $CH_2CH_2$ | H | $CH_2CH_2$ | X/Y-8 | 1-$CH_3$, 3-Cyclopropyl |
| $CH_2CH_2$ | H | $CH_2CH_2CH_2$ | X/Y-8 | 1,3-$(CH_3)_2$ |
| $CH_2CH(CH_3)$ | H | $CH_2CH_2$ | X/Y-8 | 1-$CH_3$, 3-Cyclopropyl |
| $CH(CH_3)CH_2$ | 3-F | $CH_2CH_2$ | X/Y-8 | 1-$CH_3$, 3-Cyclopropyl |
| $CH_2$ | H | $CH_2$ | X/Y-10 | 1-$CH_3$ |
| $CH_2$ | H | $CH_2$ | X/Y-10 | 1,4-$(CH_3)_2$ |
| $CH_2$ | H | $CH_2$ | X/Y-10 | 1-$CH_3$, 4-$OCH_3$ |
| $CH_2$ | H | $CH_2$ | X/Y-10 | 1-$CH_3$, 5-$CH(CH_3)_2$ |
| $CH_2$ | H | $CH_2$ | X/Y-10 | 1-$CH_3$, 5-Cyclopropyl |
| $CH(CH_3)$ | H | $CH(CH_3)$ | X/Y-10 | 1-$CH_3$, 5-Cyclopropyl |
| $CH(CH_3)$ | 3-F | $CH(CH_3)$ | X/Y-10 | 1,4-$(CH_3)_2$ |
| $CH_2CH_2$ | H | $CH_2CH_2$ | X/Y-10 | 1-$CH_3$, 5-Cyclopropyl |
| $CH_2CH_2$ | H | $CH_2CH_2CH_2$ | X/Y-10 | 1,5-$(CH_3)_2$ |
| $CH_2CH(CH_3)$ | H | $CH_2CH_2$ | X/Y-10 | 1-$CH_3$, 5-Cyclopropyl |
| $CH(CH_3)CH_2$ | 3-F | $CH_2CH_2$ | X/Y-10 | 1-$CH_3$, 5-Cyclopropyl |
| $CH_2$ | H | $CH_2$ | X/Y-7 | - |
| $CH_2$ | H | $CH_2$ | X/Y-7 | 3-$CH_3$ |
| $CH(CH_3)$ | H | $CH(CH_3)$ | X/Y-7 | 3-$CH_3$ |
| $CH(CH_3)$ | 3-F | $CH(CH_3)$ | X/Y-7 | 3-$CH_3$ |
| $CH_2CH_2$ | H | $CH_2CH_2$ | X/Y-7 | 3-$CH_3$ |
| $CH_2CH_2$ | 3-F | $CH(CH_3)CH_2CH_2$ | X/Y-7 | 3-$CH_3$ |
| $CH_2$ | H | $CH_2$ | X/Y-9 | 1-$CH_3$ |
| $CH_2$ | H | $CH_2$ | X/Y-9 | 1,3-$(CH_3)_2$ |
| $CH_2$ | H | $CH_2$ | X/Y-9 | 1,5-$(CH_3)_2$ |
| $CH(CH_3)$ | H | $CH(CH_3)$ | X/Y-9 | 1,3-$(CH_3)_2$ |
| $CH(CH_3)$ | 3-F | $CH(CH_3)$ | X/Y-9 | 1,5-$(CH_3)_2$ |
| $CH_2CH_2$ | H | $CH_2CH_2$ | X/Y-9 | 1,3-$(CH_3)_2$ |
| $CH_2CH_2$ | H | $CH_2CH_2CH_2$ | X/Y-9 | 1,5-$(CH_3)_2$ |
| $CH_2CH(CH_3)$ | H | $CH_2CH_2$ | X/Y-9 | 1,3-$(CH_3)_2$ |
| $CH(CH_3)CH_2$ | 3-F | $CH_2CH_2$ | X/Y-9 | 1,5-$(CH_3)_2$ |
| $CH_2$ | H | $CH_2$ | X/Y-25 | 5-Cyclopropyl |
| $CH_2$ | H | $CH_2$ | X/Y-25 | 5-$CH_3$ |

Tabelle B (Forts.)

| A | R1 | B | Y | Substituent an Y |
|---|---|---|---|---|
| $CH_2$ | H | $CH_2$ | X/Y-25 | 5-$CH_2CH_3$ |
| $CH_2$ | H | $CH_2$ | X/Y-25 | 5-$CH(CH_3)_2$ |
| $CH_2$ | H | $CH_2$ | X/Y-25 | 5-$OCH_3$ |
| $CH(CH_3)$ | H | $CH(CH_3)$ | X/Y-25 | 5-Cyclopropyl |
| $CH_2(CH_3)$ | H | $CH(CH_3)$ | X/Y-25 | 5-$CH_3$ |
| $CH(CH_3)$ | H | $CH(CH_3)$ | X/Y-25 | 5-$OCH_2CH_3$ |
| $CH(CH_3)$ | 3-F | $CH(CH_3)$ | X/Y-25 | 5-Cyclopropyl |
| $CH_2CH_2$ | H | $CH_2$ | X/Y-25 | 5-Cyclopropyl |
| $CH_2CH_2$ | H | $CH_2CH_2$ | X/Y-25 | 5-$CH_3$ |
| $CH_2CH_2CH_2$ | H | $CH_2$ | X/Y-25 | 5-$OCH_2CH_3$ |
| $CH_2CH_2$ | 3-F | $CH_2CH(CH_3)$ | X/Y-25 | 5-Cyclopropyl |
| $CH_2$ | H | $CH(CH_3)$ | X/Y-25 | 5-Cyclopropyl |
| $CH_2$ | H | $CH_2$ | X/Y-23 | - |
| $CH_2$ | H | $CH_2$ | X/Y-23 | 5-Cyclopropyl |
| $CH_2$ | H | $CH_2$ | X/Y-23 | 5-$CH_3$ |
| $CH_2$ | H | $CH_2$ | X/Y-23 | 5-$CH(CH_3)_2$ |
| $CH_2$ | H | $CH_2$ | X/Y-23 | 5-Cl |
| $CH_2$ | H | $CH_2$ | X/Y-23 | 5-$OCH_2CH_3$ |
| $CH_2$ | H | $CH(CH_3)$ | X/Y-23 | 5-Cyclopropyl |
| $CH_2$ | 3-F | $CH(CH_3)$ | X/Y-23 | 5-$CH_3$ |
| $CH_2$ | H | $CH(CH_3)$ | X/Y-23 | 5-$CH(CH_3)_2$ |
| $CH(CH_3)$ | H | $CH(CH_3)$ | X/Y-23 | 5-Cyclopropyl |
| $CH_2$ | H | $CH_2CH_2$ | X/Y-23 | 5-Cyclopropyl |
| $CH_2CH_2CH_2$ | H | $CH_2$ | X/Y-23 | 5-$CH_3$ |
| $CH_2CH_2$ | H | $CH_2CH_2$ | X/Y-23 | 5-$CH(CH_3)_2$ |
| $CH_2CH_2$ | H | $CH_2CH_2$ | X/Y-23 | 5-Cyclopropyl |
| $CH_2$ | H | $CH_2$ | X/Y-26 | 1-$CH_3$ |
| $CH_2$ | H | $CH_2$ | X/Y-26 | 1,5-$(CH_3)_2$ |
| $CH_2$ | H | $CH_2$ | X/Y-26 | 1-$CH_3$, 5-Cyclopropyl |
| $CH(CH_3)$ | H | $CH(CH_3)$ | X/Y-26 | 1-$CH_3$, 5-Cyclopropyl |
| $CH(CH_3)$ | H | $CH(CH_3)$ | X/Y-26 | 1,5-$(CH_3)_2$ |
| $CH(CH_3)$ | 3-F | $CH_2$ | X/Y-26 | 1-$CH_3$, 5-Cyclopropyl |
| $CH_2CH_2$ | H | $CH_2CH_2$ | X/Y-26 | 1-$CH_3$, 5-Cyclopropyl |
| $CH_2$ | H | $CH_2CH_2$ | X/Y-26 | 1,5-$(CH_3)_2$ |
| $CH_2$ | H | $CH_2$ | X/Y-28 | 1-$CH_3$ |
| $CH_2$ | H | $CH_2$ | X/Y-28 | 1,5-$(CH_3)_2$ |
| $CH_2$ | H | $CH_2$ | X/Y-28 | 1-$CH_3$, 5-Cyclopropyl |

Tabelle B (Forts.)

| A | $R^1$ | B | Y | Substituent an Y |
|---|---|---|---|---|
| $CH(CH_3)$ | H | $CH(CH_3)$ | X/Y-28 | $1-CH_3$, 5-Cyclopropyl |
| $CH(CH_3)$ | H | $CH(CH_3)$ | X/Y-28 | $1,5-(CH_3)_2$ |
| $CH(CH_3)$ | 3-F | $CH_2$ | X/Y-28 | $1-CH_3$, 5-Cyclopropyl |
| $CH_2CH_2$ | H | $CH_2CH_2$ | X/Y-28 | $1-CH_3$, 5-Cyclopropyl |
| $CH_2$ | H | $CH_2CH_2$ | X/Y-28 | $1,5-(CH_3)_2$ |
| $CH_2$ | H | $CH_2$ | X/Y-22 | - |
| $CH_2$ | H | $CH_2$ | X/Y-22 | $3-CH_3$ |
| $CH_2$ | H | $CH_2$ | X/Y-22 | 3-Cyclopropyl |
| $CH_2$ | H | $CH_2$ | X/Y-22 | $3-OCH_3$ |
| $CH(CH_3)$ | H | $CH_2$ | X/Y-22 | $3-CH_3$ |
| $CH_2$ | H | $CH(CH_3)$ | X/Y-22 | 3-Cyclopropyl |
| $CH_2$ | 3-F | $CH(CH_3)$ | X/Y-22 | $3-OCH_3$ |
| $CH_2CH_2$ | H | $CH_2CH_2$ | X/Y-22 | 3-Cyclopropyl |
| $CH_2$ | H | $CH_2CH_2$ | X/Y-22 | $3-CH_3$ |
| $CH_2$ | H | $CH_2$ | X/Y-29 | $1-CH_3$ |
| $CH_2$ | H | $CH_2$ | X/Y-29 | $1,3-(CH_3)_2$ |
| $CH_2$ | H | $CH_2$ | X/Y-29 | $1-CH_3$, 3-Cyclopropyl |
| $CH(CH_3)$ | H | $CH(CH_3)$ | X/Y-29 | $1,3-(CH_3)_2$ |
| $CH(CH_3)$ | 3-F | $CH(CH_3)$ | X/Y-29 | $1-CH_3$, 3-Cyclopropyl |
| $CH_2CH_2$ | H | $CH_2CH_2CH_2$ | X/Y-29 | $1,3-(CH_3)_2$ |
| $CH_2CH_2$ | H | $CH_2CH_2$ | X/Y-29 | $1-CH_3$, 3-Cyclopropyl |
| $CH_2$ | H | $CH_2$ | X/Y-17 | - |
| $CH_2$ | H | $CH_2$ | X/Y-17 | 5-Cyclopropyl |
| $CH_2$ | H | $CH_2$ | X/Y-17 | $5-CH_3$ |
| $CH_2$ | H | $CH_2$ | X/Y-17 | $5-OCH_2CH_3$ |
| $CH_2$ | H | $CH_2$ | X/Y-17 | 5-Cl |
| $CH_2$ | H | $CH_2$ | X/Y-17 | $4-CH_3$ |
| $CH_2$ | H | $CH_2$ | X/Y-17 | 4-Cyclopropyl |
| $CH_2$ | H | $CH_2$ | X/Y-17 | $4-OCH_2CH_3$ |
| $CH_2$ | H | $CH_2$ | X/Y-17 | 4-Cl |
| $CH(CH_3)$ | H | $CH(CH_3)$ | X/Y-17 | 5-Cyclopropyl |
| $CH(CH_3)$ | H | $CH(CH_3)$ | X/Y-17 | 4-Cyclopropyl |
| $CH(CH_3)$ | 3-F | $CH(CH_3)$ | X/Y-17 | $5-OCH_2CH_3$ |
| $CH_2CH_2$ | H | $CH_2CH_2$ | X/Y-17 | 5-Cyclopropyl |
| $CH_2CH_2$ | H | $CH_2CH_2$ | X/Y-17 | 4-Cyclopropyl |

Tabelle B (Forts.)

| A | $R^1$ | B | Y | Substituent an Y |
|---|---|---|---|---|
| $CH_2CH_2$ | H | $CH_2CH_2CH_2$ | X/Y-17 | 5-$CH_3$ |
| $CH_2$ | H | $CH_2$ | X/Y-14 | - |
| $CH_2$ | H | $CH_2$ | X/Y-14 | 5-$CH_3$ |
| $CH_2$ | H | $CH_2$ | X/Y-14 | 5-Cyclopropyl |
| $CH_2$ | H | $CH_2$ | X/Y-14 | 4-$CH_3$ |
| $CH_2$ | H | $CH_2$ | X/Y-14 | 4-Cyclopropyl |
| $CH_2$ | H | $CH_2$ | X/Y-14 | 4-$OCH_2CH_3$ |
| $CH_2$ | H | $CH_2$ | X/Y-14 | 5-$OCH_2CH_3$ |
| $CH_2$ | H | $CH_2$ | X/Y-14 | 5-Cl |
| $CH(CH_3)$ | H | $CH(CH_3)$ | X/Y-14 | 4-Cyclopropyl |
| $CH(CH_3)$ | H | $CH(CH_3)$ | X/Y-14 | 5-Cyclopropyl |
| $CH(CH_3)$ | H | $CH(CH_3)$ | X/Y-14 | 4-$CH_3$ |
| $CH(CH_3)$ | 3-F | $CH(CH_3)$ | X/Y-14 | 4-Cyclopropyl |
| $CH_2$ | 3-F | $CH_2$ | X/Y-14 | 5-$OCH_2CH_3$ |
| $CH_2CH_2$ | H | $CH_2CH_2$ | X/Y-14 | 4-Cyclopropyl |
| $CH_2CH_2$ | H | $CH_2CH_2$ | X/Y-14 | 5-Cyclopropyl |
| $CH_2CH_2$ | H | $CH_2CH_2CH_2$ | X/Y-14 | 5-Cl |
| $CH_2CH_2$ | H | $CH_2CH_2$ | X/Y-14 | 4-$CH_3$ |
| $CH_2$ | H | $CH_2$ | X/Y-20 | 1-$CH_3$ |
| $CH_2$ | H | $CH_2$ | X/Y-20 | 1,5-$(CH_3)_2$ |
| $CH(CH_3)$ | H | $CH(CH_3)$ | X/Y-20 | 1-$CH_3$ |
| $CH(CH_3)$ | 3-F | $CH(CH_3)$ | X/Y-20 | 1,5-$(CH_3)_2$ |
| $CH_2CH_2$ | H | $CH_2CH_2$ | X/Y-20 | 1-$CH_3$ |
| $CH_2CH_2$ | H | $CH_2CH_2$ | X/Y-20 | 1,5-$(CH_3)_2$ |
| $CH(CH_3)CH_2$ | 3-F | $CH(CH_3)CH_2$ | X/Y-20 | 1,5-$(CH_3)_2$ |
| $CH_2$ | H | $CH_2$ | X/Y-3 | - |
| $CH_2$ | H | $CH_2$ | X/Y-3 | 3-$CH_3$ |
| $CH_2$ | H | $CH_2$ | X/Y-3 | 3-$CH_2CH_3$ |
| $CH_2$ | H | $CH_2$ | X/Y-3 | 3-$CH(CH_3)_2$ |
| $CH_2$ | H | $CH_2$ | X/Y-3 | 3-Cyclopropyl |
| $CH_2$ | H | $CH_2$ | X/Y-3 | 3-$OCH_2CH_3$ |
| $CH_2$ | H | $CH_2$ | X/Y-3 | 3-$CH_2OCH_3$ |
| $CH_2$ | H | $CH_2$ | X/Y-3 | 3-$CF_3$ |
| $CH(CH_3)$ | H | $CH_2$ | X/Y-3 | 3-$CH_3$ |
| $CH(CH_3)$ | H | $CH_2$ | X/Y-3 | 3-$CH(CH_3)_2$ |

Tabelle B (Forts.)

| A | $R^1$ | B | Y | Substituent an Y |
|---|---|---|---|---|
| $CH(CH_3)$ | H | $CH_2$ | X/Y-3 | 3-Cyclopropyl |
| $CH_2$ | H | $CH(CH_3)$ | X/Y-3 | 3-$CH_3$ |
| $CH_2$ | H | $CH(CH_3)$ | X/Y-3 | 3-$CH(CH_3)_2$ |
| $CH_2$ | H | $CH(CH_3)$ | X/Y-3 | 3-Cyclopropyl |
| $CH(CH_3)$ | H | $CH(CH_3)$ | X/Y-3 | 3-$CH_3$ |
| $CH(CH_3)$ | 3-F | $CH(CH_3)$ | X/Y-3 | 3-$CH(CH_3)_2$ |
| $CH(CH_3)$ | H | $CH(CH_3)$ | X/Y-3 | 3-Cyclopropyl |
| $CH_2$ | H | $CH_2CH_2$ | X/Y-3 | 3-$CH_3$ |
| $CH_2CH_2$ | H | $CH_2CH_2CH_2$ | X/Y-3 | 3-$CH(CH_3)_2$ |
| $CH_2$ | H | $CH_2CH_2$ | X/Y-3 | 3-Cyclopropyl |
| $CH_2CH_2$ | H | $CH_2CH(CH_3)$ | X/Y-3 | 3-Cyclopropyl |
| $CH_2CH_2$ | H | $CH_2CH(CH_3)$ | X/Y-3 | 3-$CH(CH_3)_2$ |
| $CH_2CH_2CH_2$ | 3-F | $CH(CH_3)CH_2$ | X/Y-3 | 3-$CH_3$ |
| $CH_2$ | H | $CH_2$ | X/Y-5 | - |
| $CH_2$ | H | $CH_2$ | X/Y-5 | 5-$OCH_2CH_3$ |
| $CH_2$ | H | $CH_2$ | X/Y-5 | 5-Cyclopropyl |
| $CH_2$ | H | $CH_2$ | X/Y-5 | 5-$CH_3$ |
| $CH(CH_3)$ | 3-F | $CH(CH_3)$ | X/Y-5 | 5-$CH_3$ |
| $CH_2$ | H | $CH(CH_3)$ | X/Y-5 | 5-Cyclopropyl |
| $CH(CH_3)$ | H | $CH_2$ | X/Y-5 | 5-Cyclopropyl |
| $CH_2$ | H | $CH_2CH_2$ | X/Y-5 | 5-$OCH_2CH_3$ |
| $CH_2CH_2$ | H | $CH_2CH_2CH_2$ | X/Y-5 | 5-$CH_3$ |
| $CH_2$ | H | $CH_2$ | X/Y-4 | - |
| $CH_2$ | H | $CH_2$ | X/Y-4 | 3-$OCH_2CH_3$ |
| $CH_2$ | H | $CH_2$ | X/Y-4 | 5-$OCH_2CH_3$ |
| $CH_2$ | H | $CH_2$ | X/Y-4 | 3-$CH(CH_3)_2$ |
| $CH_2$ | H | $CH_2$ | X/Y-4 | 5-$CH_3$ |
| $CH(CH_3)$ | 3-F | $CH(CH_3)$ | X/Y-4 | 3-$CH(CH_3)_2$ |
| $CH_2$ | H | $CH_2$ | X/Y-11 | - |
| $CH_2$ | H | $CH_2$ | X/Y-11 | 3-$CH_3$ |
| $CH_2$ | H | $CH_2$ | X/Y-11 | 5-$CH_3$ |
| $CH_2$ | H | $CH(CH_3)$ | X/Y-11 | 3-$CH_3$ |
| $CH_2$ | 3-F | $CH(CH_3)$ | X/Y-11 | 5-$CH_3$ |
| $CH(CH_3)$ | H | $CH(CH_3)$ | X/Y-11 | 5-$CH_3$ |
| $CH_2$ | H | $CH_2$ | X/Y-21 | - |

Tabelle B (Forts.)

| A | $R^1$ | B | Y | Substituent an Y |
|---|---|---|---|---|
| $CH_2$ | H | $CH_2$ | X/Y-21 | 4,5-$(CH_3)_2$ |
| $CH_2$ | H | $CH_2$ | X/Y-21 | 4,5-$Br_2$ |
| $CH(CH_3)$ | H | $CH(CH_3)$ | X/Y-21 | 4,5-$Cl_2$ |
| $CH(CH_3)$ | H | $CH(CH_3)$ | X/Y-21 | 4,5-$(CH_3)_2$ |
| $CH(CH_3)$ | H | $CH(CH_3)$ | X/Y-21 | 4,5-$Br_2$ |
| $CH_2$ | H | $CH_2$ | X/Y-30 | – |
| $CH_2$ | H | $CH_2$ | X/Y-30 | 3,5-$(CH_3)_2$ |
| $CH(CH_3)$ | H | $CH(CH_3)$ | X/Y-30 | – |
| $CH(CH_3)$ | H | $CH(CH_3)$ | X/Y-30 | 3,5-$(CH_3)_2$ |
| $CH_2$ | H | $CH_2$ | X/Y-27 | – |
| $CH_2$ | H | $CH_2$ | X/Y-27 | 2-Cl |
| $CH_2$ | H | $CH_2$ | X/Y-27 | 2,5-$(CH_3)_2$ |
| $CH(CH_3)$ | H | $CH(CH_3)$ | X/Y-27 | – |
| $CH(CH_3)$ | H | $CH(CH_3)$ | X/Y-27 | 2-Cl |
| $CH(CH_3)$ | H | $CH(CH_3)$ | X/Y-27 | 2,5-$(CH_3)_2$ |

Tabelle C (zu Struktur I.C).

| A | $R^1$ | B | Y | Substituent an Y |
|---|---|---|---|---|
| $CH_2$ | H | $CH_2$ | X/Y-1 | |
| $CH(CH_3)$ | H | $CH_2$ | X/Y-1 | |
| $CH(CH_2CH_3)$ | H | $CH_2$ | X/Y-1 | |
| $CH_2$ | H | $CH(CH_3)$ | X/Y-1 | |
| $CH_2$ | H | $CH(CH_2CH_3)$ | X/Y-1 | |
| $CH_2$ | 3-$CH_3$ | $CH_2$ | X/Y-1 | |
| $CH_2$ | 3-F | $CH_2$ | X/Y-1 | |
| $CH_2$ | H | $CH_2$ | X/Y-1 | 5-$CH_3$ |
| $CH_2$ | H | $CH_2$ | X/Y-1 | 4-$CH_3$ |
| $CH_2$ | H | $CH_2$ | X/Y-1 | 3-$CH_3$ |
| $CH_2$ | H | $CH_2$ | X/Y-1 | 5-Cyclopropyl |
| $CH_2$ | H | $CH_2$ | X/Y-1 | 4-Cyclopropyl |
| $CH_2$ | H | $CH_2$ | X/Y-1 | 3-Cyclopropyl |
| $CH_2$ | H | $CH_2$ | X/Y-1 | 5-Cl |
| $CH_2$ | H | $CH_2$ | X/Y-1 | 5-Br |
| $CH_2$ | H | $CH_2$ | X/Y-1 | 4-Cl |
| $CH_2$ | H | $CH_2$ | X/Y-1 | 4-Br |
| $CH_2$ | H | $CH_2$ | X/Y-1 | 4,5-Cl,Cl |
| $CH_2$ | H | $CH_2$ | X/Y-1 | 4-$OCH_2CH_3$ |
| $CH(CH_3)$ | H | $CH_2$ | X/Y-1 | 5-Br |
| $CH(CH_3)$ | H | $CH_2$ | X/Y-1 | 4,5-Cl,Cl |
| $CH(CH_3)$ | H | $CH_2$ | X/Y-1 | 4-Cl |
| $CH_2$ | H | $CH(CH_3)$ | X/Y-1 | 5-Br |
| $CH_2$ | H | $CH(CH_3)$ | X/Y-1 | 4,5-Cl,Cl |
| $CH(CH_3)$ | H | $CH(CH_3)$ | X/Y-1 | - |
| $CH(CH_3$ | H | $CH(CH_3)$ | X/Y-1 | 4,5-Cl,Cl |
| $CH(CH_3)$ | 3-F | $CH(CH_3)$ | X/Y-1 | - |
| $CH(CH_3)$ | 3-F | $CH(CH_3)$ | X/Y-1 | 5-Br |
| $CH_2CH_2$ | H | $CH_2$ | X/Y-1 | 5-Br |
| $CH_2CH_2$ | H | $CH_2CH_2$ | X/Y-1 | 4,5-Cl,Cl |
| $CH_2CH_2CH_2$ | H | $CH_2$ | X/Y-1 | 5-Br |
| $CH_2CH_2$ | H | $CH_2CH_2CH_2$ | X/Y-1 | 4,5-Cl,Cl |
| $CH_2CH(CH_3)$ | H | $CH_2CH_2$ | X/Y-1 | 4,5-Cl,Cl |
| $CH_2CH_2$ | 3-F | $CH_2CH(CH_3)$ | X/Y-1 | 5-Br |

Tabelle C (Forts.)

| A | $R^1$ | B | Y | Substituent an Y |
|---|---|---|---|---|
| $CH_2$ | H | $CH_2$ | X/Y-2 | – |
| $CH_2$ | H | $CH_2$ | X/Y-2 | 4-Cyclopropyl |
| $CH_2$ | H | $CH_2$ | X/Y-2 | 5-Cyclopropyl |
| $CH_2$ | H | $CH_2$ | X/Y-2 | 5-Cl |
| $CH_2$ | H | $CH_2$ | X/Y-2 | 4,5-Cl,Cl |
| $CH_2$ | H | $CH_2$ | X/Y-2 | 4,5-Br,Br |
| $CH_2$ | H | $CH_2$ | X/Y-2 | 4-$CH_3$ |
| $CH_2$ | H | $CH_2$ | X/Y-2 | 5-$CH_3$ |
| $CH_2$ | H | $CH_2$ | X/Y-2 | 4-$OCH_3$ |
| $CH_2$ | H | $CH_2$ | X/Y-2 | 5-$OCH_3$ |
| $CH(CH_3)$ | H | $CH_2$ | X/Y-2 | 4,5-Cl,Cl |
| $CH(CH_3)$ | H | $CH_2$ | X/Y-2 | 4,5-Br,Br |
| $CH_2$ | H | $CH(CH_3)$ | X/Y-2 | 4,5-Cl,Cl |
| $CH_2$ | H | $CH(CH_3)$ | X/Y-2 | 4,5-Br,Br |
| $CH(CH_3)$ | H | $CH(CH_3)$ | X/Y-2 | 4,5-Cl,Cl |
| $CH(CH_3)$ | H | $CH(CH_3)$ | X/Y-2 | 4,5-Br,Br |
| $CH(CH_3)$ | 3-F | $CH(CH_3)$ | X/Y-2 | 4,5-Cl,Cl |
| $CH(CH_3)$ | 3-F | $CH(CH_3)$ | X/Y-2 | 4,5-Br,Br |
| $CH_2CH_2$ | H | $CH_2$ | X/Y-2 | 4,5-Cl,Cl |
| $CH_2CH_2$ | H | $CH_2CH_2$ | X/Y-2 | 4,5-Br,Br |
| $CH_2CH_2CH_2$ | H | $CH_2$ | X/Y-2 | 4,5-Cl,Cl |
| $CH_2CH_2$ | H | $CH_2CH_2CH_2$ | X/Y-2 | 4,5-Br,Br |
| $CH_2CH(CH_3)$ | H | $CH_2CH_2$ | X/Y-2 | 4,5-Br,Br |
| $CH_2CH_2$ | 3-F | $CH_2CH(CH_3)$ | X/Y-2 | 4,5-Cl,Cl |
| $CH_2$ | H | $CH_2$ | X/Y-15 | – |
| $CH_2$ | H | $CH_2$ | X/Y-15 | 2-$CH_3$ |
| $CH_2$ | H | $CH_2$ | X/Y-15 | 2-Cyclopropyl |
| $CH_2$ | H | $CH_2$ | X/Y-15 | 2-$OCH_2CH_3$ |
| $CH_2$ | H | $CH_2$ | X/Y-15 | 2-Cl |
| $CH(CH_3)$ | H | $CH_2$ | X/Y-15 | 2-Cyclopropyl |
| $CH(CH_3)$ | H | $CH_2$ | X/Y-15 | 2-$OCH_2CH_3$ |
| $CH_2$ | H | $CH(CH_3)$ | X/Y-15 | 2-Cyclopropyl |
| $CH_2$ | H | $CH(CH_3)$ | X/Y-15 | 2-$OCH_2CH_3$ |
| $CH(CH_3)$ | H | $CH(CH_3)$ | X/Y-15 | 2-Cyclopropyl |
| $CH(CH_3)$ | H | $CH(CH_3)$ | X/Y-15 | 2-$OCH_2CH_3$ |

Tabelle C (Forts.)

| A | $R^1$ | B | Y | Substituent an Y |
|---|---|---|---|---|
| $CH(CH_3)$ | 3-F | $CH(CH_3)$ | X/Y-15 | 2-Cyclopropyl |
| $CH(CH_3)$ | 3-F | $CH(CH_3)$ | X/Y-15 | 2-$OCH_2CH_3$ |
| $CH_2CH_2$ | H | $CH_2$ | X/Y-15 | 2-Cyclopropyl |
| $CH_2CH_2$ | H | $CH_2CH_2$ | X/Y-15 | 2-$OCH_2CH_3$ |
| $CH_2CH_2CH_2$ | H | $CH_2$ | X/Y-15 | 2-Cyclopropyl |
| $CH_2CH_2$ | H | $CH_2CH_2CH_2$ | X/Y-15 | 2-$OCH_2CH_3$ |
| $CH_2CH(CH_3)$ | H | $CH_2CH_2$ | X/Y-15 | 2-Cyclopropyl |
| $CH_2CH_2$ | 3-F | $CH_2CH(CH_3)$ | X/Y-15 | 2-$OCH_2CH_3$ |
| $CH_2$ | H | $CH_2$ | X/Y-12 | – |
| $CH_2$ | H | $CH_2$ | X/Y-12 | 2-$CH_3$ |
| $CH_2$ | H | $CH_2$ | X/Y-12 | 2-Cyclopropyl |
| $CH_2$ | H | $CH_2$ | X/Y-12 | 2-$OCH_2CH_3$ |
| $CH(CH_3)$ | H | $CH_2$ | X/Y-12 | 2-Cyclopropyl |
| $CH(CH_3)$ | H | $CH_2$ | X/Y-12 | 2-$OCH_2CH_3$ |
| $CH_2$ | H | $CH(CH_3)$ | X/Y-12 | 2-Cyclopropyl |
| $CH_2$ | H | $CH(CH_3)$ | X/Y-12 | 2-$OCH_2CH_3$ |
| $CH(CH_3)$ | H | $CH(CH_3)$ | X/Y-12 | 2-Cyclopropyl |
| $CH(CH_3)$ | 3-F | $CH(CH_3)$ | X/Y-12 | 2-$OCH_2CH_3$ |
| $CH_2CH_2$ | H | $CH_2$ | X/Y-12 | 2-Cyclopropyl |
| $CH_2CH_2$ | H | $CH_2CH_2$ | X/Y-12 | 2-$OCH_2CH_3$ |
| $CH_2CH_2CH_2$ | H | $CH_2$ | X/Y-12 | 2-Cyclopropyl |
| $CH_2CH_2$ | H | $CH_2CH_2CH_2$ | X/Y-12 | 2-$OCH_2CH_3$ |
| $CH_2CH(CH_3)$ | H | $CH_2CH_2$ | X/Y-12 | 2-Cyclopropyl |
| $CH_2CH_2$ | 3-F | $CH_2CH(CH_3)$ | X/Y-12 | 2-Cyclopropyl |
| $CH_2$ | H | $CH_2$ | X/Y-18 | 1-$CH_3$ |
| $CH_2$ | H | $CH_2$ | X/Y-18 | 1,2-$(CH_3)_2$ |
| $CH_2$ | H | $CH_2$ | X/Y-18 | 1,4-$(CH_3)_2$ |
| $CH_2$ | H | $CH_2$ | X/Y-18 | 1,2,4-$(CH_3)_2$ |
| $CH_2$ | H | $CH_2$ | X/Y-18 | 1-$CH_3$, 2-Cyclopropyl |
| $CH(CH_3)$ | H | $CH_2$ | X/Y-18 | 1-$CH_3$, 2-Cyclopropyl |
| $CH(CH_3)$ | H | $CH_2$ | X/Y-18 | 1,2,4-$(CH_3)_3$ |
| $CH_2$ | H | $CH(CH_3)$ | X/Y-18 | 1-$CH_3$, 2-Cyclopropyl |
| $CH_2$ | H | $CH(CH_3)$ | X/Y-18 | 1,2,4-$(CH_3)_3$ |
| $CH(CH_3)$ | H | $CH(CH_3)$ | X/Y-18 | 1-$CH_3$, 2-Cyclopropyl |
| $CH(CH_3)$ | 3-F | $CH(CH_3)$ | X/Y-18 | 1,2,4-$(CH_3)_3$ |

Tabelle C (Forts.)

| A | R1 | B | Y | Substituent an Y |
|---|---|---|---|---|
| $CH_2CH_2$ | H | $CH_2$ | X/Y-18 | 1-$CH_3$, 2-Cyclopropyl |
| $CH_2CH_2$ | H | $CH_2CH_2$ | X/Y-18 | 1,2,4-$(CH_3)_2$ |
| $CH_2CH_2CH_2$ | H | $CH_2$ | X/Y-18 | 1-$CH_3$, 2-Cyclopropyl |
| $CH_2CH_2$ | H | $CH_2CH_2CH_2$ | X/Y-18 | 1,2,4-$(CH_3)_2$ |
| $CH_2CH(CH_3)$ | H | $CH_2CH_2$ | X/Y-18 | 1-$CH_3$, 2-Cyclopropyl |
| $CH_2CH_2$ | 3-F | $CH_2CH(CH_3)$ | X/Y-18 | 1,2,4-$(CH_3)_2$ |
| $CH_2$ | H | $CH_2$ | X/Y-19 | 1-$CH_3$ |
| $CH_2$ | H | $CH_2$ | X/Y-19 | 1,2-$(CH_3)_2$ |
| $CH_2$ | H | $CH_2$ | X/Y-19 | 1,5-$(CH_3)_2$ |
| $CH_2$ | H | $CH_2$ | X/Y-19 | 1-$CH_3$, 2-Cyclopropyl |
| $CH(CH_3)$ | H | $CH_2$ | X/Y-19 | 1,2-$(CH_3)_2$ |
| $CH(CH_3)$ | H | $CH_2$ | X/Y-19 | 1-$CH_3$, 2-Cyclopropyl |
| $CH_2$ | H | $CH(CH_3)$ | X/Y-19 | 1,2-$(CH_3)_2$ |
| $CH_2$ | H | $CH(CH_3)$ | X/Y-19 | 1-$CH_3$, 2-Cyclopropyl |
| $CH(CH_3)$ | H | $CH(CH_3)$ | X/Y-19 | 1,2-$(CH_3)_2$ |
| $CH(CH_3)$ | H | $CH(CH_3)$ | X/Y-19 | 1-$CH_3$, 2-Cyclopropyl |
| $CH(CH_3)$ | 3-F | $CH_2$ | X/Y-19 | 1-$CH_3$, 2-Cyclopropyl |
| $CH_2CH_2$ | H | $CH_2$ | X/Y-19 | 1-$CH_3$, 2-Cyclopropyl |
| $CH_2CH_2$ | H | $CH_2CH_2$ | X/Y-19 | 1,2-$(CH_3)_2$ |
| $CH_2CH_2CH_2$ | H | $CH_2$ | X/Y-19 | 1-$CH_3$, 2-Cyclopropyl |
| $CH_2CH_2$ | H | $CH_2CH_2CH_2$ | X/Y-19 | 1,2-$(CH_3)_2$ |
| $CH_2CH(CH_3)$ | H | $CH_2CH_2$ | X/Y-19 | 1-$CH_3$, 2-Cyclopropyl |
| $CH_2CH_2$ | 3-F | $CH_2CH(CH_3)$ | X/Y-19 | 1,2-$(CH_3)_2$ |
| $CH_2$ | H | $CH_2$ | X/Y-16 | – |
| $CH_2$ | H | $CH_2$ | X/Y-16 | 2-$CH_3$ |
| $CH_2$ | H | $CH_2$ | X/Y-16 | 2-Cyclopropyl |
| $CH_2$ | H | $CH_2$ | X/Y-16 | 2-Cl |
| $CH_2$ | H | $CH_2$ | X/Y-16 | 2-$OCH_2CH_3$ |
| $CH(CH_3)$ | H | $CH_2$ | X/Y-16 | 2-$CH_3$ |
| $CH(CH_3)$ | H | $CH_2$ | X/Y-16 | 2-Cyclopropyl |
| $CH_2$ | H | $CH(CH_3)$ | X/Y-16 | 2-$CH_3$ |
| $CH_2$ | H | $CH(CH_3)$ | X/Y-16 | 2-Cyclopropyl |
| $CH_2(CH_3)$ | H | $CH(CH_3)$ | X/Y-16 | 2-$CH_3$ |
| $CH_2(CH_3)$ | H | $CH(CH_3)$ | X/Y-16 | 2-Cyclopropyl |
| $CH_2(CH_3)$ | 3-F | $CH(CH_3)$ | X/Y-16 | 2-Cyclopropyl |

Tabelle C (Forts.)

| A | R1 | B | Y | Substituent an Y |
|---|---|---|---|---|
| $CH_2CH_2$ | H | $CH_2$ | X/Y-16 | 2-Cyclopropyl |
| $CH_2CH_2$ | H | $CH_2CH_2$ | X/Y-16 | 2-$CH_3$ |
| $CH_2CH_2CH_2$ | H | $CH_2$ | X/Y-16 | 2-Cyclopropyl |
| $CH_2CH_2$ | H | $CH_2CH_2CH_2$ | X/Y-16 | 2-$CH_3$ |
| $CH_2CH(CH_3)$ | H | $CH_2CH_2$ | X/Y-16 | 2-Cyclopropyl |
| $CH_2CH_2$ | 3-F | $CH_2CH(CH_3)$ | X/Y-16 | 2-$CH_3$ |
| $CH_2$ | H | $CH_2$ | X/Y-13 | - |
| $CH_2$ | H | $CH_2$ | X/Y-13 | 2-$CH_3$ |
| $CH_2$ | H | $CH_2$ | X/Y-13 | 2-Cyclopropyl |
| $CH_2$ | H | $CH_2$ | X/Y-13 | 2-$OCH_2CH_3$ |
| $CH(CH_3)$ | H | $CH_2$ | X/Y-13 | 2-$OCH_2CH_3$ |
| $CH(CH_3)$ | H | $CH_2$ | X/Y-13 | 2-Cyclopropyl |
| $CH_2$ | H | $CH(CH_3)$ | X/Y-13 | 2-$OCH_2CH_3$ |
| $CH_2$ | H | $CH(CH_3)$ | X/Y-13 | 2-Cyclopropyl |
| $CH(CH_3)$ | H | $CH(CH_3)$ | X/Y-13 | 2-$OCH_2CH_3$ |
| $CH(CH_3)$ | 3-F | $CH(CH_3)$ | X/Y-13 | 2-Cyclopropyl |
| $CH_2CH_2$ | H | $CH_2$ | X/Y-13 | 2-Cyclopropyl |
| $CH_2CH_2$ | H | $CH_2CH_2$ | X/Y-13 | 2-$CH_3$ |
| $CH_2CH_2CH_2$ | H | $CH_2$ | X/Y-13 | 2-Cyclopropyl |
| $CH_2CH_2$ | H | $CH_2CH_2CH_2$ | X/Y-13 | 2-$CH_3$ |
| $CH(CH_3)CH_2$ | 3-F | $CH_2CH_2$ | X/Y-13 | 2-Cyclopropyl |
| $CH_2CH_2$ | H | $CH(CH_3)CH_2$ | X/Y-13 | 2-$CH_3$ |
| $CH_2$ | H | $CH_2$ | X/Y-6 | - |
| $CH_2$ | H | $CH_2$ | X/Y-6 | 3-$CH_3$ |
| $CH_2$ | H | $CH_2$ | X/Y-6 | 3-Cyclopropyl |
| $CH_2$ | H | $CH_2$ | X/Y-6 | 3-$OCH_2CH_3$ |
| $CH(CH_3)$ | H | $CH(CH_3)$ | X/Y-6 | 3-Cyclopropyl |
| $CH(CH_3)$ | 3-F | $CH(CH_3)$ | X/Y-6 | 3-Cyclopropyl |
| $CH_2CH_2$ | H | $CH_2CH_2$ | X/Y-6 | 3-Cyclopropyl |
| $CH_2CH_2$ | H | $CH_2CH_2CH_2$ | X/Y-6 | 3-$CH_3$ |
| $CH(CH_3)CH_2$ | 3-F | $CH_2CH_2$ | X/Y-6 | 3-Cyclopropyl |
| $CH_2CH_2$ | H | $CH(CH_3)CH_2$ | X/Y-6 | 3-Cyclopropyl |
| $CH_2$ | H | $CH_2$ | X/Y-8 | 1-$CH_3$ |
| $CH_2$ | H | $CH_2$ | X/Y-8 | 1,3-$(CH_3)_2$ |
| $CH_2$ | H | $CH_2$ | X/Y-8 | 1,4-$(CH_3)_2$ |

Tabelle C (Forts.)

| A | $R^1$ | B | Y | Substituent an Y |
|---|---|---|---|---|
| $CH_2$ | H | $CH_2$ | X/Y-8 | $1-CH_3$, 3-Cyclopropyl |
| $CH(CH_3)$ | H | $CH(CH_3)$ | X/Y-8 | $1-CH_3$, 3-Cyclopropyl |
| $CH(CH_3)$ | 3-F | $CH(CH_3)$ | X/Y-8 | $1-CH_3$, 3-Cyclopropyl |
| $CH_2CH_2$ | H | $CH_2CH_2$ | X/Y-8 | $1-CH_3$, 3-Cyclopropyl |
| $CH_2CH_2$ | H | $CH_2CH_2CH_2$ | X/Y-8 | $1,3-(CH_3)_2$ |
| $CH_2CH(CH_3)$ | H | $CH_2CH_2$ | X/Y-8 | $1-CH_3$, 3-Cyclopropyl |
| $CH(CH_3)CH_2$ | 3-F | $CH_2CH_2$ | X/Y-8 | $1-CH_3$, 3-Cyclopropyl |
| $CH_2$ | H | $CH_2$ | X/Y-10 | $1-CH_3$ |
| $CH_2$ | H | $CH_2$ | X/Y-10 | $1,4-(CH_3)_2$ |
| $CH_2$ | H | $CH_2$ | X/Y-10 | $1-CH_3$, $4-OCH_3$ |
| $CH_2$ | H | $CH_2$ | X/Y-10 | $1-CH_3$, $5-CH(CH_3)_2$ |
| $CH_2$ | H | $CH_2$ | X/Y-10 | $1-CH_3$, 5-Cyclopropyl |
| $CH(CH_3)$ | H | $CH(CH_3)$ | X/Y-10 | $1-CH_3$, 5-Cyclopropyl |
| $CH(CH_3)$ | 3-F | $CH(CH_3)$ | X/Y-10 | $1,4-(CH_3)_2$ |
| $CH_2CH_2$ | H | $CH_2CH_2$ | X/Y-10 | $1-CH_3$, 5-Cyclopropyl |
| $CH_2CH_2$ | H | $CH_2CH_2CH_2$ | X/Y-10 | $1,5-(CH_3)_2$ |
| $CH_2CH(CH_3)$ | H | $CH_2CH_2$ | X/Y-10 | $1-CH_3$, 5-Cyclopropyl |
| $CH(CH_3)CH_2$ | 3-F | $CH_2CH_2$ | X/Y-10 | $1-CH_3$, 5-Cyclopropyl |
| $CH_2$ | H | $CH_2$ | X/Y-7 | - |
| $CH_2$ | H | $CH_2$ | X/Y-7 | $3-CH_3$ |
| $CH(CH_3)$ | H | $CH(CH_3)$ | X/Y-7 | $3-CH_3$ |
| $CH(CH_3)$ | 3-F | $CH(CH_3)$ | X/Y-7 | $3-CH_3$ |
| $CH_2CH_2$ | H | $CH_2CH_2$ | X/Y-7 | $3-CH_3$ |
| $CH_2CH_2$ | 3-F | $CH(CH_3)CH_2CH_2$ | X/Y-7 | $3-CH_3$ |
| $CH_2$ | H | $CH_2$ | X/Y-9 | $1-CH_3$ |
| $CH_2$ | H | $CH_2$ | X/Y-9 | $1,3-(CH_3)_2$ |
| $CH_2$ | H | $CH_2$ | X/Y-9 | $1,5-(CH_3)_2$ |
| $CH(CH_3)$ | H | $CH(CH_3)$ | X/Y-9 | $1,3-(CH_3)_2$ |
| $CH(CH_3)$ | 3-F | $CH(CH_3)$ | X/Y-9 | $1,5-(CH_3)_2$ |
| $CH_2CH_2$ | H | $CH_2CH_2$ | X/Y-9 | $1,3-(CH_3)_2$ |
| $CH_2CH_2$ | H | $CH_2CH_2CH_2$ | X/Y-9 | $1,5-(CH_3)_2$ |
| $CH_2CH(CH_3)$ | H | $CH_2CH_2$ | X/Y-9 | $1,3-(CH_3)_2$ |
| $CH(CH_3)CH_2$ | 3-F | $CH_2CH_2$ | X/Y-9 | $1,5-(CH_3)_2$ |

Tabelle C (Forts.)

| A | $R^1$ | B | Y | Substituent an Y |
|---|---|---|---|---|
| $CH_2$ | H | $CH_2$ | X/Y-25 | 5-$CH_2CH_3$ |
| $CH_2$ | H | $CH_2$ | X/Y-25 | 5-$CH(CH_3)_2$ |
| $CH_2$ | H | $CH_2$ | X/Y-25 | 5-$OCH_3$ |
| $CH(CH_3)$ | H | $CH(CH_3)$ | X/Y-25 | 5-Cyclopropyl |
| $CH_2(CH_3)$ | H | $CH(CH_3)$ | X/Y-25 | 5-$CH_3$ |
| $CH(CH_3)$ | H | $CH(CH_3)$ | X/Y-25 | 5-$OCH_2CH_3$ |
| $CH(CH_3)$ | 3-F | $CH(CH_3)$ | X/Y-25 | 5-Cyclopropyl |
| $CH_2CH_2$ | H | $CH_2$ | X/Y-25 | 5-Cyclopropyl |
| $CH_2CH_2$ | H | $CH_2CH_2$ | X/Y-25 | 5-$CH_3$ |
| $CH_2CH_2CH_2$ | H | $CH_2$ | X/Y-25 | 5-$OCH_2CH_3$ |
| $CH_2CH_2$ | 3-F | $CH_2CH(CH_3)$ | X/Y-25 | 5-Cyclopropyl |
| $CH_2$ | H | $CH(CH_3)$ | X/Y-25 | 5-Cyclopropyl |
| $CH_2$ | H | $CH_2$ | X/Y-23 | – |
| $CH_2$ | H | $CH_2$ | X/Y-23 | 5-$CH_3$ |
| $CH_2$ | H | $CH_2$ | X/Y-23 | 5-$CH(CH_3)_2$ |
| $CH_2$ | H | $CH_2$ | X/Y-23 | 5-Cl |
| $CH_2$ | H | $CH_2$ | X/Y-23 | 5-$OCH_2CH_3$ |
| $CH_2$ | H | $CH(CH_3)$ | X/Y-23 | 5-Cyclopropyl |
| $CH_2$ | 3-F | $CH(CH_3)$ | X/Y-23 | 5-$CH_3$ |
| $CH_2$ | H | $CH(CH_3)$ | X/Y-23 | 5-$CH(CH_3)_2$ |
| $CH(CH_3)$ | H | $CH(CH_3)$ | X/Y-23 | 5-Cyclopropyl |
| $CH_2$ | H | $CH_2CH_2$ | X/Y-23 | 5-Cyclopropyl |
| $CH_2CH_2CH_2$ | H | $CH_2$ | X/Y-23 | 5-$CH_3$ |
| $CH_2CH_2$ | H | $CH_2CH_2$ | X/Y-23 | 5-$CH(CH_3)_2$ |
| $CH_2CH_2$ | H | $CH_2CH_2$ | X/Y-23 | 5-Cyclopropyl |
| $CH_2$ | H | $CH_2$ | X/Y-26 | 1-$CH_3$ |
| $CH_2$ | H | $CH_2$ | X/Y-26 | 1,5-$(CH_3)_2$ |
| $CH_2$ | H | $CH_2$ | X/Y-26 | 1-$CH_3$, 5-Cyclopropyl |
| $CH(CH_3)$ | H | $CH(CH_3)$ | X/Y-26 | 1-$CH_3$, 5-Cyclopropyl |
| $CH(CH_3)$ | H | $CH(CH_3)$ | X/Y-26 | 1,5-$(CH_3)_2$ |
| $CH(CH_3)$ | 3-F | $CH_2$ | X/Y-26 | 1-$CH_3$, 5-Cyclopropyl |
| $CH_2CH_2$ | H | $CH_2CH_2$ | X/Y-26 | 1-$CH_3$, 5-Cyclopropyl |
| $CH_2$ | H | $CH_2CH_2$ | X/Y-26 | 1,5-$(CH_3)_2$ |
| $CH_2$ | H | $CH_2$ | X/Y-28 | 1-$CH_3$ |
| $CH_2$ | H | $CH_2$ | X/Y-28 | 1,5-$(CH_3)_2$ |
| $CH_2$ | H | $CH_2$ | X/Y-28 | 1-$CH_3$, 5-Cyclopropyl |

Tabelle C (Forts.)

| A | $R^1$ | B | Y | Substituent an Y |
|---|---|---|---|---|
| $CH(CH_3)$ | H | $CH(CH_3)$ | X/Y–28 | 1-$CH_3$, 5-Cyclopropyl |
| $CH(CH_3)$ | H | $CH(CH_3)$ | X/Y–28 | 1,5-$(CH_3)_2$ |
| $CH(CH_3)$ | 3–F | $CH_2$ | X/Y–28 | 1-$CH_3$, 5-Cyclopropyl |
| $CH_2CH_2$ | H | $CH_2CH_2$ | X/Y–28 | 1-$CH_3$, 5-Cyclopropyl |
| $CH_2$ | H | $CH_2CH_2$ | X/Y–28 | 1,5-$(CH_3)_2$ |
| $CH_2$ | H | $CH_2$ | X/Y–22 | – |
| $CH_2$ | H | $CH_2$ | X/Y–22 | 3-$CH_3$ |
| $CH_2$ | H | $CH_2$ | X/Y–22 | 3-Cyclopropyl |
| $CH_2$ | H | $CH_2$ | X/Y–22 | 3-$OCH_3$ |
| $CH(CH_3)$ | H | $CH_2$ | X/Y–22 | 3-$CH_3$ |
| $CH_2$ | H | $CH(CH_3)$ | X/Y–22 | 3-Cyclopropyl |
| $CH_2$ | 3–F | $CH(CH_3)$ | X/Y–22 | 3-$OCH_3$ |
| $CH_2CH_2$ | H | $CH_2CH_2$ | X/Y–22 | 3-Cyclopropyl |
| $CH_2$ | H | $CH_2CH_2$ | X/Y–22 | 3-$CH_3$ |
| $CH_2$ | H | $CH_2$ | X/Y–29 | 1-$CH_3$ |
| $CH_2$ | H | $CH_2$ | X/Y–29 | 1,3-$(CH_3)_2$ |
| $CH_2$ | H | $CH_2$ | X/Y–29 | 1-$CH_3$, 3-Cyclopropyl |
| $CH(CH_3)$ | H | $CH(CH_3)$ | X/Y–29 | 1,3-$(CH_3)_2$ |
| $CH(CH_3)$ | 3–F | $CH(CH_3)$ | X/Y–29 | 1-$CH_3$, 3-Cyclopropyl |
| $CH_2CH_2$ | H | $CH_2CH_2CH_2$ | X/Y–29 | 1,3-$(CH_3)_2$ |
| $CH_2CH_2$ | H | $CH_2CH_2$ | X/Y–29 | 1-$CH_3$, 3-Cyclopropyl |
| $CH_2$ | H | $CH_2$ | X/Y–17 | – |
| $CH_2$ | H | $CH_2$ | X/Y–17 | 5-Cyclopropyl |
| $CH_2$ | H | $CH_2$ | X/Y–17 | 5-$CH_3$ |
| $CH_2$ | H | $CH_2$ | X/Y–17 | 5-$OCH_2CH_3$ |
| $CH_2$ | H | $CH_2$ | X/Y–17 | 5-Cl |
| $CH_2$ | H | $CH_2$ | X/Y–17 | 4-$CH_3$ |
| $CH_2$ | H | $CH_2$ | X/Y–17 | 4-Cyclopropyl |
| $CH_2$ | H | $CH_2$ | X/Y–17 | 4-$OCH_2CH_3$ |
| $CH_2$ | H | $CH_2$ | X/Y–17 | 4-Cl |
| $CH(CH_3)$ | H | $CH(CH_3)$ | X/Y–17 | 5-Cyclopropyl |
| $CH(CH_3)$ | H | $CH(CH_3)$ | X/Y–17 | 4-Cyclopropyl |
| $CH(CH_3)$ | 3–F | $CH(CH_3)$ | X/Y–17 | 5-$OCH_2CH_3$ |
| $CH_2CH_2$ | H | $CH_2CH_2$ | X/Y–17 | 5-Cyclopropyl |
| $CH_2CH_2$ | H | $CH_2CH_2$ | X/Y–17 | 4-Cyclopropyl |

Tabelle C (Forts.)

| A | $R^1$ | B | Y | Substituent an Y |
|---|---|---|---|---|
| $CH_2CH_2$ | H | $CH_2CH_2CH_2$ | X/Y-17 | 5-$CH_3$ |
| $CH_2$ | H | $CH_2$ | X/Y-14 | – |
| $CH_2$ | H | $CH_2$ | X/Y-14 | 5-$CH_3$ |
| $CH_2$ | H | $CH_2$ | X/Y-14 | 5-Cyclopropyl |
| $CH_2$ | H | $CH_2$ | X/Y-14 | 4-$CH_3$ |
| $CH_2$ | H | $CH_2$ | X/Y-14 | 4-Cyclopropyl |
| $CH_2$ | H | $CH_2$ | X/Y-14 | 4-$OCH_2CH_3$ |
| $CH_2$ | H | $CH_2$ | X/Y-14 | 5-$OCH_2CH_3$ |
| $CH_2$ | H | $CH_2$ | X/Y-14 | 5-Cl |
| $CH(CH_3)$ | H | $CH(CH_3)$ | X/Y-14 | 4-Cyclopropyl |
| $CH(CH_3)$ | H | $CH(CH_3)$ | X/Y-14 | 5-Cyclopropyl |
| $CH(CH_3)$ | H | $CH(CH_3)$ | X/Y-14 | 4-$CH_3$ |
| $CH(CH_3)$ | 3-F | $CH(CH_3)$ | X/Y-14 | 4-Cyclopropyl |
| $CH_2$ | 3-F | $CH_2$ | X/Y-14 | 5-$OCH_2CH_3$ |
| $CH_2CH_2$ | H | $CH_2CH_2$ | X/Y-14 | 4-Cyclopropyl |
| $CH_2CH_2$ | H | $CH_2CH_2$ | X/Y-14 | 5-Cyclopropyl |
| $CH_2CH_2$ | H | $CH_2CH_2CH_2$ | X/Y-14 | 5-Cl |
| $CH_2CH_2$ | H | $CH_2CH_2$ | X/Y-14 | 4-$CH_3$ |
| $CH_2$ | H | $CH_2$ | X/Y-20 | 1-$CH_3$ |
| $CH_2$ | H | $CH_2$ | X/Y-20 | 1,5-$(CH_3)_2$ |
| $CH(CH_3)$ | H | $CH(CH_3)$ | X/Y-20 | 1-$CH_3$ |
| $CH(CH_3)$ | 3-F | $CH(CH_3)$ | X/Y-20 | 1,5-$(CH_3)_2$ |
| $CH_2CH_2$ | H | $CH_2CH_2$ | X/Y-20 | 1-$CH_3$ |
| $CH_2CH_2$ | H | $CH_2CH_2$ | X/Y-20 | 1,5-$(CH_3)_2$ |
| $CH(CH_3)CH_2$ | 3-F | $CH(CH_3)CH_2$ | X/Y-20 | 1,5-$(CH_3)_2$ |
| $CH_2$ | H | $CH_2$ | X/Y-3 | – |
| $CH_2$ | H | $CH_2$ | X/Y-3 | 3-$CH_3$ |
| $CH_2$ | H | $CH_2$ | X/Y-3 | 3-$CH_2CH_3$ |
| $CH_2$ | H | $CH_2$ | X/Y-3 | 3-$CH(CH_3)_2$ |
| $CH_2$ | H | $CH_2$ | X/Y-3 | 3-$OCH_2CH_3$ |
| $CH_2$ | H | $CH_2$ | X/Y-3 | 3-$CH_2OCH_3$ |
| $CH_2$ | H | $CH_2$ | X/Y-3 | 3-$CF_3$ |
| $CH(CH_3)$ | H | $CH_2$ | X/Y-3 | 3-$CH_3$ |
| $CH(CH_3)$ | H | $CH_2$ | X/Y-3 | 3-$CH(CH_3)_2$ |

Tabelle C (Forts.)

| A | $R^1$ | B | Y | Substituent an Y |
|---|---|---|---|---|
| $CH(CH_3)$ | H | $CH_2$ | X/Y-3 | 3-Cyclopropyl |
| $CH_2$ | H | $CH(CH_3)$ | X/Y-3 | 3-$CH_3$ |
| $CH_2$ | H | $CH(CH_3)$ | X/Y-3 | 3-$CH(CH_3)_2$ |
| $CH_2$ | H | $CH(CH_3)$ | X/Y-3 | 3-Cyclopropyl |
| $CH(CH_3)$ | H | $CH(CH_3)$ | X/Y-3 | 3-$CH_3$ |
| $CH(CH_3)$ | 3-F | $CH(CH_3)$ | X/Y-3 | 3-$CH(CH_3)_2$ |
| $CH(CH_3)$ | H | $CH(CH_3)$ | X/Y-3 | 3-Cyclopropyl |
| $CH_2$ | H | $CH_2CH_2$ | X/Y-3 | 3-$CH_3$ |
| $CH_2CH_2$ | H | $CH_2CH_2CH_2$ | X/Y-3 | 3-$CH(CH_3)_2$ |
| $CH_2$ | H | $CH_2CH_2$ | X/Y-3 | 3-Cyclopropyl |
| $CH_2CH_2$ | H | $CH_2CH(CH_3)$ | X/Y-3 | 3-Cyclopropyl |
| $CH_2CH_2$ | H | $CH_2CH(CH_3)$ | X/Y-3 | 3-$CH(CH_3)_2$ |
| $CH_2CH_2CH_2$ | 3-F | $CH(CH_3)CH_2$ | X/Y-3 | 3-$CH_3$ |
| $CH_2$ | H | $CH_2$ | X/Y-5 | – |
| $CH_2$ | H | $CH_2$ | X/Y-5 | 5-$OCH_2CH_3$ |
| $CH_2$ | H | $CH_2$ | X/Y-5 | 5-Cyclopropyl |
| $CH_2$ | H | $CH_2$ | X/Y-5 | 5-$CH_3$ |
| $CH(CH_3)$ | 3-F | $CH(CH_3)$ | X/Y-5 | 5-$CH_3$ |
| $CH_2$ | H | $CH(CH_3)$ | X/Y-5 | 5-Cyclopropyl |
| $CH(CH_3)$ | H | $CH_2$ | X/Y-5 | 5-Cyclopropyl |
| $CH_2$ | H | $CH_2CH_2$ | X/Y-5 | 5-$OCH_2CH_3$ |
| $CH_2CH_2$ | H | $CH_2CH_2CH_2$ | X/Y-5 | 5-$CH_3$ |
| $CH_2$ | H | $CH_2$ | X/Y-4 | – |
| $CH_2$ | H | $CH_2$ | X/Y-4 | 3-$OCH_2CH_3$ |
| $CH_2$ | H | $CH_2$ | X/Y-4 | 5-$OCH_2CH_3$ |
| $CH_2$ | H | $CH_2$ | X/Y-4 | 3-$CH(CH_3)_2$ |
| $CH_2$ | H | $CH_2$ | X/Y-4 | 5-$CH_3$ |
| $CH(CH_3)$ | 3-F | $CH(CH_3)$ | X/Y-4 | 3-$CH(CH_3)_2$ |
| $CH_2$ | H | $CH_2$ | X/Y-11 | – |
| $CH_2$ | H | $CH_2$ | X/Y-11 | 3-$CH_3$ |
| $CH_2$ | H | $CH_2$ | X/Y-11 | 5-$CH_3$ |
| $CH_2$ | H | $CH(CH_3)$ | X/Y-11 | 3-$CH_3$ |
| $CH_2$ | 3-F | $CH(CH_3)$ | X/Y-11 | 5-$CH_3$ |
| $CH(CH_3)$ | H | $CH(CH_3)$ | X/Y-11 | 5-$CH_3$ |
| $CH_2$ | H | $CH_2$ | X/Y-21 | – |

Tabelle C (Forts.)

| A | $R^1$ | B | Y | Substituent an Y |
|---|---|---|---|---|
| $CH_2$ | H | $CH_2$ | X/Y-21 | 4,5-$(CH_3)_2$ |
| $CH_2$ | H | $CH_2$ | X/Y-21 | 4,5-$Br_2$ |
| $CH(CH_3)$ | H | $CH(CH_3)$ | X/Y-21 | 4,5-$Cl_2$ |
| $CH(CH_3)$ | H | $CH(CH_3)$ | X/Y-21 | 4,5-$(CH_3)_2$ |
| $CH(CH_3)$ | H | $CH(CH_3)$ | X/Y-21 | 4,5-$Br_2$ |
| $CH_2$ | H | $CH_2$ | X/Y-30 | – |
| $CH_2$ | H | $CH_2$ | X/Y-30 | 3,5-$(CH_3)_2$ |
| $CH(CH_3)$ | H | $CH(CH_3)$ | X/Y-30 | – |
| $CH(CH_3)$ | H | $CH(CH_3)$ | X/Y-30 | 3,5-$(CH_3)_2$ |
| $CH_2$ | H | $CH_2$ | X/Y-27 | – |
| $CH_2$ | H | $CH_2$ | X/Y-27 | 2-Cl |
| $CH_2$ | H | $CH_2$ | X/Y-27 | 2,5-$(CH_3)_2$ |
| $CH(CH_3)$ | H | $CH(CH_3)$ | X/Y-27 | – |
| $CH(CH_3)$ | H | $CH(CH_3)$ | X/Y-27 | 2-Cl |
| $CH(CH_3)$ | H | $CH(CH_3)$ | X/Y-27 | 2,5-$(CH_3)_2$ |

Tabelle D (zu Struktur I.D).

| A | $R^1$ | B | Y | Substituent an Y |
|---|---|---|---|---|
| $CH_2$ | H | $CH_2$ | X/Y-1 | |
| $CH(CH_3)$ | H | $CH_2$ | X/Y-1 | |
| $CH(CH_2CH_3)$ | H | $CH_2$ | X/Y-1 | |
| $CH_2$ | H | $CH(CH_3)$ | X/Y-1 | |
| $CH_2$ | H | $CH(CH_2CH_3)$ | X/Y-1 | |
| $CH_2$ | 3-$CH_3$ | $CH_2$ | X/Y-1 | |
| $CH_2$ | 3-F | $CH_2$ | X/Y-1 | |
| $CH_2$ | H | $CH_2$ | X/Y-1 | 5-$CH_3$ |
| $CH_2$ | H | $CH_2$ | X/Y-1 | 4-$CH_3$ |
| $CH_2$ | H | $CH_2$ | X/Y-1 | 3-$CH_3$ |
| $CH_2$ | H | $CH_2$ | X/Y-1 | 5-Cyclopropyl |
| $CH_2$ | H | $CH_2$ | X/Y-1 | 4-Cyclopropyl |
| $CH_2$ | H | $CH_2$ | X/Y-1 | 3-Cyclopropyl |
| $CH_2$ | H | $CH_2$ | X/Y-1 | 5-Cl |
| $CH_2$ | H | $CH_2$ | X/Y-1 | 5-Br |
| $CH_2$ | H | $CH_2$ | X/Y-1 | 4-Cl |
| $CH_2$ | H | $CH_2$ | X/Y-1 | 4-Br |
| $CH_2$ | H | $CH_2$ | X/Y-1 | 4,5-Cl,Cl |
| $CH_2$ | H | $CH_2$ | X/Y-1 | 4-$OCH_2CH_3$ |
| $CH(CH_3)$ | H | $CH_2$ | X/Y-1 | 5-Br |
| $CH(CH_3)$ | H | $CH_2$ | X/Y-1 | 4,5-Cl,Cl |
| $CH(CH_3)$ | H | $CH_2$ | X/Y-1 | 4-Cl |
| $CH_2$ | H | $CH(CH_3)$ | X/Y-1 | 5-Br |
| $CH_2$ | H | $CH(CH_3)$ | X/Y-1 | 4,5-Cl,Cl |
| $CH(CH_3)$ | H | $CH(CH_3)$ | X/Y-1 | – |
| $CH(CH_3$ | H | $CH(CH_3)$ | X/Y-1 | 4,5-Cl,Cl |
| $CH(CH_3)$ | 3-F | $CH(CH_3)$ | X/Y-1 | – |
| $CH(CH_3)$ | 3-F | $CH(CH_3)$ | X/Y-1 | 5-Br |
| $CH_2CH_2$ | H | $CH_2$ | X/Y-1 | 5-Br |
| $CH_2CH_2$ | H | $CH_2CH_2$ | X/Y-1 | 4,5-Cl,Cl |
| $CH_2CH_2CH_2$ | H | $CH_2$ | X/Y-1 | 5-Br |
| $CH_2CH_2$ | H | $CH_2CH_2CH_2$ | X/Y-1 | 4,5-Cl,Cl |
| $CH_2CH(CH_3)$ | H | $CH_2CH_2$ | X/Y-1 | 4,5-Cl,Cl |
| $CH_2CH_2$ | 3-F | $CH_2CH(CH_3)$ | X/Y-1 | 5-Br |

Tabelle D (Forts.)

| A | $R^1$ | B | Y | Substituent an Y |
|---|---|---|---|---|
| $CH_2$ | H | $CH_2$ | X/Y-2 | – |
| $CH_2$ | H | $CH_2$ | X/Y-2 | 4-Cyclopropyl |
| $CH_2$ | H | $CH_2$ | X/Y-2 | 5-Cyclopropyl |
| $CH_2$ | H | $CH_2$ | X/Y-2 | 5-Cl |
| $CH_2$ | H | $CH_2$ | X/Y-2 | 4,5-Cl,Cl |
| $CH_2$ | H | $CH_2$ | X/Y-2 | 4,5-Br,Br |
| $CH_2$ | H | $CH_2$ | X/Y-2 | 4-$CH_3$ |
| $CH_2$ | H | $CH_2$ | X/Y-2 | 5-$CH_3$ |
| $CH_2$ | H | $CH_2$ | X/Y-2 | 4-$OCH_3$ |
| $CH_2$ | H | $CH_2$ | X/Y-2 | 5-$OCH_3$ |
| $CH(CH_3)$ | H | $CH_2$ | X/Y-2 | 4,5-Cl,Cl |
| $CH(CH_3)$ | H | $CH_2$ | X/Y-2 | 4,5-Br,Br |
| $CH_2$ | H | $CH(CH_3)$ | X/Y-2 | 4,5-Cl,Cl |
| $CH_2$ | H | $CH(CH_3)$ | X/Y-2 | 4,5-Br,Br |
| $CH(CH_3)$ | H | $CH(CH_3)$ | X/Y-2 | 4,5-Cl,Cl |
| $CH(CH_3)$ | H | $CH(CH_3)$ | X/Y-2 | 4,5-Br,Br |
| $CH(CH_3)$ | 3-F | $CH(CH_3)$ | X/Y-2 | 4,5-Cl,Cl |
| $CH(CH_3)$ | 3-F | $CH(CH_3)$ | X/Y-2 | 4,5-Br,Br |
| $CH_2CH_2$ | H | $CH_2$ | X/Y-2 | 4,5-Cl,Cl |
| $CH_2CH_2$ | H | $CH_2CH_2$ | X/Y-2 | 4,5-Br,Br |
| $CH_2CH_2CH_2$ | H | $CH_2$ | X/Y-2 | 4,5-Cl,Cl |
| $CH_2CH_2$ | H | $CH_2CH_2CH_2$ | X/Y-2 | 4,5-Br,Br |
| $CH_2CH(CH_3)$ | H | $CH_2CH_2$ | X/Y-2 | 4,5-Br,Br |
| $CH_2CH_2$ | 3-F | $CH_2CH(CH_3)$ | X/Y-2 | 4,5-Cl,Cl |
| $CH_2$ | H | $CH_2$ | X/Y-15 | – |
| $CH_2$ | H | $CH_2$ | X/Y-15 | 2-$CH_3$ |
| $CH_2$ | H | $CH_2$ | X/Y-15 | 2-Cyclopropyl |
| $CH_2$ | H | $CH_2$ | X/Y-15 | 2-$OCH_2CH_3$ |
| $CH_2$ | H | $CH_2$ | X/Y-15 | 2-Cl |
| $CH(CH_3)$ | H | $CH_2$ | X/Y-15 | 2-Cyclopropyl |
| $CH(CH_3)$ | H | $CH_2$ | X/Y-15 | 2-$OCH_2CH_3$ |
| $CH_2$ | H | $CH(CH_3)$ | X/Y-15 | 2-Cyclopropyl |
| $CH_2$ | H | $CH(CH_3)$ | X/Y-15 | 2-$OCH_2CH_3$ |
| $CH(CH_3)$ | H | $CH(CH_3)$ | X/Y-15 | 2-Cyclopropyl |
| $CH(CH_3)$ | H | $CH(CH_3)$ | X/Y-15 | 2-$OCH_2CH_3$ |

Tabelle D (Forts.)

| A | $R^1$ | B | Y | Substituent an Y |
|---|---|---|---|---|
| $CH(CH_3)$ | 3-F | $CH(CH_3)$ | X/Y-15 | 2-Cyclopropyl |
| $CH(CH_3)$ | 3-F | $CH(CH_3)$ | X/Y-15 | 2-$OCH_2CH_3$ |
| $CH_2CH_2$ | H | $CH_2$ | X/Y-15 | 2-Cyclopropyl |
| $CH_2CH_2$ | H | $CH_2CH_2$ | X/Y-15 | 2-$OCH_2CH_3$ |
| $CH_2CH_2CH_2$ | H | $CH_2$ | X/Y-15 | 2-Cyclopropyl |
| $CH_2CH_2$ | H | $CH_2CH_2CH_2$ | X/Y-15 | 2-$OCH_2CH_3$ |
| $CH_2CH(CH_3)$ | H | $CH_2CH_2$ | X/Y-15 | 2-Cyclopropyl |
| $CH_2CH_2$ | 3-F | $CH_2CH(CH_3)$ | X/Y-15 | 2-$OCH_2CH_3$ |
| $CH_2$ | H | $CH_2$ | X/Y-12 | – |
| $CH_2$ | H | $CH_2$ | X/Y-12 | 2-$CH_3$ |
| $CH_2$ | H | $CH_2$ | X/Y-12 | 2-Cyclopropyl |
| $CH_2$ | H | $CH_2$ | X/Y-12 | 2-$OCH_2CH_3$ |
| $CH(CH_3)$ | H | $CH_2$ | X/Y-12 | 2-Cyclopropyl |
| $CH(CH_3)$ | H | $CH_2$ | X/Y-12 | 2-$OCH_2CH_3$ |
| $CH_2$ | H | $CH(CH_3)$ | X/Y-12 | 2-Cyclopropyl |
| $CH_2$ | H | $CH(CH_3)$ | X/Y-12 | 2-$OCH_2CH_3$ |
| $CH(CH_3)$ | H | $CH(CH_3)$ | X/Y-12 | 2-Cyclopropyl |
| $CH(CH_3)$ | 3-F | $CH(CH_3)$ | X/Y-12 | 2-$OCH_2CH_3$ |
| $CH_2CH_2$ | H | $CH_2$ | X/Y-12 | 2-Cyclopropyl |
| $CH_2CH_2$ | H | $CH_2CH_2$ | X/Y-12 | 2-$OCH_2CH_3$ |
| $CH_2CH_2CH_2$ | H | $CH_2$ | X/Y-12 | 2-Cyclopropyl |
| $CH_2CH_2$ | H | $CH_2CH_2CH_2$ | X/Y-12 | 2-$OCH_2CH_3$ |
| $CH_2CH(CH_3)$ | H | $CH_2CH_2$ | X/Y-12 | 2-Cyclopropyl |
| $CH_2CH_2$ | 3-F | $CH_2CH(CH_3)$ | X/Y-12 | 2-Cyclopropyl |
| $CH_2$ | H | $CH_2$ | X/Y-18 | 1-$CH_3$ |
| $CH_2$ | H | $CH_2$ | X/Y-18 | 1,2-$(CH_3)_2$ |
| $CH_2$ | H | $CH_2$ | X/Y-18 | 1,4-$(CH_3)_2$ |
| $CH_2$ | H | $CH_2$ | X/Y-18 | 1,2,4-$(CH_3)_2$ |
| $CH_2$ | H | $CH_2$ | X/Y-18 | 1-$CH_3$, 2-Cyclopropyl |
| $CH_2$ | H | $CH_2$ | X/Y-18 | 1-$CH_3$, 2-Cyclopropyl |
| $CH(CH_3)$ | H | $CH_2$ | X/Y-18 | 1,2,4-$(CH_3)_3$ |
| $CH(CH_3)$ | H | $CH_2$ | X/Y-18 | 1-$CH_3$, 2-Cyclopropyl |
| $CH_2$ | H | $CH(CH_3)$ | X/Y-18 | 1,2,4-$(CH_3)_3$ |
| $CH_2$ | H | $CH(CH_3)$ | X/Y-18 | 1-$CH_3$, 2-Cyclopropyl |
| $CH(CH_3)$ | H | $CH(CH_3)$ | X/Y-18 | 1,2,4-$(CH_3)_3$ |
| $CH(CH_3)$ | 3-F | $CH(CH_3)$ | X/Y-18 | 1,2,4-$(CH_3)_3$ |

Tabelle D (Forts.)

| A | R1 | B | Y | Substituent an Y |
|---|---|---|---|---|
| $CH_2CH_2$ | H | $CH_2$ | X/Y-18 | 1-$CH_3$, 2-Cyclopropyl |
| $CH_2CH_2$ | H | $CH_2CH_2$ | X/Y-18 | 1,2,4-$(CH_3)_2$ |
| $CH_2CH_2CH_2$ | H | $CH_2$ | X/Y-18 | 1-$CH_3$, 2-Cyclopropyl |
| $CH_2CH_2$ | H | $CH_2CH_2CH_2$ | X/Y-18 | 1,2,4-$(CH_3)_2$ |
| $CH_2CH(CH_3)$ | H | $CH_2CH_2$ | X/Y-18 | 1-$CH_3$, 2-Cyclopropyl |
| $CH_2CH_2$ | 3-F | $CH_2CH(CH_3)$ | X/Y-18 | 1,2,4-$(CH_3)_2$ |
| $CH_2$ | H | $CH_2$ | X/Y-19 | 1-$CH_3$ |
| $CH_2$ | H | $CH_2$ | X/Y-19 | 1,2-$(CH_3)_2$ |
| $CH_2$ | H | $CH_2$ | X/Y-19 | 1,5-$(CH_3)_2$ |
| $CH_2$ | H | $CH_2$ | X/Y-19 | 1-$CH_3$, 2-Cyclopropyl |
| $CH(CH_3)$ | H | $CH_2$ | X/Y-19 | 1,2-$(CH_3)_2$ |
| $CH(CH_3)$ | H | $CH_2$ | X/Y-19 | 1-$CH_3$, 2-Cyclopropyl |
| $CH_2$ | H | $CH(CH_3)$ | X/Y-19 | 1,2-$(CH_3)_2$ |
| $CH_2$ | H | $CH(CH_3)$ | X/Y-19 | 1-$CH_3$, 2-Cyclopropyl |
| $CH(CH_3)$ | H | $CH(CH_3)$ | X/Y-19 | 1,2-$(CH_3)_2$ |
| $CH(CH_3)$ | H | $CH(CH_3)$ | X/Y-19 | 1-$CH_3$, 2-Cyclopropyl |
| $CH(CH_3)$ | 3-F | $CH_2$ | X/Y-19 | 1-$CH_3$, 2-Cyclopropyl |
| $CH_2CH_2$ | H | $CH_2$ | X/Y-19 | 1-$CH_3$, 2-Cyclopropyl |
| $CH_2CH_2$ | H | $CH_2CH_2$ | X/Y-19 | 1,2-$(CH_3)_2$ |
| $CH_2CH_2CH_2$ | H | $CH_2$ | X/Y-19 | 1-$CH_3$, 2-Cyclopropyl |
| $CH_2CH_2$ | H | $CH_2CH_2CH_2$ | X/Y-19 | 1,2-$(CH_3)_2$ |
| $CH_2CH(CH_3)$ | H | $CH_2CH_2$ | X/Y-19 | 1-$CH_3$, 2-Cyclopropyl |
| $CH_2CH_2$ | 3-F | $CH_2CH(CH_3)$ | X/Y-19 | 1,2-$(CH_3)_2$ |
| $CH_2$ | H | $CH_2$ | X/Y-16 | – |
| $CH_2$ | H | $CH_2$ | X/Y-16 | 2-$CH_3$ |
| $CH_2$ | H | $CH_2$ | X/Y-16 | 2-Cyclopropyl |
| $CH_2$ | H | $CH_2$ | X/Y-16 | 2-Cl |
| $CH_2$ | H | $CH_2$ | X/Y-16 | 2-$OCH_2CH_3$ |
| $CH(CH_3)$ | H | $CH_2$ | X/Y-16 | 2-$CH_3$ |
| $CH(CH_3)$ | H | $CH_2$ | X/Y-16 | 2-Cyclopropyl |
| $CH_2$ | H | $CH(CH_3)$ | X/Y-16 | 2-$CH_3$ |
| $CH_2$ | H | $CH(CH_3)$ | X/Y-16 | 2-Cyclopropyl |
| $CH_2(CH_3)$ | H | $CH(CH_3)$ | X/Y-16 | 2-$CH_3$ |
| $CH_2(CH_3)$ | H | $CH(CH_3)$ | X/Y-16 | 2-Cyclopropyl |
| $CH_2(CH_3)$ | 3-F | $CH(CH_3)$ | X/Y-16 | 2-Cyclopropyl |

Tabelle D (Forts.)

| A | R1 | B | Y | Substituent an Y |
|---|---|---|---|---|
| $CH_2CH_2$ | H | $CH_2$ | X/Y-16 | 2-Cyclopropyl |
| $CH_2CH_2$ | H | $CH_2CH_2$ | X/Y-16 | $2-CH_3$ |
| $CH_2CH_2CH_2$ | H | $CH_2$ | X/Y-16 | 2-Cyclopropyl |
| $CH_2CH_2$ | H | $CH_2CH_2CH_2$ | X/Y-16 | $2-CH_3$ |
| $CH_2CH(CH_3)$ | H | $CH_2CH_2$ | X/Y-16 | 2-Cyclopropyl |
| $CH_2CH_2$ | 3-F | $CH_2CH(CH_3)$ | X/Y-16 | $2-CH_3$ |
| $CH_2$ | H | $CH_2$ | X/Y-13 | - |
| $CH_2$ | H | $CH_2$ | X/Y-13 | $2-CH_3$ |
| $CH_2$ | H | $CH_2$ | X/Y-13 | 2-Cyclopropyl |
| $CH_2$ | H | $CH_2$ | X/Y-13 | $2-OCH_2CH_3$ |
| $CH(CH_3)$ | H | $CH_2$ | X/Y-13 | $2-OCH_2CH_3$ |
| $CH(CH_3)$ | H | $CH_2$ | X/Y-13 | 2-Cyclopropyl |
| $CH_2$ | H | $CH(CH_3)$ | X/Y-13 | $2-OCH_2CH_3$ |
| $CH_2$ | H | $CH(CH_3)$ | X/Y-13 | 2-Cyclopropyl |
| $CH(CH_3)$ | H | $CH(CH_3)$ | X/Y-13 | $2-OCH_2CH_3$ |
| $CH(CH_3)$ | 3-F | $CH(CH_3)$ | X/Y-13 | 2-Cyclopropyl |
| $CH_2CH_2$ | H | $CH_2$ | X/Y-13 | 2-Cyclopropyl |
| $CH_2CH_2$ | H | $CH_2CH_2$ | X/Y-13 | $2-CH_3$ |
| $CH_2CH_2CH_2$ | H | $CH_2$ | X/Y-13 | 2-Cyclopropyl |
| $CH_2CH_2$ | H | $CH_2CH_2CH_2$ | X/Y-13 | $2-CH_3$ |
| $CH(CH_3)CH_2$ | 3-F | $CH_2CH_2$ | X/Y-13 | 2-Cyclopropyl |
| $CH_2CH_2$ | H | $CH(CH_3)CH_2$ | X/Y-13 | $2-CH_3$ |
| $CH_2$ | H | $CH_2$ | X/Y-6 | - |
| $CH_2$ | H | $CH_2$ | X/Y-6 | $3-CH_3$ |
| $CH_2$ | H | $CH_2$ | X/Y-6 | 3-Cyclopropyl |
| $CH_2$ | H | $CH_2$ | X/Y-6 | $3-OCH_2CH_3$ |
| $CH(CH_3)$ | H | $CH(CH_3)$ | X/Y-6 | 3-Cyclopropyl |
| $CH(CH_3)$ | 3-F | $CH(CH_3)$ | X/Y-6 | 3-Cyclopropyl |
| $CH_2CH_2$ | H | $CH_2CH_2$ | X/Y-6 | 3-Cyclopropyl |
| $CH_2CH_2$ | H | $CH_2CH_2CH_2$ | X/Y-6 | $3-CH_3$ |
| $CH(CH_3)CH_2$ | 3-F | $CH_2CH_2$ | X/Y-6 | 3-Cyclopropyl |
| $CH_2CH_2$ | H | $CH(CH_3)CH_2$ | X/Y-6 | 3-Cyclopropyl |
| $CH_2$ | H | $CH_2$ | X/Y-8 | $1-CH_3$ |
| $CH_2$ | H | $CH_2$ | X/Y-8 | $1,3-(CH_3)_2$ |
| $CH_2$ | H | $CH_2$ | X/Y-8 | $1,4-(CH_3)_2$ |

Tabelle D (Forts.)

| A | R1 | B | Y | Substituent an Y |
|---|---|---|---|---|
| $CH_2$ | H | $CH_2$ | X/Y-8 | 1-$CH_3$, 3-Cyclopropyl |
| $CH(CH_3)$ | H | $CH(CH_3)$ | X/Y-8 | 1-$CH_3$, 3-Cyclopropyl |
| $CH(CH_3)$ | 3-F | $CH(CH_3)$ | X/Y-8 | 1-$CH_3$, 3-Cyclopropyl |
| $CH_2CH_2$ | H | $CH_2CH_2$ | X/Y-8 | 1-$CH_3$, 3-Cyclopropyl |
| $CH_2CH_2$ | H | $CH_2CH_2CH_2$ | X/Y-8 | 1,3-$(CH_3)_2$ |
| $CH_2CH(CH_3)$ | H | $CH_2CH_2$ | X/Y-8 | 1-$CH_3$, 3-Cyclopropyl |
| $CH(CH_3)CH_2$ | 3-F | $CH_2CH_2$ | X/Y-8 | 1-$CH_3$, 3-Cyclopropyl |
| $CH_2$ | H | $CH_2$ | X/Y-10 | 1-$CH_3$ |
| $CH_2$ | H | $CH_2$ | X/Y-10 | 1,4-$(CH_3)_2$ |
| $CH_2$ | H | $CH_2$ | X/Y-10 | 1-$CH_3$, 4-$OCH_3$ |
| $CH_2$ | H | $CH_2$ | X/Y-10 | 1-$CH_3$, 5-$CH(CH_3)_2$ |
| $CH_2$ | H | $CH_2$ | X/Y-10 | 1-$CH_3$, 5-Cyclopropyl |
| $CH(CH_3)$ | H | $CH(CH_3)$ | X/Y-10 | 1-$CH_3$, 5-Cyclopropyl |
| $CH(CH_3)$ | 3-F | $CH(CH_3)$ | X/Y-10 | 1,4-$(CH_3)_2$ |
| $CH_2CH_2$ | H | $CH_2CH_2$ | X/Y-10 | 1-$CH_3$, 5-Cyclopropyl |
| $CH_2CH_2$ | H | $CH_2CH_2CH_2$ | X/Y-10 | 1,5-$(CH_3)_2$ |
| $CH_2CH(CH_3)$ | H | $CH_2CH_2$ | X/Y-10 | 1-$CH_3$, 5-Cyclopropyl |
| $CH(CH_3)CH_2$ | 3-F | $CH_2CH_2$ | X/Y-10 | 1-$CH_3$, 5-Cyclopropyl |
| $CH_2$ | H | $CH_2$ | X/Y-7 | - |
| $CH_2$ | H | $CH_2$ | X/Y-7 | 3-$CH_3$ |
| $CH(CH_3)$ | H | $CH(CH_3)$ | X/Y-7 | 3-$CH_3$ |
| $CH(CH_3)$ | 3-F | $CH(CH_3)$ | X/Y-7 | 3-$CH_3$ |
| $CH_2CH_2$ | H | $CH_2CH_2$ | X/Y-7 | 3-$CH_3$ |
| $CH_2CH_2$ | 3-F | $CH(CH_3)CH_2CH_2$ | X/Y-7 | 3-$CH_3$ |
| $CH_2$ | H | $CH_2$ | X/Y-9 | 1-$CH_3$ |
| $CH_2$ | H | $CH_2$ | X/Y-9 | 1,3-$(CH_3)_2$ |
| $CH_2$ | H | $CH_2$ | X/Y-9 | 1,5-$(CH_3)_2$ |
| $CH(CH_3)$ | H | $CH(CH_3)$ | X/Y-9 | 1,3-$(CH_3)_2$ |
| $CH(CH_3)$ | 3-F | $CH(CH_3)$ | X/Y-9 | 1,5-$(CH_3)_2$ |
| $CH_2CH_2$ | H | $CH_2CH_2$ | X/Y-9 | 1,3-$(CH_3)_2$ |
| $CH_2CH_2$ | H | $CH_2CH_2CH_2$ | X/Y-9 | 1,5-$(CH_3)_2$ |
| $CH_2CH(CH_3)$ | H | $CH_2CH_2$ | X/Y-9 | 1,3-$(CH_3)_2$ |
| $CH(CH_3)CH_2$ | 3-F | $CH_2CH_2$ | X/Y-9 | 1,5-$(CH_3)_2$ |
| $CH_2$ | H | $CH_2$ | X/Y-25 | 5-$CH_3$ |

Tabelle D (Forts.)

| A | $R^1$ | B | Y | Substituent an Y |
|---|---|---|---|---|
| $CH_2$ | H | $CH_2$ | X/Y-25 | 5-$CH_2CH_3$ |
| $CH_2$ | H | $CH_2$ | X/Y-25 | 5-$CH(CH_3)_2$ |
| $CH_2$ | H | $CH_2$ | X/Y-25 | 5-$OCH_3$ |
| $CH(CH_3)$ | H | $CH(CH_3)$ | X/Y-25 | 5-Cyclopropyl |
| $CH_2(CH_3)$ | H | $CH(CH_3)$ | X/Y-25 | 5-$CH_3$ |
| $CH(CH_3)$ | H | $CH(CH_3)$ | X/Y-25 | 5-$OCH_2CH_3$ |
| $CH(CH_3)$ | 3-F | $CH(CH_3)$ | X/Y-25 | 5-Cyclopropyl |
| $CH_2CH_2$ | H | $CH_2$ | X/Y-25 | 5-Cyclopropyl |
| $CH_2CH_2$ | H | $CH_2CH_2$ | X/Y-25 | 5-$CH_3$ |
| $CH_2CH_2CH_2$ | H | $CH_2$ | X/Y-25 | 5-$OCH_2CH_3$ |
| $CH_2CH_2$ | 3-F | $CH_2CH(CH_3)$ | X/Y-25 | 5-Cyclopropyl |
| $CH_2$ | H | $CH(CH_3)$ | X/Y-25 | 5-Cyclopropyl |
| $CH_2$ | H | $CH_2$ | X/Y-23 | – |
| $CH_2$ | H | $CH_2$ | X/Y-23 | 5-Cyclopropyl |
| $CH_2$ | H | $CH_2$ | X/Y-23 | 5-$CH_3$ |
| $CH_2$ | H | $CH_2$ | X/Y-23 | 5-$CH(CH_3)_2$ |
| $CH_2$ | H | $CH_2$ | X/Y-23 | 5-Cl |
| $CH_2$ | H | $CH_2$ | X/Y-23 | 5-$OCH_2CH_3$ |
| $CH_2$ | H | $CH(CH_3)$ | X/Y-23 | 5-Cyclopropyl |
| $CH_2$ | 3-F | $CH(CH_3)$ | X/Y-23 | 5-$CH_3$ |
| $CH_2$ | H | $CH(CH_3)$ | X/Y-23 | 5-$CH(CH_3)_2$ |
| $CH(CH_3)$ | H | $CH(CH_3)$ | X/Y-23 | 5-Cyclopropyl |
| $CH_2$ | H | $CH_2CH_2$ | X/Y-23 | 5-Cyclopropyl |
| $CH_2CH_2CH_2$ | H | $CH_2$ | X/Y-23 | 5-$CH_3$ |
| $CH_2CH_2$ | H | $CH_2CH_2$ | X/Y-23 | 5-$CH(CH_3)_2$ |
| $CH_2CH_2$ | H | $CH_2CH_2$ | X/Y-23 | 5-Cyclopropyl |
| $CH_2$ | H | $CH_2$ | X/Y-26 | 1-$CH_3$ |
| $CH_2$ | H | $CH_2$ | X/Y-26 | 1,5-$(CH_3)_2$ |
| $CH_2$ | H | $CH_2$ | X/Y-26 | 1-$CH_3$, 5-Cyclopropyl |
| $CH(CH_3)$ | H | $CH(CH_3)$ | X/Y-26 | 1-$CH_3$, 5-Cyclopropyl |
| $CH(CH_3)$ | H | $CH(CH_3)$ | X/Y-26 | 1,5-$(CH_3)_2$ |
| $CH(CH_3)$ | 3-F | $CH_2$ | X/Y-26 | 1-$CH_3$, 5-Cyclopropyl |
| $CH_2CH_2$ | H | $CH_2CH_2$ | X/Y-26 | 1-$CH_3$, 5-Cyclopropyl |
| $CH_2$ | H | $CH_2CH_2$ | X/Y-26 | 1,5-$(CH_3)_2$ |
| $CH_2$ | H | $CH_2$ | X/Y-28 | 1-$CH_3$ |
| $CH_2$ | H | $CH_2$ | X/Y-28 | 1,5-$(CH_3)_2$ |
| $CH_2$ | H | $CH_2$ | X/Y-28 | 1-$CH_3$, 5-Cyclopropyl |

Tabelle D (Forts.)

| A | R1 | B | Y | Substituent an Y |
|---|---|---|---|---|
| $CH(CH_3)$ | H | $CH(CH_3)$ | X/Y-28 | 1-$CH_3$, 5-Cyclopropyl |
| $CH(CH_3)$ | H | $CH(CH_3)$ | X/Y-28 | 1,5-$(CH_3)_2$ |
| $CH(CH_3)$ | 3-F | $CH_2$ | X/Y-28 | 1-$CH_3$, 5-Cyclopropyl |
| $CH_2CH_2$ | H | $CH_2CH_2$ | X/Y-28 | 1-$CH_3$, 5-Cyclopropyl |
| $CH_2$ | H | $CH_2CH_2$ | X/Y-28 | 1,5-$(CH_3)_2$ |
| $CH_2$ | H | $CH_2$ | X/Y-22 | - |
| $CH_2$ | H | $CH_2$ | X/Y-22 | 3-$CH_3$ |
| $CH_2$ | H | $CH_2$ | X/Y-22 | 3-Cyclopropyl |
| $CH_2$ | H | $CH_2$ | X/Y-22 | 3-$OCH_3$ |
| $CH(CH_3)$ | H | $CH_2$ | X/Y-22 | 3-$CH_3$ |
| $CH_2$ | H | $CH(CH_3)$ | X/Y-22 | 3-Cyclopropyl |
| $CH_2$ | 3-F | $CH(CH_3)$ | X/Y-22 | 3-$OCH_3$ |
| $CH_2CH_2$ | H | $CH_2CH_2$ | X/Y-22 | 3-Cyclopropyl |
| $CH_2$ | H | $CH_2CH_2$ | X/Y-22 | 3-$CH_3$ |
| $CH_2$ | H | $CH_2$ | X/Y-29 | 1-$CH_3$ |
| $CH_2$ | H | $CH_2$ | X/Y-29 | 1,3-$(CH_3)_2$ |
| $CH_2$ | H | $CH_2$ | X/Y-29 | 1-$CH_3$, 3-Cyclopropyl |
| $CH(CH_3)$ | H | $CH(CH_3)$ | X/Y-29 | 1,3-$(CH_3)_2$ |
| $CH(CH_3)$ | 3-F | $CH(CH_3)$ | X/Y-29 | 1-$CH_3$, 3-Cyclopropyl |
| $CH_2CH_2$ | H | $CH_2CH_2CH_2$ | X/Y-29 | 1,3-$(CH_3)_2$ |
| $CH_2CH_2$ | H | $CH_2CH_2$ | X/Y-29 | 1-$CH_3$, 3-Cyclopropyl |
| $CH_2$ | H | $CH_2$ | X/Y-17 | - |
| $CH_2$ | H | $CH_2$ | X/Y-17 | 5-Cyclopropyl |
| $CH_2$ | H | $CH_2$ | X/Y-17 | 5-$CH_3$ |
| $CH_2$ | H | $CH_2$ | X/Y-17 | 5-$OCH_2CH_3$ |
| $CH_2$ | H | $CH_2$ | X/Y-17 | 5-Cl |
| $CH_2$ | H | $CH_2$ | X/Y-17 | 4-$CH_3$ |
| $CH_2$ | H | $CH_2$ | X/Y-17 | 4-Cyclopropyl |
| $CH_2$ | H | $CH_2$ | X/Y-17 | 4-$OCH_2CH_3$ |
| $CH_2$ | H | $CH_2$ | X/Y-17 | 4-Cl |
| $CH(CH_3)$ | H | $CH(CH_3)$ | X/Y-17 | 5-Cyclopropyl |
| $CH(CH_3)$ | H | $CH(CH_3)$ | X/Y-17 | 4-Cyclopropyl |
| $CH(CH_3)$ | 3-F | $CH(CH_3)$ | X/Y-17 | 5-$OCH_2CH_3$ |
| $CH_2CH_2$ | H | $CH_2CH_2$ | X/Y-17 | 5-Cyclopropyl |
| $CH_2CH_2$ | H | $CH_2CH_2$ | X/Y-17 | 4-Cyclopropyl |

Tabelle D (Forts.)

| A | R1 | B | Y | Substituent an Y |
|---|---|---|---|---|
| $CH_2CH_2$ | H | $CH_2CH_2CH_2$ | X/Y-17 | 5-$CH_3$ |
| $CH_2$ | H | $CH_2$ | X/Y-14 | - |
| $CH_2$ | H | $CH_2$ | X/Y-14 | 5-$CH_3$ |
| $CH_2$ | H | $CH_2$ | X/Y-14 | 5-Cyclopropyl |
| $CH_2$ | H | $CH_2$ | X/Y-14 | 4-$CH_3$ |
| $CH_2$ | H | $CH_2$ | X/Y-14 | 4-Cyclopropyl |
| $CH_2$ | H | $CH_2$ | X/Y-14 | 4-$OCH_2CH_3$ |
| $CH_2$ | H | $CH_2$ | X/Y-14 | 5-$OCH_2CH_3$ |
| $CH_2$ | H | $CH_2$ | X/Y-14 | 5-Cl |
| $CH(CH_3)$ | H | $CH(CH_3)$ | X/Y-14 | 4-Cyclopropyl |
| $CH(CH_3)$ | H | $CH(CH_3)$ | X/Y-14 | 5-Cyclopropyl |
| $CH(CH_3)$ | H | $CH(CH_3)$ | X/Y-14 | 4-$CH_3$ |
| $CH(CH_3)$ | 3-F | $CH(CH_3)$ | X/Y-14 | 4-Cyclopropyl |
| $CH_2$ | 3-F | $CH_2$ | X/Y-14 | 5-$OCH_2CH_3$ |
| $CH_2CH_2$ | H | $CH_2CH_2$ | X/Y-14 | 4-Cyclopropyl |
| $CH_2CH_2$ | H | $CH_2CH_2$ | X/Y-14 | 5-Cyclopropyl |
| $CH_2CH_2$ | H | $CH_2CH_2CH_2$ | X/Y-14 | 5-Cl |
| $CH_2CH_2$ | H | $CH_2CH_2$ | X/Y-14 | 4-$CH_3$ |
| $CH_2$ | H | $CH_2$ | X/Y-20 | 1-$CH_3$ |
| $CH_2$ | H | $CH_2$ | X/Y-20 | 1,5-$(CH_3)_2$ |
| $CH(CH_3)$ | H | $CH(CH_3)$ | X/Y-20 | 1-$CH_3$ |
| $CH(CH_3)$ | 3-F | $CH(CH_3)$ | X/Y-20 | 1,5-$(CH_3)_2$ |
| $CH_2CH_2$ | H | $CH_2CH_2$ | X/Y-20 | 1-$CH_3$ |
| $CH_2CH_2$ | H | $CH_2CH_2$ | X/Y-20 | 1,5-$(CH_3)_2$ |
| $CH(CH_3)CH_2$ | 3-F | $CH(CH_3)CH_2$ | X/Y-20 | 1,5-$(CH_3)_2$ |
| $CH_2$ | H | $CH_2$ | X/Y-3 | - |
| $CH_2$ | H | $CH_2$ | X/Y-3 | 3-$CH_3$ |
| $CH_2$ | H | $CH_2$ | X/Y-3 | 3-$CH_2CH_3$ |
| $CH_2$ | H | $CH_2$ | X/Y-3 | 3-$CH(CH_3)_2$ |
| $CH_2$ | H | $CH_2$ | X/Y-3 | 3-$OCH_2CH_3$ |
| $CH_2$ | H | $CH_2$ | X/Y-3 | 3-$CH_2OCH_3$ |
| $CH_2$ | H | $CH_2$ | X/Y-3 | 3-$CF_3$ |
| $CH(CH_3)$ | H | $CH_2$ | X/Y-3 | 3-$CH_3$ |
| $CH(CH_3)$ | H | $CH_2$ | X/Y-3 | 3-$CH(CH_3)_2$ |

Tabelle D (Forts.)

| A | $R^1$ | B | Y | Substituent an Y |
|---|---|---|---|---|
| $CH(CH_3)$ | H | $CH_2$ | X/Y-3 | 3-Cyclopropyl |
| $CH_2$ | H | $CH(CH_3)$ | X/Y-3 | 3-$CH_3$ |
| $CH_2$ | H | $CH(CH_3)$ | X/Y-3 | 3-$CH(CH_3)_2$ |
| $CH_2$ | H | $CH(CH_3)$ | X/Y-3 | 3-Cyclopropyl |
| $CH(CH_3)$ | H | $CH(CH_3)$ | X/Y-3 | 3-$CH_3$ |
| $CH(CH_3)$ | 3-F | $CH(CH_3)$ | X/Y-3 | 3-$CH(CH_3)_2$ |
| $CH(CH_3)$ | H | $CH(CH_3)$ | X/Y-3 | 3-Cyclopropyl |
| $CH_2$ | H | $CH_2CH_2$ | X/Y-3 | 3-$CH_3$ |
| $CH_2CH_2$ | H | $CH_2CH_2CH_2$ | X/Y-3 | 3-$CH(CH_3)_2$ |
| $CH_2$ | H | $CH_2CH_2$ | X/Y-3 | 3-Cyclopropyl |
| $CH_2CH_2$ | H | $CH_2CH(CH_3)$ | X/Y-3 | 3-Cyclopropyl |
| $CH_2CH_2$ | H | $CH_2CH(CH_3)$ | X/Y-3 | 3-$CH(CH_3)_2$ |
| $CH_2CH_2CH_2$ | 3-F | $CH(CH_3)CH_2$ | X/Y-3 | 3-$CH_3$ |
| $CH_2$ | H | $CH_2$ | X/Y-5 | - |
| $CH_2$ | H | $CH_2$ | X/Y-5 | 5-$OCH_2CH_3$ |
| $CH_2$ | H | $CH_2$ | X/Y-5 | 5-Cyclopropyl |
| $CH_2$ | H | $CH_2$ | X/Y-5 | 5-$CH_3$ |
| $CH(CH_3)$ | 3-F | $CH(CH_3)$ | X/Y-5 | 5-$CH_3$ |
| $CH_2$ | H | $CH(CH_3)$ | X/Y-5 | 5-Cyclopropyl |
| $CH(CH_3)$ | H | $CH_2$ | X/Y-5 | 5-Cyclopropyl |
| $CH_2$ | H | $CH_2CH_2$ | X/Y-5 | 5-$OCH_2CH_3$ |
| $CH_2CH_2$ | H | $CH_2CH_2CH_2$ | X/Y-5 | 5-$CH_3$ |
| $CH_2$ | H | $CH_2$ | X/Y-4 | - |
| $CH_2$ | H | $CH_2$ | X/Y-4 | 3-$OCH_2CH_3$ |
| $CH_2$ | H | $CH_2$ | X/Y-4 | 5-$OCH_2CH_3$ |
| $CH_2$ | H | $CH_2$ | X/Y-4 | 3-$CH(CH_3)_2$ |
| $CH_2$ | H | $CH_2$ | X/Y-4 | 5-$CH_3$ |
| $CH(CH_3)$ | 3-F | $CH(CH_3)$ | X/Y-4 | 3-$CH(CH_3)_2$ |
| $CH_2$ | H | $CH_2$ | X/Y-11 | - |
| $CH_2$ | H | $CH_2$ | X/Y-11 | 3-$CH_3$ |
| $CH_2$ | H | $CH_2$ | X/Y-11 | 5-$CH_3$ |
| $CH_2$ | H | $CH(CH_3)$ | X/Y-11 | 3-$CH_3$ |
| $CH_2$ | 3-F | $CH(CH_3)$ | X/Y-11 | 5-$CH_3$ |
| $CH(CH_3)$ | H | $CH(CH_3)$ | X/Y-11 | 5-$CH_3$ |
| $CH_2$ | H | $CH_2$ | X/Y-21 | - |

Tabelle D (Forts.)

| A | $R^1$ | B | Y | Substituent an Y |
|---|---|---|---|---|
| $CH_2$ | H | $CH_2$ | X/Y-21 | 4,5-$(CH_3)_2$ |
| $CH_2$ | H | $CH_2$ | X/Y-21 | 4,5-$Br_2$ |
| $CH(CH_3)$ | H | $CH(CH_3)$ | X/Y-21 | 4,5-$Cl_2$ |
| $CH(CH_3)$ | H | $CH(CH_3)$ | X/Y-21 | 4,5-$(CH_3)_2$ |
| $CH(CH_3)$ | H | $CH(CH_3)$ | X/Y-21 | 4,5-$Br_2$ |
| $CH_2$ | H | $CH_2$ | X/Y-30 | - |
| $CH_2$ | H | $CH_2$ | X/Y-30 | 3,5-$(CH_3)_2$ |
| $CH(CH_3)$ | H | $CH(CH_3)$ | X/Y-30 | - |
| $CH(CH_3)$ | H | $CH(CH_3)$ | X/Y-30 | 3,5-$(CH_3)_2$ |
| $CH_2$ | H | $CH_2$ | X/Y-27 | - |
| $CH_2$ | H | $CH_2$ | X/Y-27 | 2-Cl |
| $CH_2$ | H | $CH_2$ | X/Y-27 | 2,5-$(CH_3)_2$ |
| $CH(CH_3)$ | H | $CH(CH_3)$ | X/Y-27 | - |
| $CH(CH_3)$ | H | $CH(CH_3)$ | X/Y-27 | 2-Cl |
| $CH(CH_3)$ | H | $CH(CH_3)$ | X/Y-27 | 2,5-$(CH_3)_2$ |

Tabelle E (zu Struktur I.E).

| A | $R^1$ | B | Y | Substituent an Y |
|---|---|---|---|---|
| $CH_2$ | H | $CH_2$ | X/Y-1 | |
| $CH(CH_3)$ | H | $CH_2$ | X/Y-1 | |
| $CH(CH_2CH_3)$ | H | $CH_2$ | X/Y-1 | |
| $CH_2$ | H | $CH(CH_3)$ | X/Y-1 | |
| $CH_2$ | H | $CH(CH_2CH_3)$ | X/Y-1 | |
| $CH_2$ | 3-$CH_3$ | $CH_2$ | X/Y-1 | |
| $CH_2$ | 3-F | $CH_2$ | X/Y-1 | |
| $CH_2$ | H | $CH_2$ | X/Y-1 | 5-$CH_3$ |
| $CH_2$ | H | $CH_2$ | X/Y-1 | 4-$CH_3$ |
| $CH_2$ | H | $CH_2$ | X/Y-1 | 3-$CH_3$ |
| $CH_2$ | H | $CH_2$ | X/Y-1 | 5-Cyclopropyl |
| $CH_2$ | H | $CH_2$ | X/Y-1 | 4-Cyclopropyl |
| $CH_2$ | H | $CH_2$ | X/Y-1 | 3-Cyclopropyl |
| $CH_2$ | H | $CH_2$ | X/Y-1 | 5-Cl |
| $CH_2$ | H | $CH_2$ | X/Y-1 | 5-Br |
| $CH_2$ | H | $CH_2$ | X/Y-1 | 4-Cl |
| $CH_2$ | H | $CH_2$ | X/Y-1 | 4-Br |
| $CH_2$ | H | $CH_2$ | X/Y-1 | 4,5-Cl,Cl |
| $CH_2$ | H | $CH_2$ | X/Y-1 | 4-$OCH_2CH_3$ |
| $CH(CH_3)$ | H | $CH_2$ | X/Y-1 | 5-Br |
| $CH(CH_3)$ | H | $CH_2$ | X/Y-1 | 4,5-Cl,Cl |
| $CH(CH_3)$ | H | $CH_2$ | X/Y-1 | 4-Cl |
| $CH_2$ | H | $CH(CH_3)$ | X/Y-1 | 5-Br |
| $CH_2$ | H | $CH(CH_3)$ | X/Y-1 | 4,5-Cl,Cl |
| $CH(CH_3)$ | H | $CH(CH_3)$ | X/Y-1 | - |
| $CH(CH_3$ | H | $CH(CH_3)$ | X/Y-1 | 4,5-Cl,Cl |
| $CH(CH_3)$ | 3-F | $CH(CH_3)$ | X/Y-1 | - |
| $CH(CH_3)$ | 3-F | $CH(CH_3)$ | X/Y-1 | 5-Br |
| $CH_2CH_2$ | H | $CH_2$ | X/Y-1 | 5-Br |
| $CH_2CH_2$ | H | $CH_2CH_2$ | X/Y-1 | 4,5-Cl,Cl |
| $CH_2CH_2CH_2$ | H | $CH_2$ | X/Y-1 | 5-Br |
| $CH_2CH_2$ | H | $CH_2CH_2CH_2$ | X/Y-1 | 4,5-Cl,Cl |
| $CH_2CH(CH_3)$ | H | $CH_2CH_2$ | X/Y-1 | 4,5-Cl,Cl |
| $CH_2CH_2$ | 3-F | $CH_2CH(CH_3)$ | X/Y-1 | 5-Br |

Tabelle E (Forts.)

| A | R1 | B | Y | Substituent an Y |
|---|---|---|---|---|
| $CH_2$ | H | $CH_2$ | X/Y-2 | - |
| $CH_2$ | H | $CH_2$ | X/Y-2 | 4-Cyclopropyl |
| $CH_2$ | H | $CH_2$ | X/Y-2 | 5-Cyclopropyl |
| $CH_2$ | H | $CH_2$ | X/Y-2 | 5-Cl |
| $CH_2$ | H | $CH_2$ | X/Y-2 | 4,5-Cl,Cl |
| $CH_2$ | H | $CH_2$ | X/Y-2 | 4,5-Br,Br |
| $CH_2$ | H | $CH_2$ | X/Y-2 | 4-$CH_3$ |
| $CH_2$ | H | $CH_2$ | X/Y-2 | 5-$CH_3$ |
| $CH_2$ | H | $CH_2$ | X/Y-2 | 4-$OCH_3$ |
| $CH_2$ | H | $CH_2$ | X/Y-2 | 5-$OCH_3$ |
| $CH(CH_3)$ | H | $CH_2$ | X/Y-2 | 4,5-Cl,Cl |
| $CH(CH_3)$ | H | $CH_2$ | X/Y-2 | 4,5-Br,Br |
| $CH_2$ | H | $CH(CH_3)$ | X/Y-2 | 4,5-Cl,Cl |
| $CH_2$ | H | $CH(CH_3)$ | X/Y-2 | 4,5-Br,Br |
| $CH(CH_3)$ | H | $CH(CH_3)$ | X/Y-2 | 4,5-Cl,Cl |
| $CH(CH_3)$ | H | $CH(CH_3)$ | X/Y-2 | 4,5-Br,Br |
| $CH(CH_3)$ | 3-F | $CH(CH_3)$ | X/Y-2 | 4,5-Cl,Cl |
| $CH(CH_3)$ | 3-F | $CH(CH_3)$ | X/Y-2 | 4,5-Br,Br |
| $CH_2CH_2$ | H | $CH_2$ | X/Y-2 | 4,5-Cl,Cl |
| $CH_2CH_2$ | H | $CH_2CH_2$ | X/Y-2 | 4,5-Br,Br |
| $CH_2CH_2CH_2$ | H | $CH_2$ | X/Y-2 | 4,5-Cl,Cl |
| $CH_2CH_2$ | H | $CH_2CH_2CH_2$ | X/Y-2 | 4,5-Br,Br |
| $CH_2CH(CH_3)$ | H | $CH_2CH_2$ | X/Y-2 | 4,5-Br,Br |
| $CH_2CH_2$ | 3-F | $CH_2CH(CH_3)$ | X/Y-2 | 4,5-Cl,Cl |
| $CH_2$ | H | $CH_2$ | X/Y-15 | - |
| $CH_2$ | H | $CH_2$ | X/Y-15 | 2-$CH_3$ |
| $CH_2$ | H | $CH_2$ | X/Y-15 | 2-Cyclopropyl |
| $CH_2$ | H | $CH_2$ | X/Y-15 | 2-$OCH_2CH_3$ |
| $CH_2$ | H | $CH_2$ | X/Y-15 | 2-Cl |
| $CH(CH_3)$ | H | $CH_2$ | X/Y-15 | 2-Cyclopropyl |
| $CH(CH_3)$ | H | $CH_2$ | X/Y-15 | 2-$OCH_2CH_3$ |
| $CH_2$ | H | $CH(CH_3)$ | X/Y-15 | 2-Cyclopropyl |
| $CH_2$ | H | $CH(CH_3)$ | X/Y-15 | 2-$OCH_2CH_3$ |
| $CH(CH_3)$ | H | $CH(CH_3)$ | X/Y-15 | 2-Cyclopropyl |
| $CH(CH_3)$ | H | $CH(CH_3)$ | X/Y-15 | 2-$OCH_2CH_3$ |

Tabelle E (Forts.)

| A | $R^1$ | B | Y | Substituent an Y |
|---|---|---|---|---|
| $CH(CH_3)$ | 3-F | $CH(CH_3)$ | X/Y-15 | 2-Cyclopropyl |
| $CH(CH_3)$ | 3-F | $CH(CH_3)$ | X/Y-15 | 2-$OCH_2CH_3$ |
| $CH_2CH_2$ | H | $CH_2$ | X/Y-15 | 2-Cyclopropyl |
| $CH_2CH_2$ | H | $CH_2CH_2$ | X/Y-15 | 2-$OCH_2CH_3$ |
| $CH_2CH_2CH_2$ | H | $CH_2$ | X/Y-15 | 2-Cyclopropyl |
| $CH_2CH_2$ | H | $CH_2CH_2CH_2$ | X/Y-15 | 2-$OCH_2CH_3$ |
| $CH_2CH(CH_3)$ | H | $CH_2CH_2$ | X/Y-15 | 2-Cyclopropyl |
| $CH_2CH_2$ | 3-F | $CH_2CH(CH_3)$ | X/Y-15 | 2-$OCH_2CH_3$ |
| $CH_2$ | H | $CH_2$ | X/Y-12 | - |
| $CH_2$ | H | $CH_2$ | X/Y-12 | 2-$CH_3$ |
| $CH_2$ | H | $CH_2$ | X/Y-12 | 2-Cyclopropyl |
| $CH_2$ | H | $CH_2$ | X/Y-12 | 2-$OCH_2CH_3$ |
| $CH(CH_3)$ | H | $CH_2$ | X/Y-12 | 2-Cyclopropyl |
| $CH(CH_3)$ | H | $CH_2$ | X/Y-12 | 2-$OCH_2CH_3$ |
| $CH_2$ | H | $CH(CH_3)$ | X/Y-12 | 2-Cyclopropyl |
| $CH_2$ | H | $CH(CH_3)$ | X/Y-12 | 2-$OCH_2CH_3$ |
| $CH(CH_3)$ | H | $CH(CH_3)$ | X/Y-12 | 2-Cyclopropyl |
| $CH(CH_3)$ | 3-F | $CH(CH_3)$ | X/Y-12 | 2-$OCH_2CH_3$ |
| $CH_2CH_2$ | H | $CH_2$ | X/Y-12 | 2-Cyclopropyl |
| $CH_2CH_2$ | H | $CH_2CH_2$ | X/Y-12 | 2-$OCH_2CH_3$ |
| $CH_2CH_2CH_2$ | H | $CH_2$ | X/Y-12 | 2-Cyclopropyl |
| $CH_2CH_2$ | H | $CH_2CH_2CH_2$ | X/Y-12 | 2-$OCH_2CH_3$ |
| $CH_2CH(CH_3)$ | H | $CH_2CH_2$ | X/Y-12 | 2-Cyclopropyl |
| $CH_2CH_2$ | 3-F | $CH_2CH(CH_3)$ | X/Y-12 | 2-Cyclopropyl |
| $CH_2$ | H | $CH_2$ | X/Y-18 | 1-$CH_3$ |
| $CH_2$ | H | $CH_2$ | X/Y-18 | 1,2-$(CH_3)_2$ |
| $CH_2$ | H | $CH_2$ | X/Y-18 | 1,4-$(CH_3)_2$ |
| $CH_2$ | H | $CH_2$ | X/Y-18 | 1,2,4-$(CH_3)_2$ |
| $CH_2$ | H | $CH_2$ | X/Y-18 | 1-$CH_3$, 2-Cyclopropyl |
| $CH(CH_3)$ | H | $CH_2$ | X/Y-18 | 1-$CH_3$, 2-Cyclopropyl |
| $CH(CH_3)$ | H | $CH_2$ | X/Y-18 | 1,2,4-$(CH_3)_3$ |
| $CH_2$ | H | $CH(CH_3)$ | X/Y-18 | 1-$CH_3$, 2-Cyclopropyl |
| $CH_2$ | H | $CH(CH_3)$ | X/Y-18 | 1,2,4-$(CH_3)_3$ |
| $CH(CH_3)$ | H | $CH(CH_3)$ | X/Y-18 | 1-$CH_3$, 2-Cyclopropyl |
| $CH(CH_3)$ | 3-F | $CH(CH_3)$ | X/Y-18 | 1,2,4-$(CH_3)_3$ |

Tabelle E (Forts.)

| A | $R^1$ | B | Y | Substituent an Y |
|---|---|---|---|---|
| $CH_2CH_2$ | H | $CH_2$ | X/Y-18 | 1-$CH_3$, 2-Cyclopropyl |
| $CH_2CH_2$ | H | $CH_2CH_2$ | X/Y-18 | 1,2,4-$(CH_3)_2$ |
| $CH_2CH_2CH_2$ | H | $CH_2$ | X/Y-18 | 1-$CH_3$, 2-Cyclopropyl |
| $CH_2CH_2$ | H | $CH_2CH_2CH_2$ | X/Y-18 | 1,2,4-$(CH_3)_2$ |
| $CH_2CH(CH_3)$ | H | $CH_2CH_2$ | X/Y-18 | 1-$CH_3$, 2-Cyclopropyl |
| $CH_2CH_2$ | 3-F | $CH_2CH(CH_3)$ | X/Y-18 | 1,2,4-$(CH_3)_2$ |
| $CH_2$ | H | $CH_2$ | X/Y-19 | 1-$CH_3$ |
| $CH_2$ | H | $CH_2$ | X/Y-19 | 1,2-$(CH_3)_2$ |
| $CH_2$ | H | $CH_2$ | X/Y-19 | 1,5-$(CH_3)_2$ |
| $CH_2$ | H | $CH_2$ | X/Y-19 | 1-$CH_3$, 2-Cyclopropyl |
| $CH(CH_3)$ | H | $CH_2$ | X/Y-19 | 1,2-$(CH_3)_2$ |
| $CH(CH_3)$ | H | $CH_2$ | X/Y-19 | 1-$CH_3$, 2-Cyclopropyl |
| $CH_2$ | H | $CH(CH_3)$ | X/Y-19 | 1,2-$(CH_3)_2$ |
| $CH_2$ | H | $CH(CH_3)$ | X/Y-19 | 1-$CH_3$, 2-Cyclopropyl |
| $CH(CH_3)$ | H | $CH(CH_3)$ | X/Y-19 | 1,2-$(CH_3)_2$ |
| $CH(CH_3)$ | H | $CH(CH_3)$ | X/Y-19 | 1-$CH_3$, 2-Cyclopropyl |
| $CH(CH_3)$ | 3-F | $CH_2$ | X/Y-19 | 1-$CH_3$, 2-Cyclopropyl |
| $CH_2CH_2$ | H | $CH_2$ | X/Y-19 | 1-$CH_3$, 2-Cyclopropyl |
| $CH_2CH_2$ | H | $CH_2CH_2$ | X/Y-19 | 1,2-$(CH_3)_2$ |
| $CH_2CH_2CH_2$ | H | $CH_2$ | X/Y-19 | 1-$CH_3$, 2-Cyclopropyl |
| $CH_2CH_2$ | H | $CH_2CH_2CH_2$ | X/Y-19 | 1,2-$(CH_3)_2$ |
| $CH_2CH(CH_3)$ | H | $CH_2CH_2$ | X/Y-19 | 1-$CH_3$, 2-Cyclopropyl |
| $CH_2CH_2$ | 3-F | $CH_2CH(CH_3)$ | X/Y-19 | 1,2-$(CH_3)_2$ |
| $CH_2$ | H | $CH_2$ | X/Y-16 | – |
| $CH_2$ | H | $CH_2$ | X/Y-16 | 2-$CH_3$ |
| $CH_2$ | H | $CH_2$ | X/Y-16 | 2-Cyclopropyl |
| $CH_2$ | H | $CH_2$ | X/Y-16 | 2-Cl |
| $CH_2$ | H | $CH_2$ | X/Y-16 | 2-$OCH_2CH_3$ |
| $CH(CH_3)$ | H | $CH_2$ | X/Y-16 | 2-$CH_3$ |
| $CH(CH_3)$ | H | $CH_2$ | X/Y-16 | 2-Cyclopropyl |
| $CH_2$ | H | $CH(CH_3)$ | X/Y-16 | 2-$CH_3$ |
| $CH_2$ | H | $CH(CH_3)$ | X/Y-16 | 2-Cyclopropyl |
| $CH_2(CH_3)$ | H | $CH(CH_3)$ | X/Y-16 | 2-$CH_3$ |
| $CH_2(CH_3)$ | H | $CH(CH_3)$ | X/Y-16 | 2-Cyclopropyl |
| $CH_2(CH_3)$ | 3-F | $CH(CH_3)$ | X/Y-16 | 2-Cyclopropyl |

Tabelle E (Forts.)

| A | R1 | B | Y | Substituent an Y |
|---|---|---|---|---|
| $CH_2CH_2$ | H | $CH_2$ | X/Y-16 | 2-Cyclopropyl |
| $CH_2CH_2$ | H | $CH_2CH_2$ | X/Y-16 | 2-$CH_3$ |
| $CH_2CH_2CH_2$ | H | $CH_2$ | X/Y-16 | 2-Cyclopropyl |
| $CH_2CH_2$ | H | $CH_2CH_2CH_2$ | X/Y-16 | 2-$CH_3$ |
| $CH_2CH(CH_3)$ | H | $CH_2CH_2$ | X/Y-16 | 2-Cyclopropyl |
| $CH_2CH_2$ | 3-F | $CH_2CH(CH_3)$ | X/Y-16 | 2-$CH_3$ |
| $CH_2$ | H | $CH_2$ | X/Y-13 | – |
| $CH_2$ | H | $CH_2$ | X/Y-13 | 2-$CH_3$ |
| $CH_2$ | H | $CH_2$ | X/Y-13 | 2-Cyclopropyl |
| $CH_2$ | H | $CH_2$ | X/Y-13 | 2-$OCH_2CH_3$ |
| $CH(CH_3)$ | H | $CH_2$ | X/Y-13 | 2-$OCH_2CH_3$ |
| $CH(CH_3)$ | H | $CH_2$ | X/Y-13 | 2-Cyclopropyl |
| $CH_2$ | H | $CH(CH_3)$ | X/Y-13 | 2-$OCH_2CH_3$ |
| $CH_2$ | H | $CH(CH_3)$ | X/Y-13 | 2-Cyclopropyl |
| $CH(CH_3)$ | H | $CH(CH_3)$ | X/Y-13 | 2-$OCH_2CH_3$ |
| $CH(CH_3)$ | 3-F | $CH(CH_3)$ | X/Y-13 | 2-Cyclopropyl |
| $CH_2CH_2$ | H | $CH_2$ | X/Y-13 | 2-Cyclopropyl |
| $CH_2CH_2$ | H | $CH_2CH_2$ | X/Y-13 | 2-$CH_3$ |
| $CH_2CH_2CH_2$ | H | $CH_2$ | X/Y-13 | 2-Cyclopropyl |
| $CH_2CH_2$ | H | $CH_2CH_2CH_2$ | X/Y-13 | 2-$CH_3$ |
| $CH(CH_3)CH_2$ | 3-F | $CH_2CH_2$ | X/Y-13 | 2-Cyclopropyl |
| $CH_2CH_2$ | H | $CH(CH_3)CH_2$ | X/Y-13 | 2-$CH_3$ |
| $CH_2$ | H | $CH_2$ | X/Y-6 | – |
| $CH_2$ | H | $CH_2$ | X/Y-6 | 3-$CH_3$ |
| $CH_2$ | H | $CH_2$ | X/Y-6 | 3-Cyclopropyl |
| $CH_2$ | H | $CH_2$ | X/Y-6 | 3-$OCH_2CH_3$ |
| $CH(CH_3)$ | H | $CH(CH_3)$ | X/Y-6 | 3-Cyclopropyl |
| $CH(CH_3)$ | 3-F | $CH(CH_3)$ | X/Y-6 | 3-Cyclopropyl |
| $CH_2CH_2$ | H | $CH_2CH_2$ | X/Y-6 | 3-Cyclopropyl |
| $CH_2CH_2$ | H | $CH_2CH_2CH_2$ | X/Y-6 | 3-$CH_3$ |
| $CH(CH_3)CH_2$ | 3-F | $CH_2CH_2$ | X/Y-6 | 3-Cyclopropyl |
| $CH_2CH_2$ | H | $CH(CH_3)CH_2$ | X/Y-6 | 3-Cyclopropyl |
| $CH_2$ | H | $CH_2$ | X/Y-8 | 1-$CH_3$ |
| $CH_2$ | H | $CH_2$ | X/Y-8 | 1,3-$(CH_3)_2$ |
| $CH_2$ | H | $CH_2$ | X/Y-8 | 1,4-$(CH_3)_2$ |

Tabelle E (Forts.)

| A | $R^1$ | B | Y | Substituent an Y |
|---|---|---|---|---|
| $CH_2$ | H | $CH_2$ | X/Y-8 | 1-$CH_3$, 3-Cyclopropyl |
| $CH(CH_3)$ | H | $CH(CH_3)$ | X/Y-8 | 1-$CH_3$, 3-Cyclopropyl |
| $CH(CH_3)$ | 3-F | $CH(CH_3)$ | X/Y-8 | 1-$CH_3$, 3-Cyclopropyl |
| $CH_2CH_2$ | H | $CH_2CH_2$ | X/Y-8 | 1-$CH_3$, 3-Cyclopropyl |
| $CH_2CH_2$ | H | $CH_2CH_2CH_2$ | X/Y-8 | 1,3-$(CH_3)_2$ |
| $CH_2CH(CH_3)$ | H | $CH_2CH_2$ | X/Y-8 | 1-$CH_3$, 3-Cyclopropyl |
| $CH(CH_3)CH_2$ | 3-F | $CH_2CH_2$ | X/Y-8 | 1-$CH_3$, 3-Cyclopropyl |
| $CH_2$ | H | $CH_2$ | X/Y-10 | 1-$CH_3$ |
| $CH_2$ | H | $CH_2$ | X/Y-10 | 1,4-$(CH_3)_2$ |
| $CH_2$ | H | $CH_2$ | X/Y-10 | 1-$CH_3$, 4-$OCH_3$ |
| $CH_2$ | H | $CH_2$ | X/Y-10 | 1-$CH_3$, 5-$CH(CH_3)_2$ |
| $CH_2$ | H | $CH_2$ | X/Y-10 | 1-$CH_3$, 5-Cyclopropyl |
| $CH(CH_3)$ | H | $CH(CH_3)$ | X/Y-10 | 1-$CH_3$, 5-Cyclopropyl |
| $CH(CH_3)$ | 3-F | $CH(CH_3)$ | X/Y-10 | 1,4-$(CH_3)_2$ |
| $CH_2CH_2$ | H | $CH_2CH_2$ | X/Y-10 | 1-$CH_3$, 5-Cyclopropyl |
| $CH_2CH_2$ | H | $CH_2CH_2CH_2$ | X/Y-10 | 1,5-$(CH_3)_2$ |
| $CH_2CH(CH_3)$ | H | $CH_2CH_2$ | X/Y-10 | 1-$CH_3$, 5-Cyclopropyl |
| $CH(CH_3)CH_2$ | 3-F | $CH_2CH_2$ | X/Y-10 | 1-$CH_3$, 5-Cyclopropyl |
| $CH_2$ | H | $CH_2$ | X/Y-7 | - |
| $CH_2$ | H | $CH_2$ | X/Y-7 | 3-$CH_3$ |
| $CH(CH_3)$ | H | $CH(CH_3)$ | X/Y-7 | 3-$CH_3$ |
| $CH(CH_3)$ | 3-F | $CH(CH_3)$ | X/Y-7 | 3-$CH_3$ |
| $CH_2CH_2$ | H | $CH_2CH_2$ | X/Y-7 | 3-$CH_3$ |
| $CH_2CH_2$ | 3-F | $CH(CH_3)CH_2CH_2$ | X/Y-7 | 3-$CH_3$ |
| $CH_2$ | H | $CH_2$ | X/Y-9 | 1-$CH_3$ |
| $CH_2$ | H | $CH_2$ | X/Y-9 | 1,3-$(CH_3)_2$ |
| $CH_2$ | H | $CH_2$ | X/Y-9 | 1,5-$(CH_3)_2$ |
| $CH(CH_3)$ | H | $CH(CH_3)$ | X/Y-9 | 1,3-$(CH_3)_2$ |
| $CH(CH_3)$ | 3-F | $CH(CH_3)$ | X/Y-9 | 1,5-$(CH_3)_2$ |
| $CH_2CH_2$ | H | $CH_2CH_2$ | X/Y-9 | 1,3-$(CH_3)_2$ |
| $CH_2CH_2$ | H | $CH_2CH_2CH_2$ | X/Y-9 | 1,5-$(CH_3)_2$ |
| $CH_2CH(CH_3)$ | H | $CH_2CH_2$ | X/Y-9 | 1,3-$(CH_3)_2$ |
| $CH(CH_3)CH_2$ | 3-F | $CH_2CH_2$ | X/Y-9 | 1,5-$(CH_3)_2$ |
| $CH_2$ | H | $CH_2$ | X/Y-25 | 5-$CH_3$ |

Tabelle E (Forts.)

| A | $R^1$ | B | Y | Substituent an Y |
|---|---|---|---|---|
| $CH_2$ | H | $CH_2$ | X/Y-25 | 5-$CH_2CH_3$ |
| $CH_2$ | H | $CH_2$ | X/Y-25 | 5-$CH(CH_3)_2$ |
| $CH_2$ | H | $CH_2$ | X/Y-25 | 5-$OCH_3$ |
| $CH(CH_3)$ | H | $CH(CH_3)$ | X/Y-25 | 5-Cyclopropyl |
| $CH_2(CH_3)$ | H | $CH(CH_3)$ | X/Y-25 | 5-$CH_3$ |
| $CH(CH_3)$ | H | $CH(CH_3)$ | X/Y-25 | 5-$OCH_2CH_3$ |
| $CH(CH_3)$ | 3-F | $CH(CH_3)$ | X/Y-25 | 5-Cyclopropyl |
| $CH_2CH_2$ | H | $CH_2$ | X/Y-25 | 5-Cyclopropyl |
| $CH_2CH_2$ | H | $CH_2CH_2$ | X/Y-25 | 5-$CH_3$ |
| $CH_2CH_2CH_2$ | H | $CH_2$ | X/Y-25 | 5-$OCH_2CH_3$ |
| $CH_2CH_2$ | 3-F | $CH_2CH(CH_3)$ | X/Y-25 | 5-Cyclopropyl |
| $CH_2$ | H | $CH(CH_3)$ | X/Y-25 | 5-Cyclopropyl |
| $CH_2$ | H | $CH_2$ | X/Y-23 | – |
| $CH_2$ | H | $CH_2$ | X/Y-23 | 5-$CH_3$ |
| $CH_2$ | H | $CH_2$ | X/Y-23 | 5-$CH(CH_3)_2$ |
| $CH_2$ | H | $CH_2$ | X/Y-23 | 5-Cl |
| $CH_2$ | H | $CH_2$ | X/Y-23 | 5-$OCH_2CH_3$ |
| $CH_2$ | H | $CH(CH_3)$ | X/Y-23 | 5-Cyclopropyl |
| $CH_2$ | 3-F | $CH(CH_3)$ | X/Y-23 | 5-$CH_3$ |
| $CH_2$ | H | $CH(CH_3)$ | X/Y-23 | 5-$CH(CH_3)_2$ |
| $CH(CH_3)$ | H | $CH(CH_3)$ | X/Y-23 | 5-Cyclopropyl |
| $CH_2$ | H | $CH_2CH_2$ | X/Y-23 | 5-Cyclopropyl |
| $CH_2CH_2CH_2$ | H | $CH_2$ | X/Y-23 | 5-$CH_3$ |
| $CH_2CH_2$ | H | $CH_2CH_2$ | X/Y-23 | 5-$CH(CH_3)_2$ |
| $CH_2CH_2$ | H | $CH_2CH_2$ | X/Y-23 | 5-Cyclopropyl |
| $CH_2$ | H | $CH_2$ | X/Y-26 | 1-$CH_3$ |
| $CH_2$ | H | $CH_2$ | X/Y-26 | 1,5-$(CH_3)_2$ |
| $CH_2$ | H | $CH_2$ | X/Y-26 | 1-$CH_3$, 5-Cyclopropyl |
| $CH(CH_3)$ | H | $CH(CH_3)$ | X/Y-26 | 1-$CH_3$, 5-Cyclopropyl |
| $CH(CH_3)$ | H | $CH(CH_3)$ | X/Y-26 | 1,5-$(CH_3)_2$ |
| $CH(CH_3)$ | 3-F | $CH_2$ | X/Y-26 | 1-$CH_3$, 5-Cyclopropyl |
| $CH_2CH_2$ | H | $CH_2CH_2$ | X/Y-26 | 1-$CH_3$, 5-Cyclopropyl |
| $CH_2$ | H | $CH_2CH_2$ | X/Y-26 | 1,5-$(CH_3)_2$ |
| $CH_2$ | H | $CH_2$ | X/Y-28 | 1-$CH_3$ |
| $CH_2$ | H | $CH_2$ | X/Y-28 | 1,5-$(CH_3)_2$ |
| $CH_2$ | H | $CH_2$ | X/Y-28 | 1-$CH_3$, 5-Cyclopropyl |

Tabelle E (Forts.)

| A | $R^1$ | B | Y | Substituent an Y |
|---|---|---|---|---|
| $CH(CH_3)$ | H | $CH(CH_3)$ | X/Y-28 | $1-CH_3$, 5-Cyclopropyl |
| $CH(CH_3)$ | H | $CH(CH_3)$ | X/Y-28 | $1,5-(CH_3)_2$ |
| $CH(CH_3)$ | 3-F | $CH_2$ | X/Y-28 | $1-CH_3$, 5-Cyclopropyl |
| $CH_2CH_2$ | H | $CH_2CH_2$ | X/Y-28 | $1-CH_3$, 5-Cyclopropyl |
| $CH_2$ | H | $CH_2CH_2$ | X/Y-28 | $1,5-(CH_3)_2$ |
| $CH_2$ | H | $CH_2$ | X/Y-22 | – |
| $CH_2$ | H | $CH_2$ | X/Y-22 | $3-CH_3$ |
| $CH_2$ | H | $CH_2$ | X/Y-22 | 3-Cyclopropyl |
| $CH_2$ | H | $CH_2$ | X/Y-22 | $3-OCH_3$ |
| $CH(CH_3)$ | H | $CH_2$ | X/Y-22 | $3-CH_3$ |
| $CH_2$ | H | $CH(CH_3)$ | X/Y-22 | 3-Cyclopropyl |
| $CH_2$ | 3-F | $CH(CH_3)$ | X/Y-22 | $3-OCH_3$ |
| $CH_2CH_2$ | H | $CH_2CH_2$ | X/Y-22 | 3-Cyclopropyl |
| $CH_2$ | H | $CH_2CH_2$ | X/Y-22 | $3-CH_3$ |
| $CH_2$ | H | $CH_2$ | X/Y-29 | $1-CH_3$ |
| $CH_2$ | H | $CH_2$ | X/Y-29 | $1,3-(CH_3)_2$ |
| $CH_2$ | H | $CH_2$ | X/Y-29 | $1-CH_3$, 3-Cyclopropyl |
| $CH(CH_3)$ | H | $CH(CH_3)$ | X/Y-29 | $1,3-(CH_3)_2$ |
| $CH(CH_3)$ | 3-F | $CH(CH_3)$ | X/Y-29 | $1-CH_3$, 3-Cyclopropyl |
| $CH_2CH_2$ | H | $CH_2CH_2CH_2$ | X/Y-29 | $1,3-(CH_3)_2$ |
| $CH_2CH_2$ | H | $CH_2CH_2$ | X/Y-29 | $1-CH_3$, 3-Cyclopropyl |
| $CH_2$ | H | $CH_2$ | X/Y-17 | – |
| $CH_2$ | H | $CH_2$ | X/Y-17 | 5-Cyclopropyl |
| $CH_2$ | H | $CH_2$ | X/Y-17 | $5-CH_3$ |
| $CH_2$ | H | $CH_2$ | X/Y-17 | $5-OCH_2CH_3$ |
| $CH_2$ | H | $CH_2$ | X/Y-17 | 5-Cl |
| $CH_2$ | H | $CH_2$ | X/Y-17 | $4-CH_3$ |
| $CH_2$ | H | $CH_2$ | X/Y-17 | 4-Cyclopropyl |
| $CH_2$ | H | $CH_2$ | X/Y-17 | $4-OCH_2CH_3$ |
| $CH_2$ | H | $CH_2$ | X/Y-17 | 4-Cl |
| $CH(CH_3)$ | H | $CH(CH_3)$ | X/Y-17 | 5-Cyclopropyl |
| $CH(CH_3)$ | H | $CH(CH_3)$ | X/Y-17 | 4-Cyclopropyl |
| $CH(CH_3)$ | 3-F | $CH(CH_3)$ | X/Y-17 | $5-OCH_2CH_3$ |
| $CH_2CH_2$ | H | $CH_2CH_2$ | X/Y-17 | 5-Cyclopropyl |
| $CH_2CH_2$ | H | $CH_2CH_2$ | X/Y-17 | 4-Cyclopropyl |

Tabelle E (Forts.)

| A | R1 | B | Y | Substituent an Y |
|---|---|---|---|---|
| $CH_2CH_2$ | H | $CH_2CH_2CH_2$ | X/Y-17 | 5-$CH_3$ |
| $CH_2$ | H | $CH_2$ | X/Y-14 | – |
| $CH_2$ | H | $CH_2$ | X/Y-14 | 5-$CH_3$ |
| $CH_2$ | H | $CH_2$ | X/Y-14 | 5-Cyclopropyl |
| $CH_2$ | H | $CH_2$ | X/Y-14 | 4-$CH_3$ |
| $CH_2$ | H | $CH_2$ | X/Y-14 | 4-Cyclopropyl |
| $CH_2$ | H | $CH_2$ | X/Y-14 | 4-$OCH_2CH_3$ |
| $CH_2$ | H | $CH_2$ | X/Y-14 | 5-$OCH_2CH_3$ |
| $CH_2$ | H | $CH_2$ | X/Y-14 | 5-Cl |
| $CH(CH_3)$ | H | $CH(CH_3)$ | X/Y-14 | 4-Cyclopropyl |
| $CH(CH_3)$ | H | $CH(CH_3)$ | X/Y-14 | 5-Cyclopropyl |
| $CH(CH_3)$ | H | $CH(CH_3)$ | X/Y-14 | 4-$CH_3$ |
| $CH(CH_3)$ | 3–F | $CH(CH_3)$ | X/Y-14 | 4-Cyclopropyl |
| $CH_2$ | 3–F | $CH_2$ | X/Y-14 | 5-$OCH_2CH_3$ |
| $CH_2CH_2$ | H | $CH_2CH_2$ | X/Y-14 | 4-Cyclopropyl |
| $CH_2CH_2$ | H | $CH_2CH_2$ | X/Y-14 | 5-Cyclopropyl |
| $CH_2CH_2$ | H | $CH_2CH_2CH_2$ | X/Y-14 | 5-Cl |
| $CH_2CH_2$ | H | $CH_2CH_2$ | X/Y-14 | 4-$CH_3$ |
| $CH_2$ | H | $CH_2$ | X/Y-20 | 1-$CH_3$ |
| $CH_2$ | H | $CH_2$ | X/Y-20 | 1,5-$(CH_3)_2$ |
| $CH(CH_3)$ | H | $CH(CH_3)$ | X/Y-20 | 1-$CH_3$ |
| $CH(CH_3)$ | 3–F | $CH(CH_3)$ | X/Y-20 | 1,5-$(CH_3)_2$ |
| $CH_2CH_2$ | H | $CH_2CH_2$ | X/Y-20 | 1-$CH_3$ |
| $CH_2CH_2$ | H | $CH_2CH_2$ | X/Y-20 | 1,5-$(CH_3)_2$ |
| $CH(CH_3)CH_2$ | 3–F | $CH(CH_3)CH_2$ | X/Y-20 | 1,5-$(CH_3)_2$ |
| $CH_2$ | H | $CH_2$ | X/Y-3 | – |
| $CH_2$ | H | $CH_2$ | X/Y-3 | 3-$CH_3$ |
| $CH_2$ | H | $CH_2$ | X/Y-3 | 3-$CH(CH_3)_2$ |
| $CH_2$ | H | $CH_2$ | X/Y-3 | 3-$OCH_2CH_3$ |
| $CH_2$ | H | $CH_2$ | X/Y-3 | 3-$CH_2OCH_3$ |
| $CH_2$ | H | $CH_2$ | X/Y-3 | 3-$CF_3$ |
| $CH(CH_3)$ | H | $CH_2$ | X/Y-3 | 3-$CH_3$ |
| $CH(CH_3)$ | H | $CH_2$ | X/Y-3 | 3-$CH(CH_3)_2$ |

Tabelle E (Forts.)

| A | $R^1$ | B | Y | Substituent an Y |
|---|---|---|---|---|
| $CH(CH_3)$ | H | $CH_2$ | X/Y-3 | 3-Cyclopropyl |
| $CH_2$ | H | $CH(CH_3)$ | X/Y-3 | $3-CH_3$ |
| $CH_2$ | H | $CH(CH_3)$ | X/Y-3 | $3-CH(CH_3)_2$ |
| $CH_2$ | H | $CH(CH_3)$ | X/Y-3 | 3-Cyclopropyl |
| $CH(CH_3)$ | H | $CH(CH_3)$ | X/Y-3 | $3-CH_3$ |
| $CH(CH_3)$ | 3-F | $CH(CH_3)$ | X/Y-3 | $3-CH(CH_3)_2$ |
| $CH(CH_3)$ | H | $CH(CH_3)$ | X/Y-3 | 3-Cyclopropyl |
| $CH_2$ | H | $CH_2CH_2$ | X/Y-3 | $3-CH_3$ |
| $CH_2CH_2$ | H | $CH_2CH_2CH_2$ | X/Y-3 | $3-CH(CH_3)_2$ |
| $CH_2$ | H | $CH_2CH_2$ | X/Y-3 | 3-Cyclopropyl |
| $CH_2CH_2$ | H | $CH_2CH(CH_3)$ | X/Y-3 | 3-Cyclopropyl |
| $CH_2CH_2$ | H | $CH_2CH(CH_3)$ | X/Y-3 | $3-CH(CH_3)_2$ |
| $CH_2CH_2CH_2$ | 3-F | $CH(CH_3)CH_2$ | X/Y-3 | $3-CH_3$ |
| $CH_2$ | H | $CH_2$ | X/Y-5 | – |
| $CH_2$ | H | $CH_2$ | X/Y-5 | $5-OCH_2CH_3$ |
| $CH_2$ | H | $CH_2$ | X/Y-5 | 5-Cyclopropyl |
| $CH_2$ | H | $CH_2$ | X/Y-5 | $5-CH_3$ |
| $CH(CH_3)$ | 3-F | $CH(CH_3)$ | X/Y-5 | $5-CH_3$ |
| $CH_2$ | H | $CH(CH_3)$ | X/Y-5 | 5-Cyclopropyl |
| $CH(CH_3)$ | H | $CH_2$ | X/Y-5 | 5-Cyclopropyl |
| $CH_2$ | H | $CH_2CH_2$ | X/Y-5 | $5-OCH_2CH_3$ |
| $CH_2CH_2$ | H | $CH_2CH_2CH_2$ | X/Y-5 | $5-CH_3$ |
| $CH_2$ | H | $CH_2$ | X/Y-4 | – |
| $CH_2$ | H | $CH_2$ | X/Y-4 | $3-OCH_2CH_3$ |
| $CH_2$ | H | $CH_2$ | X/Y-4 | $5-OCH_2CH_3$ |
| $CH_2$ | H | $CH_2$ | X/Y-4 | $3-CH(CH_3)_2$ |
| $CH_2$ | H | $CH_2$ | X/Y-4 | $5-CH_3$ |
| $CH(CH_3)$ | 3-F | $CH(CH_3)$ | X/Y-4 | $3-CH(CH_3)_2$ |
| $CH_2$ | H | $CH_2$ | X/Y-11 | – |
| $CH_2$ | H | $CH_2$ | X/Y-11 | $3-CH_3$ |
| $CH_2$ | H | $CH_2$ | X/Y-11 | $5-CH_3$ |
| $CH_2$ | H | $CH(CH_3)$ | X/Y-11 | $3-CH_3$ |
| $CH_2$ | 3-F | $CH(CH_3)$ | X/Y-11 | $5-CH_3$ |
| $CH(CH_3)$ | H | $CH(CH_3)$ | X/Y-11 | $5-CH_3$ |
| $CH_2$ | H | $CH_2$ | X/Y-21 | – |

Tabelle E (Forts.)

| A | $R^1$ | B | Y | Substituent an Y |
|---|---|---|---|---|
| $CH_2$ | H | $CH_2$ | X/Y-21 | 4,5-$(CH_3)_2$ |
| $CH_2$ | H | $CH_2$ | X/Y-21 | 4,5-$Br_2$ |
| $CH(CH_3)$ | H | $CH(CH_3)$ | X/Y-21 | 4,5-$Cl_2$ |
| $CH(CH_3)$ | H | $CH(CH_3)$ | X/Y-21 | 4,5-$(CH_3)_2$ |
| $CH(CH_3)$ | H | $CH(CH_3)$ | X/Y-21 | 4,5-$Br_2$ |
| $CH_2$ | H | $CH_2$ | X/Y-30 | - |
| $CH_2$ | H | $CH_2$ | X/Y-30 | 3,5-$(CH_3)_2$ |
| $CH(CH_3)$ | H | $CH(CH_3)$ | X/Y-30 | - |
| $CH(CH_3)$ | H | $CH(CH_3)$ | X/Y-30 | 3,5-$(CH_3)_2$ |
| $CH_2$ | H | $CH_2$ | X/Y-27 | - |
| $CH_2$ | H | $CH_2$ | X/Y-27 | 2-Cl |
| $CH_2$ | H | $CH_2$ | X/Y-27 | 2,5-$(CH_3)_2$ |
| $CH(CH_3)$ | H | $CH(CH_3)$ | X/Y-27 | - |
| $CH(CH_3)$ | H | $CH(CH_3)$ | X/Y-27 | 2-Cl |
| $CH(CH_3)$ | H | $CH(CH_3)$ | X/Y-27 | 2,5-$(CH_3)_2$ |

Tabelle F (zu Struktur I.F).

| A | $R^1$ | B | Y | Substituent an Y |
|---|---|---|---|---|
| $CH_2$ | H | $CH_2$ | X/Y-1 | |
| $CH(CH_3)$ | H | $CH_2$ | X/Y-1 | |
| $CH(CH_2CH_3)$ | H | $CH_2$ | X/Y-1 | |
| $CH_2$ | H | $CH(CH_3)$ | X/Y-1 | |
| $CH_2$ | H | $CH(CH_2CH_3)$ | X/Y-1 | |
| $CH_2$ | $3-CH_3$ | $CH_2$ | X/Y-1 | |
| $CH_2$ | 3-F | $CH_2$ | X/Y-1 | |
| $CH_2$ | H | $CH_2$ | X/Y-1 | $5-CH_3$ |
| $CH_2$ | H | $CH_2$ | X/Y-1 | $4-CH_3$ |
| $CH_2$ | H | $CH_2$ | X/Y-1 | $3-CH_3$ |
| $CH_2$ | H | $CH_2$ | X/Y-1 | 5-Cyclopropyl |
| $CH_2$ | H | $CH_2$ | X/Y-1 | 4-Cyclopropyl |
| $CH_2$ | H | $CH_2$ | X/Y-1 | 3-Cyclopropyl |
| $CH_2$ | H | $CH_2$ | X/Y-1 | 5-Cl |
| $CH_2$ | H | $CH_2$ | X/Y-1 | 5-Br |
| $CH_2$ | H | $CH_2$ | X/Y-1 | 4-Cl |
| $CH_2$ | H | $CH_2$ | X/Y-1 | 4-Br |
| $CH_2$ | H | $CH_2$ | X/Y-1 | 4,5-Cl,Cl |
| $CH_2$ | H | $CH_2$ | X/Y-1 | $4-OCH_2CH_3$ |
| $CH(CH_3)$ | H | $CH_2$ | X/Y-1 | 5-Br |
| $CH(CH_3)$ | H | $CH_2$ | X/Y-1 | 4,5-Cl,Cl |
| $CH(CH_3)$ | H | $CH_2$ | X/Y-1 | 4-Cl |
| $CH_2$ | H | $CH(CH_3)$ | X/Y-1 | 5-Br |
| $CH_2$ | H | $CH(CH_3)$ | X/Y-1 | 4,5-Cl,Cl |
| $CH(CH_3)$ | H | $CH(CH_3)$ | X/Y-1 | – |
| $CH(CH_3$ | H | $CH(CH_3)$ | X/Y-1 | 4,5-Cl,Cl |
| $CH(CH_3)$ | 3-F | $CH(CH_3)$ | X/Y-1 | – |
| $CH(CH_3)$ | 3-F | $CH(CH_3)$ | X/Y-1 | 5-Br |
| $CH_2CH_2$ | H | $CH_2$ | X/Y-1 | 5-Br |
| $CH_2CH_2$ | H | $CH_2CH_2$ | X/Y-1 | 4,5-Cl,Cl |
| $CH_2CH_2CH_2$ | H | $CH_2$ | X/Y-1 | 5-Br |
| $CH_2CH_2$ | H | $CH_2CH_2CH_2$ | X/Y-1 | 4,5-Cl,Cl |
| $CH_2CH(CH_3)$ | H | $CH_2CH_2$ | X/Y-1 | 4,5-Cl,Cl |
| $CH_2CH_2$ | 3-F | $CH_2CH(CH_3)$ | X/Y-1 | 5-Br |

Tabelle F (Forts.)

| A | $R^1$ | B | Y | Substituent an Y |
|---|---|---|---|---|
| $CH_2$ | H | $CH_2$ | X/Y-2 | - |
| $CH_2$ | H | $CH_2$ | X/Y-2 | 4-Cyclopropyl |
| $CH_2$ | H | $CH_2$ | X/Y-2 | 5-Cyclopropyl |
| $CH_2$ | H | $CH_2$ | X/Y-2 | 5-Cl |
| $CH_2$ | H | $CH_2$ | X/Y-2 | 4,5-Cl,Cl |
| $CH_2$ | H | $CH_2$ | X/Y-2 | 4,5-Br,Br |
| $CH_2$ | H | $CH_2$ | X/Y-2 | 4-$CH_3$ |
| $CH_2$ | H | $CH_2$ | X/Y-2 | 5-$CH_3$ |
| $CH_2$ | H | $CH_2$ | X/Y-2 | 4-$OCH_3$ |
| $CH_2$ | H | $CH_2$ | X/Y-2 | 5-$OCH_3$ |
| $CH(CH_3)$ | H | $CH_2$ | X/Y-2 | 4,5-Cl,Cl |
| $CH(CH_3)$ | H | $CH_2$ | X/Y-2 | 4,5-Br,Br |
| $CH_2$ | H | $CH(CH_3)$ | X/Y-2 | 4,5-Cl,Cl |
| $CH_2$ | H | $CH(CH_3)$ | X/Y-2 | 4,5-Br,Br |
| $CH(CH_3)$ | H | $CH(CH_3)$ | X/Y-2 | 4,5-Cl,Cl |
| $CH(CH_3)$ | H | $CH(CH_3)$ | X/Y-2 | 4,5-Br,Br |
| $CH(CH_3)$ | 3-F | $CH(CH_3)$ | X/Y-2 | 4,5-Cl,Cl |
| $CH(CH_3)$ | 3-F | $CH(CH_3)$ | X/Y-2 | 4,5-Br,Br |
| $CH_2CH_2$ | H | $CH_2$ | X/Y-2 | 4,5-Cl,Cl |
| $CH_2CH_2$ | H | $CH_2CH_2$ | X/Y-2 | 4,5-Br,Br |
| $CH_2CH_2CH_2$ | H | $CH_2$ | X/Y-2 | 4,5-Cl,Cl |
| $CH_2CH_2$ | H | $CH_2CH_2CH_2$ | X/Y-2 | 4,5-Br,Br |
| $CH_2CH(CH_3)$ | H | $CH_2CH_2$ | X/Y-2 | 4,5-Br,Br |
| $CH_2CH_2$ | 3-F | $CH_2CH(CH_3)$ | X/Y-2 | 4,5-Cl,Cl |
| $CH_2$ | H | $CH_2$ | X/Y-15 | - |
| $CH_2$ | H | $CH_2$ | X/Y-15 | 2-$CH_3$ |
| $CH_2$ | H | $CH_2$ | X/Y-15 | 2-Cyclopropyl |
| $CH_2$ | H | $CH_2$ | X/Y-15 | 2-$OCH_2CH_3$ |
| $CH_2$ | H | $CH_2$ | X/Y-15 | 2-Cl |
| $CH(CH_3)$ | H | $CH_2$ | X/Y-15 | 2-Cyclopropyl |
| $CH(CH_3)$ | H | $CH_2$ | X/Y-15 | 2-$OCH_2CH_3$ |
| $CH_2$ | H | $CH(CH_3)$ | X/Y-15 | 2-Cyclopropyl |
| $CH_2$ | H | $CH(CH_3)$ | X/Y-15 | 2-$OCH_2CH_3$ |
| $CH(CH_3)$ | H | $CH(CH_3)$ | X/Y-15 | 2-Cyclopropyl |
| $CH(CH_3)$ | H | $CH(CH_3)$ | X/Y-15 | 2-$OCH_2CH_3$ |

Tabelle F (Forts.)

| A | $R^1$ | B | Y | Substituent an Y |
|---|---|---|---|---|
| $CH(CH_3)$ | 3–F | $CH(CH_3)$ | X/Y–15 | 2-Cyclopropyl |
| $CH(CH_3)$ | 3–F | $CH(CH_3)$ | X/Y–15 | $2-OCH_2CH_3$ |
| $CH_2CH_2$ | H | $CH_2$ | X/Y–15 | 2-Cyclopropyl |
| $CH_2CH_2$ | H | $CH_2CH_2$ | X/Y–15 | $2-OCH_2CH_3$ |
| $CH_2CH_2CH_2$ | H | $CH_2$ | X/Y–15 | 2-Cyclopropyl |
| $CH_2CH_2$ | H | $CH_2CH_2CH_2$ | X/Y–15 | $2-OCH_2CH_3$ |
| $CH_2CH(CH_3)$ | H | $CH_2CH_2$ | X/Y–15 | 2-Cyclopropyl |
| $CH_2CH_2$ | 3–F | $CH_2CH(CH_3)$ | X/Y–15 | $2-OCH_2CH_3$ |
| $CH_2$ | H | $CH_2$ | X/Y–12 | – |
| $CH_2$ | H | $CH_2$ | X/Y–12 | $2-CH_3$ |
| $CH_2$ | H | $CH_2$ | X/Y–12 | 2-Cyclopropyl |
| $CH_2$ | H | $CH_2$ | X/Y–12 | $2-OCH_2CH_3$ |
| $CH(CH_3)$ | H | $CH_2$ | X/Y–12 | 2-Cyclopropyl |
| $CH(CH_3)$ | H | $CH_2$ | X/Y–12 | $2-OCH_2CH_3$ |
| $CH_2$ | H | $CH(CH_3)$ | X/Y–12 | 2-Cyclopropyl |
| $CH_2$ | H | $CH(CH_3)$ | X/Y–12 | $2-OCH_2CH_3$ |
| $CH(CH_3)$ | H | $CH(CH_3)$ | X/Y–12 | 2-Cyclopropyl |
| $CH(CH_3)$ | 3–F | $CH(CH_3)$ | X/Y–12 | $2-OCH_2CH_3$ |
| $CH_2CH_2$ | H | $CH_2$ | X/Y–12 | 2-Cyclopropyl |
| $CH_2CH_2$ | H | $CH_2CH_2$ | X/Y–12 | $2-OCH_2CH_3$ |
| $CH_2CH_2CH_2$ | H | $CH_2$ | X/Y–12 | 2-Cyclopropyl |
| $CH_2CH_2$ | H | $CH_2CH_2CH_2$ | X/Y–12 | $2-OCH_2CH_3$ |
| $CH_2CH(CH_3)$ | H | $CH_2CH_2$ | X/Y–12 | 2-Cyclopropyl |
| $CH_2CH_2$ | 3–F | $CH_2CH(CH_3)$ | X/Y–12 | 2-Cyclopropyl |
| $CH_2$ | H | $CH_2$ | X/Y–18 | $1-CH_3$ |
| $CH_2$ | H | $CH_2$ | X/Y–18 | $1,2-(CH_3)_2$ |
| $CH_2$ | H | $CH_2$ | X/Y–18 | $1,4-(CH_3)_2$ |
| $CH_2$ | H | $CH_2$ | X/Y–18 | $1,2,4-(CH_3)_2$ |
| $CH_2$ | H | $CH_2$ | X/Y–18 | $1-CH_3$, 2-Cyclopropyl |
| $CH(CH_3)$ | H | $CH_2$ | X/Y–18 | $1-CH_3$, 2-Cyclopropyl |
| $CH(CH_3)$ | H | $CH_2$ | X/Y–18 | $1,2,4-(CH_3)_3$ |
| $CH_2$ | H | $CH(CH_3)$ | X/Y–18 | $1-CH_3$, 2-Cyclopropyl |
| $CH_2$ | H | $CH(CH_3)$ | X/Y–18 | $1,2,4-(CH_3)_3$ |
| $CH(CH_3)$ | H | $CH(CH_3)$ | X/Y–18 | $1-CH_3$, 2-Cyclopropyl |
| $CH(CH_3)$ | 3–F | $CH(CH_3)$ | X/Y–18 | $1,2,4-(CH_3)_3$ |

Tabelle F (Forts.)

| A | R1 | B | Y | Substituent an Y |
|---|---|---|---|---|
| $CH_2CH_2$ | H | $CH_2$ | X/Y-18 | 1-$CH_3$, 2-Cyclopropyl |
| $CH_2CH_2$ | H | $CH_2CH_2$ | X/Y-18 | 1,2,4-$(CH_3)_2$ |
| $CH_2CH_2CH_2$ | H | $CH_2$ | X/Y-18 | 1-$CH_3$, 2-Cyclopropyl |
| $CH_2CH_2$ | H | $CH_2CH_2CH_2$ | X/Y-18 | 1,2,4-$(CH_3)_2$ |
| $CH_2CH(CH_3)$ | H | $CH_2CH_2$ | X/Y-18 | 1-$CH_3$, 2-Cyclopropyl |
| $CH_2CH_2$ | 3-F | $CH_2CH(CH_3)$ | X/Y-18 | 1,2,4-$(CH_3)_2$ |
| $CH_2$ | H | $CH_2$ | X/Y-19 | 1-$CH_3$ |
| $CH_2$ | H | $CH_2$ | X/Y-19 | 1,2-$(CH_3)_2$ |
| $CH_2$ | H | $CH_2$ | X/Y-19 | 1,5-$(CH_3)_2$ |
| $CH_2$ | H | $CH_2$ | X/Y-19 | 1-$CH_3$, 2-Cyclopropyl |
| $CH(CH_3)$ | H | $CH_2$ | X/Y-19 | 1,2-$(CH_3)_2$ |
| $CH(CH_3)$ | H | $CH_2$ | X/Y-19 | 1-$CH_3$, 2-Cyclopropyl |
| $CH_2$ | H | $CH(CH_3)$ | X/Y-19 | 1,2-$(CH_3)_2$ |
| $CH_2$ | H | $CH(CH_3)$ | X/Y-19 | 1-$CH_3$, 2-Cyclopropyl |
| $CH(CH_3)$ | H | $CH(CH_3)$ | X/Y-19 | 1,2-$(CH_3)_2$ |
| $CH(CH_3)$ | H | $CH(CH_3)$ | X/Y-19 | 1-$CH_3$, 2-Cyclopropyl |
| $CH(CH_3)$ | 3-F | $CH_2$ | X/Y-19 | 1-$CH_3$, 2-Cyclopropyl |
| $CH_2CH_2$ | H | $CH_2$ | X/Y-19 | 1-$CH_3$, 2-Cyclopropyl |
| $CH_2CH_2$ | H | $CH_2CH_2$ | X/Y-19 | 1,2-$(CH_3)_2$ |
| $CH_2CH_2CH_2$ | H | $CH_2$ | X/Y-19 | 1-$CH_3$, 2-Cyclopropyl |
| $CH_2CH_2$ | H | $CH_2CH_2CH_2$ | X/Y-19 | 1,2-$(CH_3)_2$ |
| $CH_2CH(CH_3)$ | H | $CH_2CH_2$ | X/Y-19 | 1-$CH_3$, 2-Cyclopropyl |
| $CH_2CH_2$ | 3-F | $CH_2CH(CH_3)$ | X/Y-19 | 1,2-$(CH_3)_2$ |
| $CH_2$ | H | $CH_2$ | X/Y-16 | - |
| $CH_2$ | H | $CH_2$ | X/Y-16 | 2-$CH_3$ |
| $CH_2$ | H | $CH_2$ | X/Y-16 | 2-Cyclopropyl |
| $CH_2$ | H | $CH_2$ | X/Y-16 | 2-Cl |
| $CH_2$ | H | $CH_2$ | X/Y-16 | 2-$OCH_2CH_3$ |
| $CH(CH_3)$ | H | $CH_2$ | X/Y-16 | 2-$CH_3$ |
| $CH(CH_3)$ | H | $CH_2$ | X/Y-16 | 2-Cyclopropyl |
| $CH_2$ | H | $CH(CH_3)$ | X/Y-16 | 2-$CH_3$ |
| $CH_2$ | H | $CH(CH_3)$ | X/Y-16 | 2-Cyclopropyl |
| $CH_2(CH_3)$ | H | $CH(CH_3)$ | X/Y-16 | 2-$CH_3$ |
| $CH_2(CH_3)$ | H | $CH(CH_3)$ | X/Y-16 | 2-Cyclopropyl |
| $CH_2(CH_3)$ | 3-F | $CH(CH_3)$ | X/Y-16 | 2-Cyclopropyl |

Tabelle F (Forts.)

| A | $R^1$ | B | Y | Substituent an Y |
|---|---|---|---|---|
| $CH_2CH_2$ | H | $CH_2$ | X/Y-16 | 2-Cyclopropyl |
| $CH_2CH_2$ | H | $CH_2CH_2$ | X/Y-16 | 2-$CH_3$ |
| $CH_2CH_2CH_2$ | H | $CH_2$ | X/Y-16 | 2-Cyclopropyl |
| $CH_2CH_2$ | H | $CH_2CH_2CH_2$ | X/Y-16 | 2-$CH_3$ |
| $CH_2CH(CH_3)$ | H | $CH_2CH_2$ | X/Y-16 | 2-Cyclopropyl |
| $CH_2CH_2$ | 3-F | $CH_2CH(CH_3)$ | X/Y-16 | 2-$CH_3$ |
| $CH_2$ | H | $CH_2$ | X/Y-13 | – |
| $CH_2$ | H | $CH_2$ | X/Y-13 | 2-$CH_3$ |
| $CH_2$ | H | $CH_2$ | X/Y-13 | 2-Cyclopropyl |
| $CH_2$ | H | $CH_2$ | X/Y-13 | 2-$OCH_2CH_3$ |
| $CH(CH_3)$ | H | $CH_2$ | X/Y-13 | 2-$OCH_2CH_3$ |
| $CH(CH_3)$ | H | $CH_2$ | X/Y-13 | 2-Cyclopropyl |
| $CH_2$ | H | $CH(CH_3)$ | X/Y-13 | 2-$OCH_2CH_3$ |
| $CH_2$ | H | $CH(CH_3)$ | X/Y-13 | 2-Cyclopropyl |
| $CH(CH_3)$ | H | $CH(CH_3)$ | X/Y-13 | 2-$OCH_2CH_3$ |
| $CH(CH_3)$ | 3-F | $CH(CH_3)$ | X/Y-13 | 2-Cyclopropyl |
| $CH_2CH_2$ | H | $CH_2$ | X/Y-13 | 2-Cyclopropyl |
| $CH_2CH_2$ | H | $CH_2CH_2$ | X/Y-13 | 2-$CH_3$ |
| $CH_2CH_2CH_2$ | H | $CH_2$ | X/Y-13 | 2-Cyclopropyl |
| $CH_2CH_2$ | H | $CH_2CH_2CH_2$ | X/Y-13 | 2-$CH_3$ |
| $CH(CH_3)CH_2$ | 3-F | $CH_2CH_2$ | X/Y-13 | 2-Cyclopropyl |
| $CH_2CH_2$ | H | $CH(CH_3)CH_2$ | X/Y-13 | 2-$CH_3$ |
| $CH_2$ | H | $CH_2$ | X/Y-6 | – |
| $CH_2$ | H | $CH_2$ | X/Y-6 | 3-$CH_3$ |
| $CH_2$ | H | $CH_2$ | X/Y-6 | 3-Cyclopropyl |
| $CH_2$ | H | $CH_2$ | X/Y-6 | 3-$OCH_2CH_3$ |
| $CH(CH_3)$ | H | $CH(CH_3)$ | X/Y-6 | 3-Cyclopropyl |
| $CH(CH_3)$ | 3-F | $CH(CH_3)$ | X/Y-6 | 3-Cyclopropyl |
| $CH_2CH_2$ | H | $CH_2CH_2$ | X/Y-6 | 3-Cyclopropyl |
| $CH_2CH_2$ | H | $CH_2CH_2CH_2$ | X/Y-6 | 3-$CH_3$ |
| $CH(CH_3)CH_2$ | 3-F | $CH_2CH_2$ | X/Y-6 | 3-Cyclopropyl |
| $CH_2CH_2$ | H | $CH(CH_3)CH_2$ | X/Y-6 | 3-Cyclopropyl |
| $CH_2$ | H | $CH_2$ | X/Y-8 | 1-$CH_3$ |
| $CH_2$ | H | $CH_2$ | X/Y-8 | 1,3-$(CH_3)_2$ |
| $CH_2$ | H | $CH_2$ | X/Y-8 | 1,4-$(CH_3)_2$ |

Tabelle F (Forts.)

| A | $R^1$ | B | Y | Substituent an Y |
|---|---|---|---|---|
| $CH_2$ | H | $CH_2$ | X/Y-8 | 1-$CH_3$, 3-Cyclopropyl |
| $CH(CH_3)$ | H | $CH(CH_3)$ | X/Y-8 | 1-$CH_3$, 3-Cyclopropyl |
| $CH(CH_3)$ | 3-F | $CH(CH_3)$ | X/Y-8 | 1-$CH_3$, 3-Cyclopropyl |
| $CH_2CH_2$ | H | $CH_2CH_2$ | X/Y-8 | 1-$CH_3$, 3-Cyclopropyl |
| $CH_2CH_2$ | H | $CH_2CH_2CH_2$ | X/Y-8 | 1,3-$(CH_3)_2$ |
| $CH_2CH(CH_3)$ | H | $CH_2CH_2$ | X/Y-8 | 1-$CH_3$, 3-Cyclopropyl |
| $CH(CH_3)CH_2$ | 3-F | $CH_2CH_2$ | X/Y-8 | 1-$CH_3$, 3-Cyclopropyl |
| $CH_2$ | H | $CH_2$ | X/Y-10 | 1-$CH_3$ |
| $CH_2$ | H | $CH_2$ | X/Y-10 | 1,4-$(CH_3)_2$ |
| $CH_2$ | H | $CH_2$ | X/Y-10 | 1-$CH_3$, 4-$OCH_3$ |
| $CH_2$ | H | $CH_2$ | X/Y-10 | 1-$CH_3$, 5-$CH(CH_3)_2$ |
| $CH_2$ | H | $CH_2$ | X/Y-10 | 1-$CH_3$, 5-Cyclopropyl |
| $CH(CH_3)$ | H | $CH(CH_3)$ | X/Y-10 | 1-$CH_3$, 5-Cyclopropyl |
| $CH(CH_3)$ | 3-F | $CH(CH_3)$ | X/Y-10 | 1,4-$(CH_3)_2$ |
| $CH_2CH_2$ | H | $CH_2CH_2$ | X/Y-10 | 1-$CH_3$, 5-Cyclopropyl |
| $CH_2CH_2$ | H | $CH_2CH_2CH_2$ | X/Y-10 | 1,5-$(CH_3)_2$ |
| $CH_2CH(CH_3)$ | H | $CH_2CH_2$ | X/Y-10 | 1-$CH_3$, 5-Cyclopropyl |
| $CH(CH_3)CH_2$ | 3-F | $CH_2CH_2$ | X/Y-10 | 1-$CH_3$, 5-Cyclopropyl |
| $CH_2$ | H | $CH_2$ | X/Y-7 | - |
| $CH_2$ | H | $CH_2$ | X/Y-7 | 3-$CH_3$ |
| $CH(CH_3)$ | H | $CH(CH_3)$ | X/Y-7 | 3-$CH_3$ |
| $CH(CH_3)$ | 3-F | $CH(CH_3)$ | X/Y-7 | 3-$CH_3$ |
| $CH_2CH_2$ | H | $CH_2CH_2$ | X/Y-7 | 3-$CH_3$ |
| $CH_2CH_2$ | 3-F | $CH(CH_3)CH_2CH_2$ | X/Y-7 | 3-$CH_3$ |
| $CH_2$ | H | $CH_2$ | X/Y-9 | 1-$CH_3$ |
| $CH_2$ | H | $CH_2$ | X/Y-9 | 1,3-$(CH_3)_2$ |
| $CH_2$ | H | $CH_2$ | X/Y-9 | 1,5-$(CH_3)_2$ |
| $CH(CH_3)$ | H | $CH(CH_3)$ | X/Y-9 | 1,3-$(CH_3)_2$ |
| $CH(CH_3)$ | 3-F | $CH(CH_3)$ | X/Y-9 | 1,5-$(CH_3)_2$ |
| $CH_2CH_2$ | H | $CH_2CH_2$ | X/Y-9 | 1,3-$(CH_3)_2$ |
| $CH_2CH_2$ | H | $CH_2CH_2CH_2$ | X/Y-9 | 1,5-$(CH_3)_2$ |
| $CH_2CH(CH_3)$ | H | $CH_2CH_2$ | X/Y-9 | 1,3-$(CH_3)_2$ |
| $CH(CH_3)CH_2$ | 3-F | $CH_2CH_2$ | X/Y-9 | 1,5-$(CH_3)_2$ |
| $CH_2$ | H | $CH_2$ | X/Y-25 | 5-Cyclopropyl |
| $CH_2$ | H | $CH_2$ | X/Y-25 | 5-$CH_3$ |

Tabelle F (Forts.)

| A | $R^1$ | B | Y | Substituent an Y |
|---|---|---|---|---|
| $CH_2$ | H | $CH_2$ | X/Y-25 | 5-$CH_2CH_3$ |
| $CH_2$ | H | $CH_2$ | X/Y-25 | 5-$CH(CH_3)_2$ |
| $CH_2$ | H | $CH_2$ | X/Y-25 | 5-$OCH_3$ |
| $CH(CH_3)$ | H | $CH(CH_3)$ | X/Y-25 | 5-Cyclopropyl |
| $CH_2(CH_3)$ | H | $CH(CH_3)$ | X/Y-25 | 5-$CH_3$ |
| $CH(CH_3)$ | H | $CH(CH_3)$ | X/Y-25 | 5-$OCH_2CH_3$ |
| $CH(CH_3)$ | 3-F | $CH(CH_3)$ | X/Y-25 | 5-Cyclopropyl |
| $CH_2CH_2$ | H | $CH_2$ | X/Y-25 | 5-Cyclopropyl |
| $CH_2CH_2$ | H | $CH_2CH_2$ | X/Y-25 | 5-$CH_3$ |
| $CH_2CH_2CH_2$ | H | $CH_2$ | X/Y-25 | 5-$OCH_2CH_3$ |
| $CH_2CH_2$ | 3-F | $CH_2CH(CH_3)$ | X/Y-25 | 5-Cyclopropyl |
| $CH_2$ | H | $CH(CH_3)$ | X/Y-25 | 5-Cyclopropyl |
| $CH_2$ | H | $CH_2$ | X/Y-23 | – |
| $CH_2$ | H | $CH_2$ | X/Y-23 | 5-Cyclopropyl |
| $CH_2$ | H | $CH_2$ | X/Y-23 | 5-$CH_3$ |
| $CH_2$ | H | $CH_2$ | X/Y-23 | 5-$CH(CH_3)_2$ |
| $CH_2$ | H | $CH_2$ | X/Y-23 | 5-Cl |
| $CH_2$ | H | $CH_2$ | X/Y-23 | 5-$OCH_2CH_3$ |
| $CH_2$ | H | $CH(CH_3)$ | X/Y-23 | 5-Cyclopropyl |
| $CH_2$ | 3-F | $CH(CH_3)$ | X/Y-23 | 5-$CH_3$ |
| $CH_2$ | H | $CH(CH_3)$ | X/Y-23 | 5-$CH(CH_3)_2$ |
| $CH(CH_3)$ | H | $CH(CH_3)$ | X/Y-23 | 5-Cyclopropyl |
| $CH_2$ | H | $CH_2CH_2$ | X/Y-23 | 5-Cyclopropyl |
| $CH_2CH_2CH_2$ | H | $CH_2$ | X/Y-23 | 5-$CH_3$ |
| $CH_2CH_2$ | H | $CH_2CH_2$ | X/Y-23 | 5-$CH(CH_3)_2$ |
| $CH_2CH_2$ | H | $CH_2CH_2$ | X/Y-23 | 5-Cyclopropyl |
| $CH_2$ | H | $CH_2$ | X/Y-26 | 1-$CH_3$ |
| $CH_2$ | H | $CH_2$ | X/Y-26 | 1,5-$(CH_3)_2$ |
| $CH_2$ | H | $CH_2$ | X/Y-26 | 1-$CH_3$, 5-Cyclopropyl |
| $CH(CH_3)$ | H | $CH(CH_3)$ | X/Y-26 | 1-$CH_3$, 5-Cyclopropyl |
| $CH(CH_3)$ | H | $CH(CH_3)$ | X/Y-26 | 1,5-$(CH_3)_2$ |
| $CH(CH_3)$ | 3-F | $CH_2$ | X/Y-26 | 1-$CH_3$, 5-Cyclopropyl |
| $CH_2CH_2$ | H | $CH_2CH_2$ | X/Y-26 | 1-$CH_3$, 5-Cyclopropyl |
| $CH_2$ | H | $CH_2CH_2$ | X/Y-26 | 1,5-$(CH_3)_2$ |
| $CH_2$ | H | $CH_2$ | X/Y-28 | 1-$CH_3$ |
| $CH_2$ | H | $CH_2$ | X/Y-28 | 1,5-$(CH_3)_2$ |
| $CH_2$ | H | $CH_2$ | X/Y-28 | 1-$CH_3$, 5-Cyclopropyl |

Tabelle F (Forts.)

| A | R1 | B | Y | Substituent an Y |
|---|---|---|---|---|
| $CH(CH_3)$ | H | $CH(CH_3)$ | X/Y-28 | 1-$CH_3$, 5-Cyclopropyl |
| $CH(CH_3)$ | H | $CH(CH_3)$ | X/Y-28 | 1,5-$(CH_3)_2$ |
| $CH(CH_3)$ | 3-F | $CH_2$ | X/Y-28 | 1-$CH_3$, 5-Cyclopropyl |
| $CH_2CH_2$ | H | $CH_2CH_2$ | X/Y-28 | 1-$CH_3$, 5-Cyclopropyl |
| $CH_2$ | H | $CH_2CH_2$ | X/Y-28 | 1,5-$(CH_3)_2$ |
| $CH_2$ | H | $CH_2$ | X/Y-22 | – |
| $CH_2$ | H | $CH_2$ | X/Y-22 | 3-$CH_3$ |
| $CH_2$ | H | $CH_2$ | X/Y-22 | 3-Cyclopropyl |
| $CH_2$ | H | $CH_2$ | X/Y-22 | 3-$OCH_3$ |
| $CH(CH_3)$ | H | $CH_2$ | X/Y-22 | 3-$CH_3$ |
| $CH_2$ | H | $CH(CH_3)$ | X/Y-22 | 3-Cyclopropyl |
| $CH_2$ | 3-F | $CH(CH_3)$ | X/Y-22 | 3-$OCH_3$ |
| $CH_2CH_2$ | H | $CH_2CH_2$ | X/Y-22 | 3-Cyclopropyl |
| $CH_2$ | H | $CH_2CH_2$ | X/Y-22 | 3-$CH_3$ |
| $CH_2$ | H | $CH_2$ | X/Y-29 | 1-$CH_3$ |
| $CH_2$ | H | $CH_2$ | X/Y-29 | 1,3-$(CH_3)_2$ |
| $CH_2$ | H | $CH_2$ | X/Y-29 | 1-$CH_3$, 3-Cyclopropyl |
| $CH(CH_3)$ | H | $CH(CH_3)$ | X/Y-29 | 1,3-$(CH_3)_2$ |
| $CH(CH_3)$ | 3-F | $CH(CH_3)$ | X/Y-29 | 1-$CH_3$, 3-Cyclopropyl |
| $CH_2CH_2$ | H | $CH_2CH_2CH_2$ | X/Y-29 | 1,3-$(CH_3)_2$ |
| $CH_2CH_2$ | H | $CH_2CH_2$ | X/Y-29 | 1-$CH_3$, 3-Cyclopropyl |
| $CH_2$ | H | $CH_2$ | X/Y-17 | – |
| $CH_2$ | H | $CH_2$ | X/Y-17 | 5-Cyclopropyl |
| $CH_2$ | H | $CH_2$ | X/Y-17 | 5-$CH_3$ |
| $CH_2$ | H | $CH_2$ | X/Y-17 | 5-$OCH_2CH_3$ |
| $CH_2$ | H | $CH_2$ | X/Y-17 | 5-Cl |
| $CH_2$ | H | $CH_2$ | X/Y-17 | 4-$CH_3$ |
| $CH_2$ | H | $CH_2$ | X/Y-17 | 4-Cyclopropyl |
| $CH_2$ | H | $CH_2$ | X/Y-17 | 4-$OCH_2CH_3$ |
| $CH_2$ | H | $CH_2$ | X/Y-17 | 4-Cl |
| $CH(CH_3)$ | H | $CH(CH_3)$ | X/Y-17 | 5-Cyclopropyl |
| $CH(CH_3)$ | H | $CH(CH_3)$ | X/Y-17 | 4-Cyclopropyl |
| $CH(CH_3)$ | 3-F | $CH(CH_3)$ | X/Y-17 | 5-$OCH_2CH_3$ |
| $CH_2CH_2$ | H | $CH_2CH_2$ | X/Y-17 | 5-Cyclopropyl |
| $CH_2CH_2$ | H | $CH_2CH_2$ | X/Y-17 | 4-Cyclopropyl |

Tabelle F (Forts.)

| A | $R^1$ | B | Y | Substituent an Y |
|---|---|---|---|---|
| $CH_2CH_2$ | H | $CH_2CH_2CH_2$ | X/Y-17 | 5-$CH_3$ |
| $CH_2$ | H | $CH_2$ | X/Y-14 | – |
| $CH_2$ | H | $CH_2$ | X/Y-14 | 5-$CH_3$ |
| $CH_2$ | H | $CH_2$ | X/Y-14 | 5-Cyclopropyl |
| $CH_2$ | H | $CH_2$ | X/Y-14 | 4-$CH_3$ |
| $CH_2$ | H | $CH_2$ | X/Y-14 | 4-Cyclopropyl |
| $CH_2$ | H | $CH_2$ | X/Y-14 | 4-$OCH_2CH_3$ |
| $CH_2$ | H | $CH_2$ | X/Y-14 | 5-$OCH_2CH_3$ |
| $CH_2$ | H | $CH_2$ | X/Y-14 | 5-Cl |
| $CH(CH_3)$ | H | $CH(CH_3)$ | X/Y-14 | 4-Cyclopropyl |
| $CH(CH_3)$ | H | $CH(CH_3)$ | X/Y-14 | 5-Cyclopropyl |
| $CH(CH_3)$ | H | $CH(CH_3)$ | X/Y-14 | 4-$CH_3$ |
| $CH(CH_3)$ | 3-F | $CH(CH_3)$ | X/Y-14 | 4-Cyclopropyl |
| $CH_2$ | 3-F | $CH_2$ | X/Y-14 | 5-$OCH_2CH_3$ |
| $CH_2CH_2$ | H | $CH_2CH_2$ | X/Y-14 | 4-Cyclopropyl |
| $CH_2CH_2$ | H | $CH_2CH_2$ | X/Y-14 | 5-Cyclopropyl |
| $CH_2CH_2$ | H | $CH_2CH_2CH_2$ | X/Y-14 | 5-Cl |
| $CH_2CH_2$ | H | $CH_2CH_2$ | X/Y-14 | 4-$CH_3$ |
| $CH_2$ | H | $CH_2$ | X/Y-20 | 1-$CH_3$ |
| $CH_2$ | H | $CH_2$ | X/Y-20 | 1,5-$(CH_3)_2$ |
| $CH(CH_3)$ | H | $CH(CH_3)$ | X/Y-20 | 1-$CH_3$ |
| $CH(CH_3)$ | 3-F | $CH(CH_3)$ | X/Y-20 | 1,5-$(CH_3)_2$ |
| $CH_2CH_2$ | H | $CH_2CH_2$ | X/Y-20 | 1-$CH_3$ |
| $CH_2CH_2$ | H | $CH_2CH_2$ | X/Y-20 | 1,5-$(CH_3)_2$ |
| $CH(CH_3)CH_2$ | 3-F | $CH(CH_3)CH_2$ | X/Y-20 | 1,5-$(CH_3)_2$ |
| $CH_2$ | H | $CH_2$ | X/Y-3 | – |
| $CH_2$ | H | $CH_2$ | X/Y-3 | 3-$CH_3$ |
| $CH_2$ | H | $CH_2$ | X/Y-3 | 3-$CH_2CH_3$ |
| $CH_2$ | H | $CH_2$ | X/Y-3 | 3-$CH(CH_3)_2$ |
| $CH_2$ | H | $CH_2$ | X/Y-3 | 3-$OCH_2CH_3$ |
| $CH_2$ | H | $CH_2$ | X/Y-3 | 3-$CH_2OCH_3$ |
| $CH_2$ | H | $CH_2$ | X/Y-3 | 3-$CF_3$ |
| $CH(CH_3)$ | H | $CH_2$ | X/Y-3 | 3-$CH_3$ |
| $CH(CH_3)$ | H | $CH_2$ | X/Y-3 | 3-$CH(CH_3)_2$ |

Tabelle F (Forts.)

| A | $R^1$ | B | Y | Substituent an Y |
|---|---|---|---|---|
| $CH(CH_3)$ | H | $CH_2$ | X/Y-3 | 3-Cyclopropyl |
| $CH_2$ | H | $CH(CH_3)$ | X/Y-3 | 3-$CH_3$ |
| $CH_2$ | H | $CH(CH_3)$ | X/Y-3 | 3-$CH(CH_3)_2$ |
| $CH_2$ | H | $CH(CH_3)$ | X/Y-3 | 3-Cyclopropyl |
| $CH(CH_3)$ | H | $CH(CH_3)$ | X/Y-3 | 3-$CH_3$ |
| $CH(CH_3)$ | 3-F | $CH(CH_3)$ | X/Y-3 | 3-$CH(CH_3)_2$ |
| $CH(CH_3)$ | H | $CH(CH_3)$ | X/Y-3 | 3-Cyclopropyl |
| $CH_2$ | H | $CH_2CH_2$ | X/Y-3 | 3-$CH_3$ |
| $CH_2CH_2$ | H | $CH_2CH_2CH_2$ | X/Y-3 | 3-$CH(CH_3)_2$ |
| $CH_2$ | H | $CH_2CH_2$ | X/Y-3 | 3-Cyclopropyl |
| $CH_2CH_2$ | H | $CH_2CH(CH_3)$ | X/Y-3 | 3-Cyclopropyl |
| $CH_2CH_2$ | H | $CH_2CH(CH_3)$ | X/Y-3 | 3-$CH(CH_3)_2$ |
| $CH_2CH_2CH_2$ | 3-F | $CH(CH_3)CH_2$ | X/Y-3 | 3-$CH_3$ |
| $CH_2$ | H | $CH_2$ | X/Y-5 | - |
| $CH_2$ | H | $CH_2$ | X/Y-5 | 5-$OCH_2CH_3$ |
| $CH_2$ | H | $CH_2$ | X/Y-5 | 5-Cyclopropyl |
| $CH_2$ | H | $CH_2$ | X/Y-5 | 5-$CH_3$ |
| $CH(CH_3)$ | 3-F | $CH(CH_3)$ | X/Y-5 | 5-$CH_3$ |
| $CH_2$ | H | $CH(CH_3)$ | X/Y-5 | 5-Cyclopropyl |
| $CH(CH_3)$ | H | $CH_2$ | X/Y-5 | 5-Cyclopropyl |
| $CH_2$ | H | $CH_2CH_2$ | X/Y-5 | 5-$OCH_2CH_3$ |
| $CH_2CH_2$ | H | $CH_2CH_2CH_2$ | X/Y-5 | 5-$CH_3$ |
| $CH_2$ | H | $CH_2$ | X/Y-4 | - |
| $CH_2$ | H | $CH_2$ | X/Y-4 | 3-$OCH_2CH_3$ |
| $CH_2$ | H | $CH_2$ | X/Y-4 | 5-$OCH_2CH_3$ |
| $CH_2$ | H | $CH_2$ | X/Y-4 | 3-$CH(CH_3)_2$ |
| $CH_2$ | H | $CH_2$ | X/Y-4 | 5-$CH_3$ |
| $CH(CH_3)$ | 3-F | $CH(CH_3)$ | X/Y-4 | 3-$CH(CH_3)_2$ |
| $CH_2$ | H | $CH_2$ | X/Y-11 | - |
| $CH_2$ | H | $CH_2$ | X/Y-11 | 3-$CH_3$ |
| $CH_2$ | H | $CH_2$ | X/Y-11 | 5-$CH_3$ |
| $CH_2$ | H | $CH(CH_3)$ | X/Y-11 | 3-$CH_3$ |
| $CH_2$ | 3-F | $CH(CH_3)$ | X/Y-11 | 5-$CH_3$ |
| $CH(CH_3)$ | H | $CH(CH_3)$ | X/Y-11 | 5-$CH_3$ |
| $CH_2$ | H | $CH_2$ | X/Y-21 | - |

Tabelle F (Forts.)

| A | $R^1$ | B | Y | Substituent an Y |
|---|---|---|---|---|
| $CH_2$ | H | $CH_2$ | X/Y-21 | 4,5-$(CH_3)_2$ |
| $CH_2$ | H | $CH_2$ | X/Y-21 | 4,5-$Br_2$ |
| $CH(CH_3)$ | H | $CH(CH_3)$ | X/Y-21 | 4,5-$Cl_2$ |
| $CH(CH_3)$ | H | $CH(CH_3)$ | X/Y-21 | 4,5-$(CH_3)_2$ |
| $CH(CH_3)$ | H | $CH(CH_3)$ | X/Y-21 | 4,5-$Br_2$ |
| $CH_2$ | H | $CH_2$ | X/Y-30 | – |
| $CH_2$ | H | $CH_2$ | X/Y-30 | 3,5-$(CH_3)_2$ |
| $CH(CH_3)$ | H | $CH(CH_3)$ | X/Y-30 | – |
| $CH(CH_3)$ | H | $CH(CH_3)$ | X/Y-30 | 3,5-$(CH_3)_2$ |
| $CH_2$ | H | $CH_2$ | X/Y-27 | – |
| $CH_2$ | H | $CH_2$ | X/Y-27 | 2-Cl |
| $CH_2$ | H | $CH_2$ | X/Y-27 | 2,5-$(CH_3)_2$ |
| $CH(CH_3)$ | H | $CH(CH_3)$ | X/Y-27 | – |
| $CH(CH_3)$ | H | $CH(CH_3)$ | X/Y-27 | 2-Cl |
| $CH(CH_3)$ | H | $CH(CH_3)$ | X/Y-27 | 2,5-$(CH_3)_2$ |

Tabelle G (zu Struktur I.G).

| A | R1 | B | Y | Substituent an Y |
|---|---|---|---|---|
| $CH_2$ | H | $CH_2$ | X/Y-1 | |
| $CH(CH_3)$ | H | $CH_2$ | X/Y-1 | |
| $CH(CH_2CH_3)$ | H | $CH_2$ | X/Y-1 | |
| $CH_2$ | H | $CH(CH_3)$ | X/Y-1 | |
| $CH_2$ | H | $CH(CH_2CH_3)$ | X/Y-1 | |
| $CH_2$ | 3-$CH_3$ | $CH_2$ | X/Y-1 | |
| $CH_2$ | 3-F | $CH_2$ | X/Y-1 | |
| $CH_2$ | H | $CH_2$ | X/Y-1 | 5-$CH_3$ |
| $CH_2$ | H | $CH_2$ | X/Y-1 | 4-$CH_3$ |
| $CH_2$ | H | $CH_2$ | X/Y-1 | 3-$CH_3$ |
| $CH_2$ | H | $CH_2$ | X/Y-1 | 5-Cyclopropyl |
| $CH_2$ | H | $CH_2$ | X/Y-1 | 4-Cyclopropyl |
| $CH_2$ | H | $CH_2$ | X/Y-1 | 3-Cyclopropyl |
| $CH_2$ | H | $CH_2$ | X/Y-1 | 5-Cl |
| $CH_2$ | H | $CH_2$ | X/Y-1 | 5-Br |
| $CH_2$ | H | $CH_2$ | X/Y-1 | 4-Cl |
| $CH_2$ | H | $CH_2$ | X/Y-1 | 4-Br |
| $CH_2$ | H | $CH_2$ | X/Y-1 | 4,5-Cl,Cl |
| $CH_2$ | H | $CH_2$ | X/Y-1 | 4-$OCH_2CH_3$ |
| $CH(CH_3)$ | H | $CH_2$ | X/Y-1 | 5-Br |
| $CH(CH_3)$ | H | $CH_2$ | X/Y-1 | 4,5-Cl,Cl |
| $CH(CH_3)$ | H | $CH_2$ | X/Y-1 | 4-Cl |
| $CH_2$ | H | $CH(CH_3)$ | X/Y-1 | 5-Br |
| $CH_2$ | H | $CH(CH_3)$ | X/Y-1 | 4,5-Cl,Cl |
| $CH(CH_3)$ | H | $CH(CH_3)$ | X/Y-1 | – |
| $CH(CH_3$ | H | $CH(CH_3)$ | X/Y-1 | 4,5-Cl,Cl |
| $CH(CH_3)$ | 3-F | $CH(CH_3)$ | X/Y-1 | – |
| $CH(CH_3)$ | 3-F | $CH(CH_3)$ | X/Y-1 | 5-Br |
| $CH_2CH_2$ | H | $CH_2$ | X/Y-1 | 5-Br |
| $CH_2CH_2$ | H | $CH_2CH_2$ | X/Y-1 | 4,5-Cl,Cl |
| $CH_2CH_2CH_2$ | H | $CH_2$ | X/Y-1 | 5-Br |
| $CH_2CH_2$ | H | $CH_2CH_2CH_2$ | X/Y-1 | 4,5-Cl,Cl |
| $CH_2CH(CH_3)$ | H | $CH_2CH_2$ | X/Y-1 | 4,5-Cl,Cl |
| $CH_2CH_2$ | 3-F | $CH_2CH(CH_3)$ | X/Y-1 | 5-Br |

Tabelle G (Forts.)

| A | $R^1$ | B | Y | Substituent an Y |
|---|---|---|---|---|
| $CH_2$ | H | $CH_2$ | X/Y-2 | – |
| $CH_2$ | H | $CH_2$ | X/Y-2 | 4-Cyclopropyl |
| $CH_2$ | H | $CH_2$ | X/Y-2 | 5-Cyclopropyl |
| $CH_2$ | H | $CH_2$ | X/Y-2 | 5-Cl |
| $CH_2$ | H | $CH_2$ | X/Y-2 | 4,5-Cl,Cl |
| $CH_2$ | H | $CH_2$ | X/Y-2 | 4,5-Br,Br |
| $CH_2$ | H | $CH_2$ | X/Y-2 | $4-CH_3$ |
| $CH_2$ | H | $CH_2$ | X/Y-2 | $5-CH_3$ |
| $CH_2$ | H | $CH_2$ | X/Y-2 | $4-OCH_3$ |
| $CH_2$ | H | $CH_2$ | X/Y-2 | $5-OCH_3$ |
| $CH(CH_3)$ | H | $CH_2$ | X/Y-2 | 4,5-Cl,Cl |
| $CH(CH_3)$ | H | $CH_2$ | X/Y-2 | 4,5-Br,Br |
| $CH_2$ | H | $CH(CH_3)$ | X/Y-2 | 4,5-Cl,Cl |
| $CH_2$ | H | $CH(CH_3)$ | X/Y-2 | 4,5-Br,Br |
| $CH(CH_3)$ | H | $CH(CH_3)$ | X/Y-2 | 4,5-Cl,Cl |
| $CH(CH_3)$ | H | $CH(CH_3)$ | X/Y-2 | 4,5-Br,Br |
| $CH(CH_3)$ | 3-F | $CH(CH_3)$ | X/Y-2 | 4,5-Cl,Cl |
| $CH(CH_3)$ | 3-F | $CH(CH_3)$ | X/Y-2 | 4,5-Br,Br |
| $CH_2CH_2$ | H | $CH_2$ | X/Y-2 | 4,5-Cl,Cl |
| $CH_2CH_2$ | H | $CH_2CH_2$ | X/Y-2 | 4,5-Br,Br |
| $CH_2CH_2CH_2$ | H | $CH_2$ | X/Y-2 | 4,5-Cl,Cl |
| $CH_2CH_2$ | H | $CH_2CH_2CH_2$ | X/Y-2 | 4,5-Br,Br |
| $CH_2CH(CH_3)$ | H | $CH_2CH_2$ | X/Y-2 | 4,5-Br,Br |
| $CH_2CH_2$ | 3-F | $CH_2CH(CH_3)$ | X/Y-2 | 4,5-Cl,Cl |
| $CH_2$ | H | $CH_2$ | X/Y-15 | – |
| $CH_2$ | H | $CH_2$ | X/Y-15 | $2-CH_3$ |
| $CH_2$ | H | $CH_2$ | X/Y-15 | 2-Cyclopropyl |
| $CH_2$ | H | $CH_2$ | X/Y-15 | $2-OCH_2CH_3$ |
| $CH_2$ | H | $CH_2$ | X/Y-15 | 2-Cl |
| $CH(CH_3)$ | H | $CH_2$ | X/Y-15 | 2-Cyclopropyl |
| $CH(CH_3)$ | H | $CH_2$ | X/Y-15 | $2-OCH_2CH_3$ |
| $CH_2$ | H | $CH(CH_3)$ | X/Y-15 | 2-Cyclopropyl |
| $CH_2$ | H | $CH(CH_3)$ | X/Y-15 | $2-OCH_2CH_3$ |
| $CH(CH_3)$ | H | $CH(CH_3)$ | X/Y-15 | 2-Cyclopropyl |
| $CH(CH_3)$ | H | $CH(CH_3)$ | X/Y-15 | $2-OCH_2CH_3$ |

Tabelle G (Forts.)

| A | $R^1$ | B | Y | Substituent an Y |
|---|---|---|---|---|
| $CH(CH_3)$ | 3–F | $CH(CH_3)$ | X/Y–15 | 2-Cyclopropyl |
| $CH(CH_3)$ | 3–F | $CH(CH_3)$ | X/Y–15 | 2-$OCH_2CH_3$ |
| $CH_2CH_2$ | H | $CH_2$ | X/Y–15 | 2-Cyclopropyl |
| $CH_2CH_2$ | H | $CH_2CH_2$ | X/Y–15 | 2-$OCH_2CH_3$ |
| $CH_2CH_2CH_2$ | H | $CH_2$ | X/Y–15 | 2-Cyclopropyl |
| $CH_2CH_2$ | H | $CH_2CH_2CH_2$ | X/Y–15 | 2-$OCH_2CH_3$ |
| $CH_2CH(CH_3)$ | H | $CH_2CH_2$ | X/Y–15 | 2-Cyclopropyl |
| $CH_2CH_2$ | 3–F | $CH_2CH(CH_3)$ | X/Y–15 | 2-$OCH_2CH_3$ |
| $CH_2$ | H | $CH_2$ | X/Y–12 | – |
| $CH_2$ | H | $CH_2$ | X/Y–12 | 2-$CH_3$ |
| $CH_2$ | H | $CH_2$ | X/Y–12 | 2-Cyclopropyl |
| $CH_2$ | H | $CH_2$ | X/Y–12 | 2-$OCH_2CH_3$ |
| $CH(CH_3)$ | H | $CH_2$ | X/Y–12 | 2-Cyclopropyl |
| $CH(CH_3)$ | H | $CH_2$ | X/Y–12 | 2-$OCH_2CH_3$ |
| $CH_2$ | H | $CH(CH_3)$ | X/Y–12 | 2-Cyclopropyl |
| $CH_2$ | H | $CH(CH_3)$ | X/Y–12 | 2-$OCH_2CH_3$ |
| $CH(CH_3)$ | H | $CH(CH_3)$ | X/Y–12 | 2-Cyclopropyl |
| $CH(CH_3)$ | 3–F | $CH(CH_3)$ | X/Y–12 | 2-$OCH_2CH_3$ |
| $CH_2CH_2$ | H | $CH_2$ | X/Y–12 | 2-Cyclopropyl |
| $CH_2CH_2$ | H | $CH_2CH_2$ | X/Y–12 | 2-$OCH_2CH_3$ |
| $CH_2CH_2CH_2$ | H | $CH_2$ | X/Y–12 | 2-Cyclopropyl |
| $CH_2CH_2$ | H | $CH_2CH_2CH_2$ | X/Y–12 | 2-$OCH_2CH_3$ |
| $CH_2CH(CH_3)$ | H | $CH_2CH_2$ | X/Y–12 | 2-Cyclopropyl |
| $CH_2CH_2$ | 3–F | $CH_2CH(CH_3)$ | X/Y–12 | 2-Cyclopropyl |
| $CH_2$ | H | $CH_2$ | X/Y–18 | 1-$CH_3$ |
| $CH_2$ | H | $CH_2$ | X/Y–18 | 1,2-$(CH_3)_2$ |
| $CH_2$ | H | $CH_2$ | X/Y–18 | 1,4-$(CH_3)_2$ |
| $CH_2$ | H | $CH_2$ | X/Y–18 | 1,2,4-$(CH_3)_2$ |
| $CH_2$ | H | $CH_2$ | X/Y–18 | 1-$CH_3$, 2-Cyclopropyl |
| $CH(CH_3)$ | H | $CH_2$ | X/Y–18 | 1-$CH_3$, 2-Cyclopropyl |
| $CH(CH_3)$ | H | $CH_2$ | X/Y–18 | 1,2,4-$(CH_3)_3$ |
| $CH_2$ | H | $CH(CH_3)$ | X/Y–18 | 1-$CH_3$, 2-Cyclopropyl |
| $CH_2$ | H | $CH(CH_3)$ | X/Y–18 | 1,2,4-$(CH_3)_3$ |
| $CH(CH_3)$ | H | $CH(CH_3)$ | X/Y–18 | 1-$CH_3$, 2-Cyclopropyl |
| $CH(CH_3)$ | 3–F | $CH(CH_3)$ | X/Y–18 | 1,2,4-$(CH_3)_3$ |

Tabelle G (Forts.)

| A | $R^1$ | B | Y | Substituent an Y |
|---|---|---|---|---|
| $CH_2CH_2$ | H | $CH_2$ | X/Y-18 | 1-$CH_3$, 2-Cyclopropyl |
| $CH_2CH_2$ | H | $CH_2CH_2$ | X/Y-18 | 1,2,4-$(CH_3)_2$ |
| $CH_2CH_2CH_2$ | H | $CH_2$ | X/Y-18 | 1-$CH_3$, 2-Cyclopropyl |
| $CH_2CH_2$ | H | $CH_2CH_2CH_2$ | X/Y-18 | 1,2,4-$(CH_3)_2$ |
| $CH_2CH(CH_3)$ | H | $CH_2CH_2$ | X/Y-18 | 1-$CH_3$, 2-Cyclopropyl |
| $CH_2CH_2$ | 3-F | $CH_2CH(CH_3)$ | X/Y-18 | 1,2,4-$(CH_3)_2$ |
| $CH_2$ | H | $CH_2$ | X/Y-19 | 1-$CH_3$ |
| $CH_2$ | H | $CH_2$ | X/Y-19 | 1,2-$(CH_3)_2$ |
| $CH_2$ | H | $CH_2$ | X/Y-19 | 1,5-$(CH_3)_2$ |
| $CH_2$ | H | $CH_2$ | X/Y-19 | 1-$CH_3$, 2-Cyclopropyl |
| $CH(CH_3)$ | H | $CH_2$ | X/Y-19 | 1,2-$(CH_3)_2$ |
| $CH(CH_3)$ | H | $CH_2$ | X/Y-19 | 1-$CH_3$, 2-Cyclopropyl |
| $CH_2$ | H | $CH(CH_3)$ | X/Y-19 | 1,2-$(CH_3)_2$ |
| $CH_2$ | H | $CH(CH_3)$ | X/Y-19 | 1-$CH_3$, 2-Cyclopropyl |
| $CH(CH_3)$ | H | $CH(CH_3)$ | X/Y-19 | 1,2-$(CH_3)_2$ |
| $CH(CH_3)$ | H | $CH(CH_3)$ | X/Y-19 | 1-$CH_3$, 2-Cyclopropyl |
| $CH(CH_3)$ | 3-F | $CH_2$ | X/Y-19 | 1-$CH_3$, 2-Cyclopropyl |
| $CH_2CH_2$ | H | $CH_2$ | X/Y-19 | 1-$CH_3$, 2-Cyclopropyl |
| $CH_2CH_2$ | H | $CH_2CH_2$ | X/Y-19 | 1,2-$(CH_3)_2$ |
| $CH_2CH_2CH_2$ | H | $CH_2$ | X/Y-19 | 1-$CH_3$, 2-Cyclopropyl |
| $CH_2CH_2$ | H | $CH_2CH_2CH_2$ | X/Y-19 | 1,2-$(CH_3)_2$ |
| $CH_2CH(CH_3)$ | H | $CH_2CH_2$ | X/Y-19 | 1-$CH_3$, 2-Cyclopropyl |
| $CH_2CH_2$ | 3-F | $CH_2CH(CH_3)$ | X/Y-19 | 1,2-$(CH_3)_2$ |
| $CH_2$ | H | $CH_2$ | X/Y-16 | - |
| $CH_2$ | H | $CH_2$ | X/Y-16 | 2-$CH_3$ |
| $CH_2$ | H | $CH_2$ | X/Y-16 | 2-Cyclopropyl |
| $CH_2$ | H | $CH_2$ | X/Y-16 | 2-Cl |
| $CH_2$ | H | $CH_2$ | X/Y-16 | 2-$OCH_2CH_3$ |
| $CH(CH_3)$ | H | $CH_2$ | X/Y-16 | 2-$CH_3$ |
| $CH(CH_3)$ | H | $CH_2$ | X/Y-16 | 2-Cyclopropyl |
| $CH_2$ | H | $CH(CH_3)$ | X/Y-16 | 2-$CH_3$ |
| $CH_2$ | H | $CH(CH_3)$ | X/Y-16 | 2-Cyclopropyl |
| $CH_2(CH_3)$ | H | $CH(CH_3)$ | X/Y-16 | 2-$CH_3$ |
| $CH_2(CH_3)$ | H | $CH(CH_3)$ | X/Y-16 | 2-Cyclopropyl |
| $CH_2(CH_3)$ | 3-F | $CH(CH_3)$ | X/Y-16 | 2-Cyclopropyl |

Tabelle G (Forts.)

| A | $R^1$ | B | Y | Substituent an Y |
|---|---|---|---|---|
| $CH_2CH_2$ | H | $CH_2$ | X/Y-16 | 2-Cyclopropyl |
| $CH_2CH_2$ | H | $CH_2CH_2$ | X/Y-16 | 2-$CH_3$ |
| $CH_2CH_2CH_2$ | H | $CH_2$ | X/Y-16 | 2-Cyclopropyl |
| $CH_2CH_2$ | H | $CH_2CH_2CH_2$ | X/Y-16 | 2-$CH_3$ |
| $CH_2CH(CH_3)$ | H | $CH_2CH_2$ | X/Y-16 | 2-Cyclopropyl |
| $CH_2CH_2$ | 3-F | $CH_2CH(CH_3)$ | X/Y-16 | 2-$CH_3$ |
| $CH_2$ | H | $CH_2$ | X/Y-13 | - |
| $CH_2$ | H | $CH_2$ | X/Y-13 | 2-$CH_3$ |
| $CH_2$ | H | $CH_2$ | X/Y-13 | 2-Cyclopropyl |
| $CH_2$ | H | $CH_2$ | X/Y-13 | 2-$OCH_2CH_3$ |
| $CH(CH_3)$ | H | $CH_2$ | X/Y-13 | 2-$OCH_2CH_3$ |
| $CH(CH_3)$ | H | $CH_2$ | X/Y-13 | 2-Cyclopropyl |
| $CH_2$ | H | $CH(CH_3)$ | X/Y-13 | 2-$OCH_2CH_3$ |
| $CH_2$ | H | $CH(CH_3)$ | X/Y-13 | 2-Cyclopropyl |
| $CH(CH_3)$ | H | $CH(CH_3)$ | X/Y-13 | 2-$OCH_2CH_3$ |
| $CH(CH_3)$ | 3-F | $CH(CH_3)$ | X/Y-13 | 2-Cyclopropyl |
| $CH_2CH_2$ | H | $CH_2$ | X/Y-13 | 2-Cyclopropyl |
| $CH_2CH_2$ | H | $CH_2CH_2$ | X/Y-13 | 2-$CH_3$ |
| $CH_2CH_2CH_2$ | H | $CH_2$ | X/Y-13 | 2-Cyclopropyl |
| $CH_2CH_2$ | H | $CH_2CH_2CH_2$ | X/Y-13 | 2-$CH_3$ |
| $CH(CH_3)CH_2$ | 3-F | $CH_2CH_2$ | X/Y-13 | 2-Cyclopropyl |
| $CH_2CH_2$ | H | $CH(CH_3)CH_2$ | X/Y-13 | 2-$CH_3$ |
| $CH_2$ | H | $CH_2$ | X/Y-6 | - |
| $CH_2$ | H | $CH_2$ | X/Y-6 | 3-$CH_3$ |
| $CH_2$ | H | $CH_2$ | X/Y-6 | 3-Cyclopropyl |
| $CH_2$ | H | $CH_2$ | X/Y-6 | 3-$OCH_2CH_3$ |
| $CH(CH_3)$ | H | $CH(CH_3)$ | X/Y-6 | 3-Cyclopropyl |
| $CH(CH_3)$ | 3-F | $CH(CH_3)$ | X/Y-6 | 3-Cyclopropyl |
| $CH_2CH_2$ | H | $CH_2CH_2$ | X/Y-6 | 3-Cyclopropyl |
| $CH_2CH_2$ | H | $CH_2CH_2CH_2$ | X/Y-6 | 3-$CH_3$ |
| $CH(CH_3)CH_2$ | 3-F | $CH_2CH_2$ | X/Y-6 | 3-Cyclopropyl |
| $CH_2CH_2$ | H | $CH(CH_3)CH_2$ | X/Y-6 | 3-Cyclopropyl |
| $CH_2$ | H | $CH_2$ | X/Y-8 | 1-$CH_3$ |
| $CH_2$ | H | $CH_2$ | X/Y-8 | 1,3-$(CH_3)_2$ |
| $CH_2$ | H | $CH_2$ | X/Y-8 | 1,4-$(CH_3)_2$ |

Tabelle G (Forts.)

| A | $R^1$ | B | Y | Substituent an Y |
|---|---|---|---|---|
| $CH_2$ | H | $CH_2$ | X/Y-8 | 1-$CH_3$, 3-Cyclopropyl |
| $CH(CH_3)$ | H | $CH(CH_3)$ | X/Y-8 | 1-$CH_3$, 3-Cyclopropyl |
| $CH(CH_3)$ | 3-F | $CH(CH_3)$ | X/Y-8 | 1-$CH_3$, 3-Cyclopropyl |
| $CH_2CH_2$ | H | $CH_2CH_2$ | X/Y-8 | 1-$CH_3$, 3-Cyclopropyl |
| $CH_2CH_2$ | H | $CH_2CH_2CH_2$ | X/Y-8 | 1,3-$(CH_3)_2$ |
| $CH_2CH(CH_3)$ | H | $CH_2CH_2$ | X/Y-8 | 1-$CH_3$, 3-Cyclopropyl |
| $CH(CH_3)CH_2$ | 3-F | $CH_2CH_2$ | X/Y-8 | 1-$CH_3$, 3-Cyclopropyl |
| $CH_2$ | H | $CH_2$ | X/Y-10 | 1-$CH_3$ |
| $CH_2$ | H | $CH_2$ | X/Y-10 | 1,4-$(CH_3)_2$ |
| $CH_2$ | H | $CH_2$ | X/Y-10 | 1-$CH_3$, 4-$OCH_3$ |
| $CH_2$ | H | $CH_2$ | X/Y-10 | 1-$CH_3$, 5-$CH(CH_3)_2$ |
| $CH_2$ | H | $CH_2$ | X/Y-10 | 1-$CH_3$, 5-Cyclopropyl |
| $CH(CH_3)$ | H | $CH(CH_3)$ | X/Y-10 | 1-$CH_3$, 5-Cyclopropyl |
| $CH(CH_3)$ | 3-F | $CH(CH_3)$ | X/Y-10 | 1,4-$(CH_3)_2$ |
| $CH_2CH_2$ | H | $CH_2CH_2$ | X/Y-10 | 1-$CH_3$, 5-Cyclopropyl |
| $CH_2CH_2$ | H | $CH_2CH_2CH_2$ | X/Y-10 | 1,5-$(CH_3)_2$ |
| $CH_2CH(CH_3)$ | H | $CH_2CH_2$ | X/Y-10 | 1-$CH_3$, 5-Cyclopropyl |
| $CH(CH_3)CH_2$ | 3-F | $CH_2CH_2$ | X/Y-10 | 1-$CH_3$, 5-Cyclopropyl |
| $CH_2$ | H | $CH_2$ | X/Y-7 | - |
| $CH_2$ | H | $CH_2$ | X/Y-7 | 3-$CH_3$ |
| $CH(CH_3)$ | H | $CH(CH_3)$ | X/Y-7 | 3-$CH_3$ |
| $CH(CH_3)$ | 3-F | $CH(CH_3)$ | X/Y-7 | 3-$CH_3$ |
| $CH_2CH_2$ | H | $CH_2CH_2$ | X/Y-7 | 3-$CH_3$ |
| $CH_2CH_2$ | 3-F | $CH(CH_3)CH_2CH_2$ | X/Y-7 | 3-$CH_3$ |
| $CH_2$ | H | $CH_2$ | X/Y-9 | 1-$CH_3$ |
| $CH_2$ | H | $CH_2$ | X/Y-9 | 1,3-$(CH_3)_2$ |
| $CH_2$ | H | $CH_2$ | X/Y-9 | 1,5-$(CH_3)_2$ |
| $CH(CH_3)$ | H | $CH(CH_3)$ | X/Y-9 | 1,3-$(CH_3)_2$ |
| $CH(CH_3)$ | 3-F | $CH(CH_3)$ | X/Y-9 | 1,5-$(CH_3)_2$ |
| $CH_2CH_2$ | H | $CH_2CH_2$ | X/Y-9 | 1,3-$(CH_3)_2$ |
| $CH_2CH_2$ | H | $CH_2CH_2CH_2$ | X/Y-9 | 1,5-$(CH_3)_2$ |
| $CH_2CH(CH_3)$ | H | $CH_2CH_2$ | X/Y-9 | 1,3-$(CH_3)_2$ |
| $CH(CH_3)CH_2$ | 3-F | $CH_2CH_2$ | X/Y-9 | 1,5-$(CH_3)_2$ |
| $CH_2$ | H | $CH_2$ | X/Y-25 | 5-$CH_3$ |

Tabelle G (Forts.)

| A | $R^1$ | B | Y | Substituent an Y |
|---|---|---|---|---|
| $CH_2$ | H | $CH_2$ | X/Y-25 | $5-CH_2CH_3$ |
| $CH_2$ | H | $CH_2$ | X/Y-25 | $5-CH(CH_3)_2$ |
| $CH_2$ | H | $CH_2$ | X/Y-25 | $5-OCH_3$ |
| $CH(CH_3)$ | H | $CH(CH_3)$ | X/Y-25 | 5-Cyclopropyl |
| $CH_2(CH_3)$ | H | $CH(CH_3)$ | X/Y-25 | $5-CH_3$ |
| $CH(CH_3)$ | H | $CH(CH_3)$ | X/Y-25 | $5-OCH_2CH_3$ |
| $CH(CH_3)$ | 3-F | $CH(CH_3)$ | X/Y-25 | 5-Cyclopropyl |
| $CH_2CH_2$ | H | $CH_2$ | X/Y-25 | 5-Cyclopropyl |
| $CH_2CH_2$ | H | $CH_2CH_2$ | X/Y-25 | $5-CH_3$ |
| $CH_2CH_2CH_2$ | H | $CH_2$ | X/Y-25 | $5-OCH_2CH_3$ |
| $CH_2CH_2$ | 3-F | $CH_2CH(CH_3)$ | X/Y-25 | 5-Cyclopropyl |
| $CH_2$ | H | $CH(CH_3)$ | X/Y-25 | 5-Cyclopropyl |
| $CH_2$ | H | $CH_2$ | X/Y-23 | – |
| $CH_2$ | H | $CH_2$ | X/Y-23 | $5-CH_3$ |
| $CH_2$ | H | $CH_2$ | X/Y-23 | $5-CH(CH_3)_2$ |
| $CH_2$ | H | $CH_2$ | X/Y-23 | 5-Cl |
| $CH_2$ | H | $CH_2$ | X/Y-23 | $5-OCH_2CH_3$ |
| $CH_2$ | H | $CH(CH_3)$ | X/Y-23 | 5-Cyclopropyl |
| $CH_2$ | 3-F | $CH(CH_3)$ | X/Y-23 | $5-CH_3$ |
| $CH_2$ | H | $CH(CH_3)$ | X/Y-23 | $5-CH(CH_3)_2$ |
| $CH(CH_3)$ | H | $CH(CH_3)$ | X/Y-23 | 5-Cyclopropyl |
| $CH_2$ | H | $CH_2CH_2$ | X/Y-23 | 5-Cyclopropyl |
| $CH_2CH_2CH_2$ | H | $CH_2$ | X/Y-23 | $5-CH_3$ |
| $CH_2CH_2$ | H | $CH_2CH_2$ | X/Y-23 | $5-CH(CH_3)_2$ |
| $CH_2CH_2$ | H | $CH_2CH_2$ | X/Y-23 | 5-Cyclopropyl |
| $CH_2$ | H | $CH_2$ | X/Y-26 | $1-CH_3$ |
| $CH_2$ | H | $CH_2$ | X/Y-26 | $1,5-(CH_3)_2$ |
| $CH_2$ | H | $CH_2$ | X/Y-26 | $1-CH_3$, 5-Cyclopropyl |
| $CH(CH_3)$ | H | $CH(CH_3)$ | X/Y-26 | $1-CH_3$, 5-Cyclopropyl |
| $CH(CH_3)$ | H | $CH(CH_3)$ | X/Y-26 | $1,5-(CH_3)_2$ |
| $CH(CH_3)$ | 3-F | $CH_2$ | X/Y-26 | $1-CH_3$, 5-Cyclopropyl |
| $CH_2CH_2$ | H | $CH_2CH_2$ | X/Y-26 | $1-CH_3$, 5-Cyclopropyl |
| $CH_2$ | H | $CH_2CH_2$ | X/Y-26 | $1,5-(CH_3)_2$ |
| $CH_2$ | H | $CH_2$ | X/Y-28 | $1-CH_3$ |
| $CH_2$ | H | $CH_2$ | X/Y-28 | $1,5-(CH_3)_2$ |
| $CH_2$ | H | $CH_2$ | X/Y-28 | $1-CH_3$, 5-Cyclopropyl |

Tabelle G (Forts.)

| A | $R^1$ | B | Y | Substituent an Y |
|---|---|---|---|---|
| $CH(CH_3)$ | H | $CH(CH_3)$ | X/Y-28 | 1-$CH_3$, 5-Cyclopropyl |
| $CH(CH_3)$ | H | $CH(CH_3)$ | X/Y-28 | 1,5-$(CH_3)_2$ |
| $CH(CH_3)$ | 3-F | $CH_2$ | X/Y-28 | 1-$CH_3$, 5-Cyclopropyl |
| $CH_2CH_2$ | H | $CH_2CH_2$ | X/Y-28 | 1-$CH_3$, 5-Cyclopropyl |
| $CH_2$ | H | $CH_2CH_2$ | X/Y-28 | 1,5-$(CH_3)_2$ |
| $CH_2$ | H | $CH_2$ | X/Y-22 | - |
| $CH_2$ | H | $CH_2$ | X/Y-22 | 3-$CH_3$ |
| $CH_2$ | H | $CH_2$ | X/Y-22 | 3-Cyclopropyl |
| $CH_2$ | H | $CH_2$ | X/Y-22 | 3-$OCH_3$ |
| $CH(CH_3)$ | H | $CH_2$ | X/Y-22 | 3-$CH_3$ |
| $CH_2$ | H | $CH(CH_3)$ | X/Y-22 | 3-Cyclopropyl |
| $CH_2$ | 3-F | $CH(CH_3)$ | X/Y-22 | 3-$OCH_3$ |
| $CH_2CH_2$ | H | $CH_2CH_2$ | X/Y-22 | 3-Cyclopropyl |
| $CH_2$ | H | $CH_2CH_2$ | X/Y-22 | 3-$CH_3$ |
| $CH_2$ | H | $CH_2$ | X/Y-29 | 1-$CH_3$ |
| $CH_2$ | H | $CH_2$ | X/Y-29 | 1,3-$(CH_3)_2$ |
| $CH_2$ | H | $CH_2$ | X/Y-29 | 1-$CH_3$, 3-Cyclopropyl |
| $CH(CH_3)$ | H | $CH(CH_3)$ | X/Y-29 | 1,3-$(CH_3)_2$ |
| $CH(CH_3)$ | 3-F | $CH(CH_3)$ | X/Y-29 | 1-$CH_3$, 3-Cyclopropyl |
| $CH_2CH_2$ | H | $CH_2CH_2CH_2$ | X/Y-29 | 1,3-$(CH_3)_2$ |
| $CH_2CH_2$ | H | $CH_2CH_2$ | X/Y-29 | 1-$CH_3$, 3-Cyclopropyl |
| $CH_2$ | H | $CH_2$ | X/Y-17 | - |
| $CH_2$ | H | $CH_2$ | X/Y-17 | 5-Cyclopropyl |
| $CH_2$ | H | $CH_2$ | X/Y-17 | 5-$CH_3$ |
| $CH_2$ | H | $CH_2$ | X/Y-17 | 5-$OCH_2CH_3$ |
| $CH_2$ | H | $CH_2$ | X/Y-17 | 5-Cl |
| $CH_2$ | H | $CH_2$ | X/Y-17 | 4-$CH_3$ |
| $CH_2$ | H | $CH_2$ | X/Y-17 | 4-Cyclopropyl |
| $CH_2$ | H | $CH_2$ | X/Y-17 | 4-$OCH_2CH_3$ |
| $CH_2$ | H | $CH_2$ | X/Y-17 | 4-Cl |
| $CH(CH_3)$ | H | $CH(CH_3)$ | X/Y-17 | 5-Cyclopropyl |
| $CH(CH_3)$ | H | $CH(CH_3)$ | X/Y-17 | 4-Cyclopropyl |
| $CH(CH_3)$ | 3-F | $CH(CH_3)$ | X/Y-17 | 5-$OCH_2CH_3$ |
| $CH_2CH_2$ | H | $CH_2CH_2$ | X/Y-17 | 5-Cyclopropyl |
| $CH_2CH_2$ | H | $CH_2CH_2$ | X/Y-17 | 4-Cyclopropyl |

Tabelle G (Forts.)

| A | $R^1$ | B | Y | Substituent an Y |
|---|---|---|---|---|
| $CH_2CH_2$ | H | $CH_2CH_2CH_2$ | X/Y-17 | 5-$CH_3$ |
| $CH_2$ | H | $CH_2$ | X/Y-14 | – |
| $CH_2$ | H | $CH_2$ | X/Y-14 | 5-$CH_3$ |
| $CH_2$ | H | $CH_2$ | X/Y-14 | 5-Cyclopropyl |
| $CH_2$ | H | $CH_2$ | X/Y-14 | 4-$CH_3$ |
| $CH_2$ | H | $CH_2$ | X/Y-14 | 4-Cyclopropyl |
| $CH_2$ | H | $CH_2$ | X/Y-14 | 4-$OCH_2CH_3$ |
| $CH_2$ | H | $CH_2$ | X/Y-14 | 5-$OCH_2CH_3$ |
| $CH_2$ | H | $CH_2$ | X/Y-14 | 5-Cl |
| $CH(CH_3)$ | H | $CH(CH_3)$ | X/Y-14 | 4-Cyclopropyl |
| $CH(CH_3)$ | H | $CH(CH_3)$ | X/Y-14 | 5-Cyclopropyl |
| $CH(CH_3)$ | H | $CH(CH_3)$ | X/Y-14 | 4-$CH_3$ |
| $CH(CH_3)$ | 3-F | $CH(CH_3)$ | X/Y-14 | 4-Cyclopropyl |
| $CH_2$ | 3-F | $CH_2$ | X/Y-14 | 5-$OCH_2CH_3$ |
| $CH_2CH_2$ | H | $CH_2CH_2$ | X/Y-14 | 4-Cyclopropyl |
| $CH_2CH_2$ | H | $CH_2CH_2$ | X/Y-14 | 5-Cyclopropyl |
| $CH_2CH_2$ | H | $CH_2CH_2CH_2$ | X/Y-14 | 5-Cl |
| $CH_2CH_2$ | H | $CH_2CH_2$ | X/Y-14 | 4-$CH_3$ |
| $CH_2$ | H | $CH_2$ | X/Y-20 | 1-$CH_3$ |
| $CH_2$ | H | $CH_2$ | X/Y-20 | 1,5-$(CH_3)_2$ |
| $CH(CH_3)$ | H | $CH(CH_3)$ | X/Y-20 | 1-$CH_3$ |
| $CH(CH_3)$ | 3-F | $CH(CH_3)$ | X/Y-20 | 1,5-$(CH_3)_2$ |
| $CH_2CH_2$ | H | $CH_2CH_2$ | X/Y-20 | 1-$CH_3$ |
| $CH_2CH_2$ | H | $CH_2CH_2$ | X/Y-20 | 1,5-$(CH_3)_2$ |
| $CH(CH_3)CH_2$ | 3-F | $CH(CH_3)CH_2$ | X/Y-20 | 1,5-$(CH_3)_2$ |
| $CH_2$ | H | $CH_2$ | X/Y-3 | – |
| $CH_2$ | H | $CH_2$ | X/Y-3 | 3-$CH_3$ |
| $CH_2$ | H | $CH_2$ | X/Y-3 | 3-$CH_2CH_3$ |
| $CH_2$ | H | $CH_2$ | X/Y-3 | 3-$CH(CH_3)_2$ |
| $CH_2$ | H | $CH_2$ | X/Y-3 | 3-$OCH_2CH_3$ |
| $CH_2$ | H | $CH_2$ | X/Y-3 | 3-$CH_2OCH_3$ |
| $CH_2$ | H | $CH_2$ | X/Y-3 | 3-$CF_3$ |
| $CH(CH_3)$ | H | $CH_2$ | X/Y-3 | 3-$CH_3$ |
| $CH(CH_3)$ | H | $CH_2$ | X/Y-3 | 3-$CH(CH_3)_2$ |

Tabelle G (Forts.)

| A | $R^1$ | B | Y | Substituent an Y |
|---|---|---|---|---|
| $CH(CH_3)$ | H | $CH_2$ | X/Y-3 | 3-Cyclopropyl |
| $CH_2$ | H | $CH(CH_3)$ | X/Y-3 | 3-$CH_3$ |
| $CH_2$ | H | $CH(CH_3)$ | X/Y-3 | 3-$CH(CH_3)_2$ |
| $CH_2$ | H | $CH(CH_3)$ | X/Y-3 | 3-Cyclopropyl |
| $CH(CH_3)$ | H | $CH(CH_3)$ | X/Y-3 | 3-$CH_3$ |
| $CH(CH_3)$ | 3-F | $CH(CH_3)$ | X/Y-3 | 3-$CH(CH_3)_2$ |
| $CH(CH_3)$ | H | $CH(CH_3)$ | X/Y-3 | 3-Cyclopropyl |
| $CH_2$ | H | $CH_2CH_2$ | X/Y-3 | 3-$CH_3$ |
| $CH_2CH_2$ | H | $CH_2CH_2CH_2$ | X/Y-3 | 3-$CH(CH_3)_2$ |
| $CH_2$ | H | $CH_2CH_2$ | X/Y-3 | 3-Cyclopropyl |
| $CH_2CH_2$ | H | $CH_2CH(CH_3)$ | X/Y-3 | 3-Cyclopropyl |
| $CH_2CH_2$ | H | $CH_2CH(CH_3)$ | X/Y-3 | 3-$CH(CH_3)_2$ |
| $CH_2CH_2CH_2$ | 3-F | $CH(CH_3)CH_2$ | X/Y-3 | 3-$CH_3$ |
| $CH_2$ | H | $CH_2$ | X/Y-5 | – |
| $CH_2$ | H | $CH_2$ | X/Y-5 | 5-$OCH_2CH_3$ |
| $CH_2$ | H | $CH_2$ | X/Y-5 | 5-Cyclopropyl |
| $CH_2$ | H | $CH_2$ | X/Y-5 | 5-$CH_3$ |
| $CH(CH_3)$ | 3-F | $CH(CH_3)$ | X/Y-5 | 5-$CH_3$ |
| $CH_2$ | H | $CH(CH_3)$ | X/Y-5 | 5-Cyclopropyl |
| $CH(CH_3)$ | H | $CH_2$ | X/Y-5 | 5-Cyclopropyl |
| $CH_2$ | H | $CH_2CH_2$ | X/Y-5 | 5-$OCH_2CH_3$ |
| $CH_2CH_2$ | H | $CH_2CH_2CH_2$ | X/Y-5 | 5-$CH_3$ |
| $CH_2$ | H | $CH_2$ | X/Y-4 | – |
| $CH_2$ | H | $CH_2$ | X/Y-4 | 3-$OCH_2CH_3$ |
| $CH_2$ | H | $CH_2$ | X/Y-4 | 5-$OCH_2CH_3$ |
| $CH_2$ | H | $CH_2$ | X/Y-4 | 3-$CH(CH_3)_2$ |
| $CH_2$ | H | $CH_2$ | X/Y-4 | 5-$CH_3$ |
| $CH(CH_3)$ | 3-F | $CH(CH_3)$ | X/Y-4 | 3-$CH(CH_3)_2$ |
| $CH_2$ | H | $CH_2$ | X/Y-11 | – |
| $CH_2$ | H | $CH_2$ | X/Y-11 | 3-$CH_3$ |
| $CH_2$ | H | $CH_2$ | X/Y-11 | 5-$CH_3$ |
| $CH_2$ | H | $CH(CH_3)$ | X/Y-11 | 3-$CH_3$ |
| $CH_2$ | 3-F | $CH(CH_3)$ | X/Y-11 | 5-$CH_3$ |
| $CH(CH_3)$ | H | $CH(CH_3)$ | X/Y-11 | 5-$CH_3$ |
| $CH_2$ | H | $CH_2$ | X/Y-21 | – |

Tabelle G (Forts.)

| A | $R^1$ | B | Y | Substituent an Y |
|---|---|---|---|---|
| $CH_2$ | H | $CH_2$ | X/Y-21 | $4,5-(CH_3)_2$ |
| $CH_2$ | H | $CH_2$ | X/Y-21 | $4,5-Br_2$ |
| $CH(CH_3)$ | H | $CH(CH_3)$ | X/Y-21 | $4,5-Cl_2$ |
| $CH(CH_3)$ | H | $CH(CH_3)$ | X/Y-21 | $4,5-(CH_3)_2$ |
| $CH(CH_3)$ | H | $CH(CH_3)$ | X/Y-21 | $4,5-Br_2$ |
| $CH_2$ | H | $CH_2$ | X/Y-30 | - |
| $CH_2$ | H | $CH_2$ | X/Y-30 | $3,5-(CH_3)_2$ |
| $CH(CH_3)$ | H | $CH(CH_3)$ | X/Y-30 | - |
| $CH(CH_3)$ | H | $CH(CH_3)$ | X/Y-30 | $3,5-(CH_3)_2$ |
| $CH_2$ | H | $CH_2$ | X/Y-27 | - |
| $CH_2$ | H | $CH_2$ | X/Y-27 | 2-Cl |
| $CH_2$ | H | $CH_2$ | X/Y-27 | $2,5-(CH_3)_2$ |
| $CH(CH_3)$ | H | $CH(CH_3)$ | X/Y-27 | - |
| $CH(CH_3)$ | H | $CH(CH_3)$ | X/Y-27 | 2-Cl |
| $CH(CH_3)$ | H | $CH(CH_3)$ | X/Y-27 | $2,5-(CH_3)_2$ |

Die Hydrochinondiether der Formel I sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flamea, Pectinophora gossypiella, Peridroma saucia, Phalera bucephala, Phthorimaea operculella, Phyllocnistis citrella, Pieris brassicae, Plathypena, scarbra, Plutella xylostella, Pseudoplusia includens, Phyacionia frustrana, Scrobipalpula absoluta, Sitotroga cerelella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha

ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossia morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euchistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dyasphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus birittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amglyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobins megnini, Paratetranychus pilosus, Permanyssus gallinae, Phyllocaptrata oleivora, Polyphagotarsonemus latus, Psoroptes ovis, Rhipicephalus appendiculatus, Rhipicephalus evertsi, Saccoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycinae, Heterodera schatii, Hetrodera triflolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon,

Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin-Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.-%, vorzugsweise zwischen 0,1 und 90 Gew.-%, des Wirkstoffs. Die Wirkstoffe werden dabei in einer Reinheit von 90 % bis 100 %, vorzugsweise 95 % bis 100 % (nach NMR-Spektrum) eingesetzt.

Beispiele für Formulierungen sind:

I. 5 Gew.-Teile der Verbindung Nr. 1.010 werden mit 95 Gew.-Teilen feinteiligem Kaolin innig vermischt. Man erhält auf diese Weise ein Stäubemittel, das 5 Gew.-% des Wirkstoffs enthält.

II. 30 Gew.-Teile der Verbindung Nr. 1.001 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.-%).

III. 10 Gew.-Teile der Verbindung Nr. 1.013 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.-%).

IV. 20 Gew.-Teile der Verbindung Nr. 1.007 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.-%).

V. 80 Gew.-Teile der Verbindung Nr. 1.008 werden mit 3 Gew.-Teilen des Natriumsalzes der Diisobutylnaphthalin-alpha-sulfonsäure 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.-%).

VI. Man vermischt 90 Gew.-Teile der Verbindung Nr. 1.003 mit 10 Gew.-Teilen N-Methyl-α-pyrrolidon und erhält eine Lösung, die zur Anwendung in Form kleinster Tropfen geeignet ist (Wirkstoffgehalt 90 Gew.-%).

VII. 20 Gew.-Teile der Verbindung Nr. 1.005 werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100.000 Gew.-Teilen Wasser erhält man eine wäßrige Dispersion, die 0,02 Gew.-% des Wirkstoffs enthält.

VIII. 20 Gew.-Teile des Wirkstoffs Nr. 1.011 werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfitablauge und 60 Gew.-Teilen pulverförmigem Kieselsäuregel gut vermischt und in einer Hammermühle vermahlen. Durch feines Verteilen der Mischung in 20.000 Gew.-Teilen Wasser erhält man eine Spritzbrühe, die 0,1 Gew.-% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B.

Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff beträgt unter Freilandbedingungen 0,001 bis 10, vorzugsweise 0,1 bis 1 kg/ha.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Synthesebeispiele

Die in den nachstehenden Synthesebeispielen wiedergegebenen Arbeitsvorschriften wurden unter entsprechender Abwandlung der Ausgangsstoffe zur Herstellung weiterer Verbindungen I benutzt. Die so erhaltenen Verbindungen sind in den nachstehenden Tabellen mit physikalischen Angaben aufgeführt.

Herstellung von 1-[(5-Cyclopropyl-1,3,4-thiadiazol-2-yl)methoxy]-4-(4,5-dichlor-1-imidazolylmethoxy)-benzol

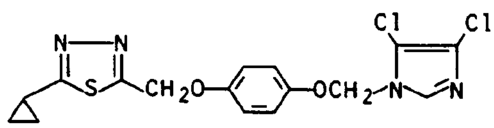

a) 4-tert.-Butoxy-1-[(5-cyclopropyl-1,3,4-thiadiazol-2-yl)methoxy]benzol

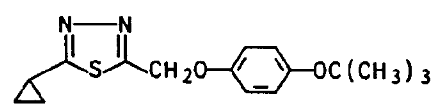

Zu 5,7 g 80 % Natriumhydrid (0,19 mol) in 37 $cm^3$ Dimethylformamid (DMF) werden 28,5 g (0,172 mol) 4-tert.-Butoxyphenol in 260 $cm^3$ DMF getropft. Nach zweistündigem Rühren bei 40 bis 50°C wird auf 25°C abgekühlt und mit 30 g 2-Chlormethyl-5-cyclopropylthiadiazol(1,3,4) in 250 $cm^3$ DMF versetzt. Nach 5-stündigem Rühren bei 80°C wird das Lösungsmittel entfernt, der Rückstand in Wasser aufgenommen und mit Essigsäureethylester extrahiert. Die organische Phase wird getrocknet und eingeengt.

Nach einer Flash-Chromatographie über Kieselgel mit Cyclohexan/Essigester als Fließmittel erhält man das gewünschte Produkt als weiße Kristalle. Fp.: 53-55°C; Ausbeute: 50,5 % der Theorie

b) 1-[(5-Cyclopropyl-1,3,4-thiadiazol-2-yl)methoxy]-4-hydroxybenzol

26,4 g des Produktes aus a) werden in 215 $cm^3$ Ethanol mit einigen Tropfen wäßriger Salzsäure versetzt und 8 Stunden am Rückfluß erhitzt. Nach dem Einengen erhält man das gewünschte Produkt in quantitativer Ausbeute als weißes Pulver, Fp.: 79-83°C.

c) 1-[(5-Cyclopropyl-1,3,4-thiadiazol-2-yl)methoxy]-4-(4,5-dichlor-1-imidazolylmethoxy)-benzol

Zu 0,63 g (0,021 mol) 80 %igem Natriumhydrid in 6 $cm^3$ Dimethylformamid (DMF) werden bei 25°C 4,7 g (0,0191 mol) des Produktes aus b) in 25 $cm^3$ DMF getropft. Die Mischung wird 30 Minuten bei 25°C gerührt und dann mit 3,54 g (0,0191 mol) 4,5-Dichlor-1-chlormethylimidazol in 10 $cm^3$ DMF versetzt. Nach 4-stündigem Erwärmen auf 80°C wird das Lösungsmittel entfernt, der Rückstand in Wasser aufgenommen und mit Essigsäureethylester extrahiert. Aus der organischen Phase erhält man nach Trocknen und Einengen das Rohprodukt. Nach einer Flash-Chromatographie über Kieselgel mit Cyclohexan/Essigester als Fließmittel erhält man das gewünschte Produkt als farblose Kristalle. Fp: 108-109°C; Ausbeute: 5,0 g; 66 % der Theorie

IR: (Fingerprintbereich; $cm^{-1}$) 1510, 1257, 1236, 1227, 1202, 1034, 825.

Tabelle 1

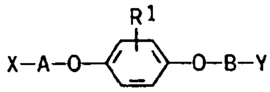 I

| Beispiel Nr. | X | A | $R^1$ | B | Y | phys. Daten [Fp(°C); NMR (δ in ppm)] |
|---|---|---|---|---|---|---|
| 1.001 | 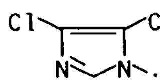 | $CH_2$ | H | $CH_2$ | 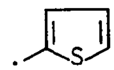 | 121 |
| 1.002 | 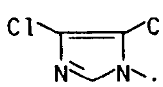 | $CH_2$ | H | $CH_2$ | 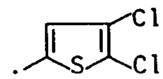 | 128 |
| 1.003 | 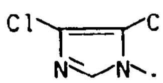 | $CH_2$ | H | $CH_2$ | 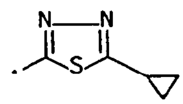 | 108-109 |
| 1.004 | 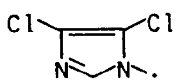 | $CH_2$ | H | $CH_2$ | 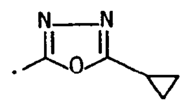 | 70-71 |
| 1.005 | 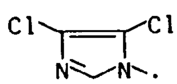 | $CH_2$ | H | $CH_2$ | 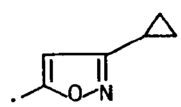 | 123 |
| 1.006 | 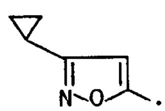 | $CH_2$ | H | $CH_2$ | 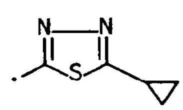 | 102 |
| 1.007 | 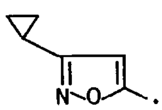 | $CH_2$ | H | $CH_2$ | 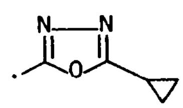 | 91 |
| 1.008 | 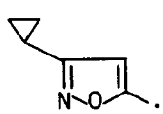 | $CH_2$ | H | $CH_2$ | 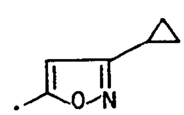 | 117 |
| 1.009 | 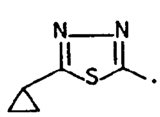 | $CH_2$ | H | $CH_2$ | 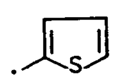 | 97-98 |

Tabelle 1 (Fortsetzung)

| Beispiel Nr. | X | A | $R^1$ | B | Y | phys. Daten [Fp(°C); NMR (δ in ppm)] |
|---|---|---|---|---|---|---|
| 1.010 | 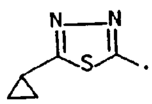 | $CH_2$ | H | $CH_2$ | 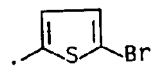 | 86-87 |
| 1.011 | 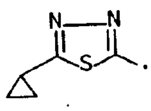 | $CH_2$ | H | $CH_2$ | 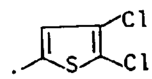 | 121 |
| 1.012 | 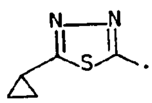 | $CH_2$ | H | $CH_2$ | 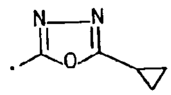 | 74 |
| 1.013 | 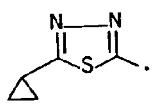 | $CH_2$ | H | $CH_2$ | 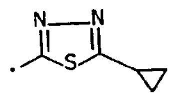 | 118-120 |
| 1.014 | 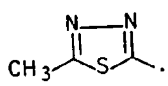 | $CH_2$ | H | $CH_2$ | 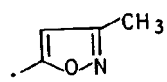 | 120-125 |
| 1.015 | 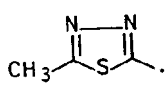 | $CH_2$ | H | $CH_2$ | 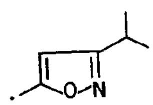 | 82- 83 |
| 1.016 | 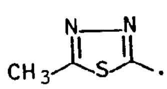 | $CH_2$ | H | $CH_2$ | 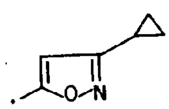 | 98-100 |
| 1.017 | 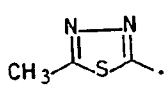 | $CH_2$ | H | $CH_2$ | 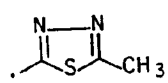 | 165-170 |
| 1.018 | 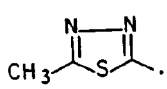 | $CH_2$ | H | $CH_2$ | 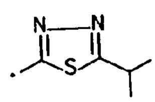 | 120-125 |
| 1.019 | 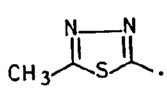 | $CH_2$ | H | $CH_2$ | 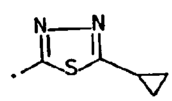 | 104-106 |
| 1.020 | 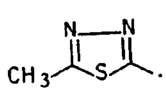 | $CH_2$ | H | $CH_2$ | 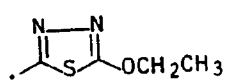 | 102-105 |

Tabelle 1 (Fortsetzung)

| Beispiel Nr. | X | A | $R^1$ | B | Y | phys. Daten [Fp(°C); NMR (δ in ppm)] |
|---|---|---|---|---|---|---|
| 1.021 | 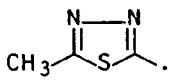 | $CH_2$ | H | $CH_2$ | 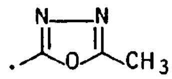 | 112-121 |
| 1.022 | 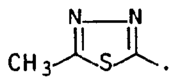 | $CH_2$ | H | $CH_2$ | 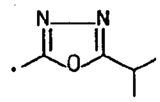 | 54- 58 |
| 1.023 | 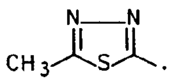 | $CH_2$ | H | $CH_2$ | 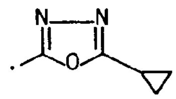 | 84- 87 |
| 1.024 | 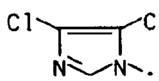 | $CH_2$ | H | $CH_2$ | 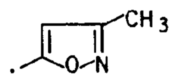 | 86- 90 |
| 1.025 | 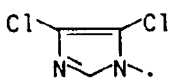 | $CH_2$ | H | $CH_2$ | 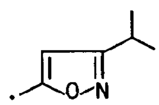 | 93- 95 |
| 1.026 | 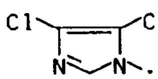 | $CH_2$ | H | $CH_2$ | 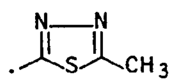 | 113-117 |
| 1.027 | 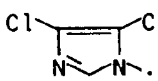 | $CH_2$ | H | $CH_2$ | 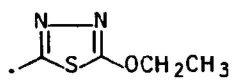 | 77- 79 |
| 1.028 | 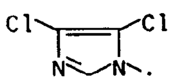 | $CH_2$ | H | $CH_2$ | 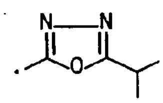 | A 5,17<br>B 5,70 |
| 1.029 | 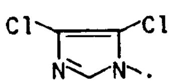 | $CH_2$ | H | $CH_2$ | 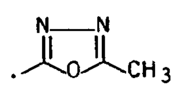 | A 5,17<br>B 5,67 |
| 1.030 | 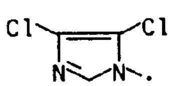 | $CH_2$ | H | $CH_2$ | 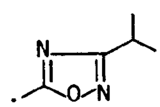 | 68- 72 |
| 1.031 | 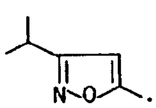 | $CH_2$ | H | $CH_2$ | 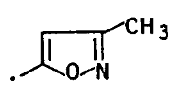 | 76- 77 |
| 1.032 | 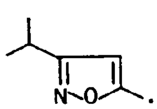 | $CH_2$ | H | $CH_2$ | 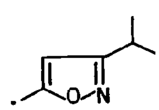 | 82- 83 |

Tabelle 1 (Fortsetzung)

| Beispiel Nr. | X | A | $R^1$ | B | Y | phys. Daten [Fp(°C); NMR (δ in ppm)] |
|---|---|---|---|---|---|---|
| 1.033 | 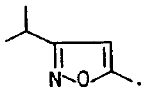 | $CH_2$ | H | $CH_2$ | 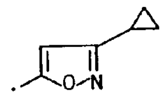 | 79- 85 |
| 1.034 | 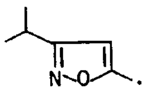 | $CH_2$ | H | $CH_2$ | 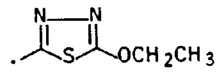 | 63- 66 |
| 1.035 | 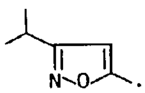 | $CH_2$ | H | $CH_2$ | 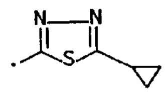 | 57- 60 |
| 1.036 | 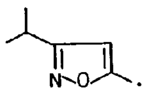 | $CH_2$ | H | $CH_2$ | 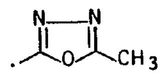 | 66- 69 |
| 1.037 | 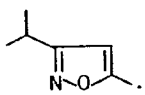 | $CH_2$ | H | $CH_2$ | 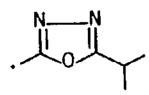 | 60- 65 |
| 1.038 | 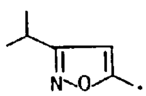 | $CH_2$ | H | $CH_2$ | 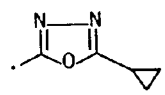 | 59- 60 |
| 1.039 | 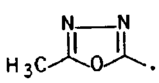 | $CH_2$ | H | $CH_2$ | 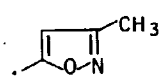 | 123-128 |
| 1.040 | 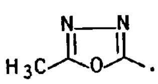 | $CH_2$ | H | $CH_2$ | 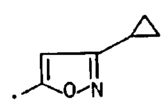 | 97-100 |
| 1.041 | 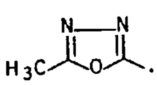 | $CH_2$ | H | $CH_2$ | 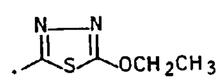 | 81- 85 |
| 1.042 | 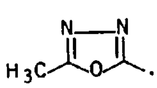 | $CH_2$ | H | $CH_2$ | 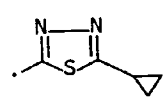 | 78- 80 |
| 1.043 | 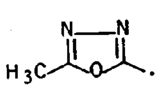 | $CH_2$ | H | $CH_2$ | 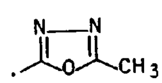 | 131-134 |

Tabelle 1 (Fortsetzung)

| Beispiel Nr. | X | A | $R^1$ | B | Y | phys. Daten [Fp(°C); NMR (δ in ppm)] |
|---|---|---|---|---|---|---|
| 1.044 | 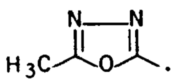 | $CH_2$ | H | $CH_2$ | 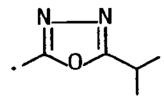 | 54- 61 |
| 1.045 | 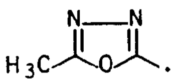 | $CH_2$ | H | $CH_2$ | 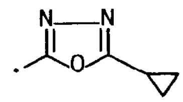 | 103-105 |
| 1.046 | 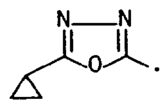 | $CH_2$ | H | $CH_2$ | 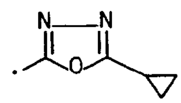 | 124-126 |
| 1.047 | 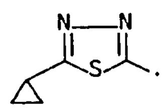 | $CH_2$ | H | $CH_2$ | 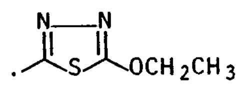 | 108-110 |

Anwendungsbeispiele

Die insektizide Wirkung der Verbindungen der allgemeinen Formel I ließ sich durch folgende Versuche zeigen: Die Wirkstoffe wurden

a) als 0,1 %-ige Lösung in Aceton oder

b) als 10 %-ige Emulsion in einem Gemisch aus 70 Gew.-% Cyclohexanol, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)

aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindungen im Vergleich zu unbehandelten Kontrollversuchen noch eine 80 - 100 %-ige Hemmung bzw. Mortalität hervorriefen (Wirkschwelle bzw. Minimalkonzentration).

A) Aphis fabae (Schwarze Laus), Kontaktwirkung

Stark befallene Buschbohnen (Vicia faba) wurden mit der wässrigen Wirkstoffaufbereitung behandelt.

Nach 24 h wurde die Mortalitätsrate bestimmt.

In diesem Test zeigten die getesteten Verbindungen Wirkschwellen von 200 bis 400 ppm.

B) Dysdercus intermedius (Baumwollwanze), Ovizidtest

Normierte Klebestreifen wurden mit den Eiern von Dysdercus intermedius belegt und anschließend mit der wäßrigen Wirkstoffaufbereitung benetzt.

Nach dem Schlüpfen der Larven in einem Kontrollexperiment (ca. 8 Tage) wurde die Mortalität bestimmt.

In diesem Test zeigten die getesteten Verbindungen Wirkschwellen von 2 bis 1000 ppm.

C) Prodenia litura (Ägypt. Baumwollwurm), Zuchtversuch

Fünf Raupen des Entwicklungsstadiums L3 (10 - 12 mm), die keine feststellbare Schädigung im Kontaktversuch erlitten hatten, wurden auf Standardnährboden aufgebracht, der zuvor mit der wässrigen Wirkstoffaufbereitung benetzt worden war.

Die Beobachtung erstrekte sich bis zum Schlüpfen der Falter in einem Kontrollversuch ohne Wirkstoff.

In diesem Test zeigten die getesteten Verbindungen Wirkschwellen von 0,4 bis 0,000002 ppm.

## Patentansprüche

1. Hydrochinondiether der allgemeinen Formel I

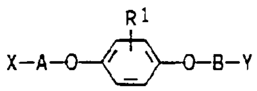

I,

in der die Substituenten folgende Bedeutung haben:

$R^1$ Wasserstoff, Halogen oder $C_1$-$C_6$-Alkyl;

A,B Methylen, Ethylen oder Propylen, wobei diese Gruppen ein oder zwei $C_1$-$C_3$-Alkylreste tragen können;

X,Y ein 5-gliedriger Heteroaromat, enthaltend neben Kohlenstoffatomen 1 bis 3 Heteroatome aus der Gruppe Stickstoff, Sauerstoff und Schwefel, welcher eine bis drei der folgenden Gruppen tragen kann: Halogen, $C_1$-$C_6$-Alkyl, $C_1$-$C_4$-Halogenalkyl, $C_1$-$C_6$-Alkoxy, $C_1$-$C_4$-Halogenalkoxy, $C_1$-$C_3$-Alkoxy-$C_1$-$C_4$-alkyl, $C_3$-$C_8$-Cycloalkyl, $C_2$-$C_8$-Alkenyl, Aryl oder Aryl-$C_1$-$C_{10}$-alkyl, wobei diese aromatischen Reste ihrerseits ein bis fünf Halogenatome und ein bis drei der folgenden Gruppen tragen können: $C_1$-$C_4$-Alkyl, $C_1$-$C_4$-Halogenalkyl, $C_1$-$C_4$-Alkoxy, $C_1$-$C_4$-Halogenalkoxy oder $C_1$-$C_4$-Alkylthio,

wobei X und Y nicht gleichzeitig für 3-Phenylisoxazol-5-yl stehen, wenn A und B Methylen und $R^1$ Wasserstoff bedeuten und wobei X und Y nicht gleichzeitig für 3,5-Diethylpyrazol-4-yl oder 1-Methyl-3,5-diethylpyrazol-4-yl stehen, wenn A und B Propylen und $R^1$ Wasserstoff bedeuten.

2. Verfahren zur Herstellung der Verbindungen I gemäß Anspruch 1, dadurch gekennzeichnet, daß man einen 4-Hydroxyphenolether der allgemeinen Formel II

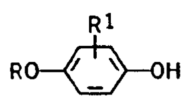

II,

in der R eine inerte Schutzgruppe bedeutet, in an sich bekannter Weise in einem inerten organischen Lösungsmittel in Gegenwart einer Base mit einem Hetarylalkylderivat der Formel IIIa oder IIIb

| | |
|---|---|
| Z-A-X | Z-B-Y |
| IIIa | IIIb |

in der Z eine nucleofuge Abgangsgruppe bedeutet, verethert, das so erhaltene Dietherderivat IVa bzw. IVb

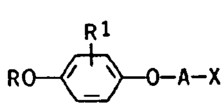

IVa

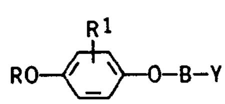

IVb

in an sich bekannter Weise durch Abspaltung der Schutzgruppe R in den entsprechenden Monoether Va bzw. Vb

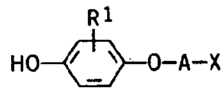

Va

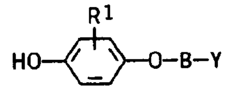

Vb

überführt, welcher anschließend in an sich bekannter Weise mit einem Hetarylalkylderivat der Formel IIIa oder IIIb zu I verethert wird.

3. Schädlingsbekämpfungsmittel enthaltend einen Hydrochinondiether der allgemeinen Formel I gemäß Anspruch 1.

4. Schädlingsbekämpfungsmittel enthaltend einen Hydrochinondiether der allgemeinen Formel I gemäß Anspruch 1 und inerte Zusatzstoffe.

5. Verfahren zur Bekämpfung von Schädlingen, dadurch gekennzeichnet, daß man eine wirksame Menge eines Hydrochinondiethers der Formel IA

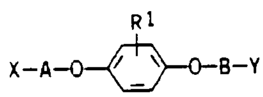

**IA**

in der die Substituenten folgende Bedeutung haben:

$R^1$ Wasserstoff, Halogen oder $C_1$-$C_6$-Alkyl;

A,B Methylen, Ethylen oder Propylen, wobei diese Gruppen ein oder zwei $C_1$-$C_3$-Alkylreste tragen können;

X,Y ein 5-gliedriger Heteroaromat, enthaltend neben Kohlenstoffatomen 1 bis 3 Heteroatome aus der Gruppe Stickstoff, Sauerstoff und Schwefel, welcher eine bis drei der folgenden Gruppen tragen kann: Halogen, $C_1$-$C_6$-Alkyl, $C_1$-$C_4$-Halogenalkyl, $C_1$-$C_6$-Alkoxy, $C_1$-$C_4$-Halogenalkoxy, $C_1$-$C_3$-Alkoxy-$C_1$-$C_4$-alkyl, $C_3$-$C_8$-Cycloalkyl, $C_2$-$C_8$-Alkenyl, Aryl oder Aryl-$C_1$-$C_{10}$-alkyl, wobei diese aromatischen Reste ihrerseits ein bis fünf Halogenatome und ein bis drei der folgenden Gruppen tragen können: $C_1$-$C_4$-Alkyl, $C_1$-$C_4$-Halogenalkyl, $C_1$-$C_4$-Alkoxy, $C_1$-$C_4$-Halogenalkoxy oder $C_1$-$C_4$-Alkylthio,

auf die Schädlinge und/oder deren Lebensraum einwirken läßt.

## Claims

1. A hydroquinone diether of the formula I

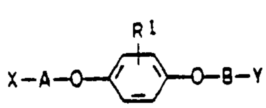

**I,**

where

$R^1$ is hydrogen, halogen or $C_1$-$C_6$-alkyl,

A and B are each methylene, ethylene or propylene, where these groups may carry one or two $C_1$-$C_3$-alkyl radicals, and

X and Y are each a 5-membered heteroaromatic structure containing, in addition to carbon atoms, from 1 to 3 hetero atoms from the group consisting of nitrogen, oxygen and sulfur, which may carry from one to three of the following groups: halogen, $C_1$-$C_6$-alkyl, $C_1$-$C_4$-haloalkyl, $C_1$-$C_6$-alkoxy, $C_1$-$C_4$-haloalkoxy, $C_1$-$C_3$-alkoxy-$C_1$-$C_4$- alkyl, $C_3$-$C_8$-cycloalkyl, $C_2$-$C_8$-alkenyl, aryl or aryl-$C_1$-$C_{10}$-alkyl, where these aromatic radicals in turn may carry from one to five halogen atoms and from one to three of the following groups: $C_1$-$C_4$-alkyl, $C_1$-$C_4$-haloalkyl, $C_1$-$C_4$-alkoxy, $C_1$-$C_4$-haloalkoxy or $C_1$-$C_4$-alkylthio, where X and Y are not simultaneously 3-phenylisoxazol-5-yl when A and B are each methylene and $R^1$ is hydrogen, and where X and Y are not simultaneously 3,5-diethylpyrazol-4-yl or 1-methyl-3,5-diethylpyrazol-4-yl when A and B are each propylene and $R^1$ is hydrogen.

2. A process for the preparation of the compound I as claimed in claim 1, wherein 4-hydroxyphenol ether of the formula II

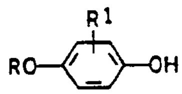

II,

where R is an inert protective group, is etherified with a hetarylalkyl derivative of the formula IIIa or IIIb

Z-A-X

IIIa

Z-B-Y

IIIb

where Z is a nucleofugic leaving group, in a conventional manner in an inert organic solvent in the presence of a base and the resulting diether derivative IVa or IVb

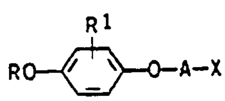

IVa

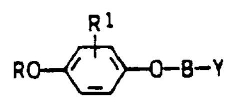

IVb

is converted in a conventional manner, by eliminating the protective group R, into the corresponding monoether Va or Vb

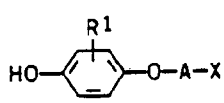

Va

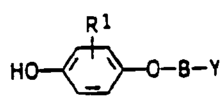

Vb

which is then etherified in a conventional manner with a hetarylalkyl derivative of the formula IIIa or IIIb to give I.

3. A pesticide containing a hydroquinone diether of the formula I as claimed in claim 1.

4. A pesticide containing a hydroquinone diether of the formula I as claimed in claim 1 and inert additives.

5. A method for controlling pests, wherein an effective amount of a hydroquinone diether of the formula IA

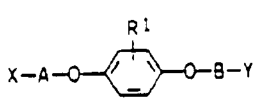

IA

where
$R^1$ is hydrogen, halogen or $C_1$-$C_6$-alkyl,
A and B are each methylene, ethylene or propylene, where these groups may carry one or two $C_1$-$C_3$-alkyl radicals, and
X and Y are each a 5-membered heteroaromatic structure containing, in addition to carbon atoms, from 1 to 3 hetero atoms from the group consisting of nitrogen, oxygen and sulfur, which may carry from one

to three of the following groups: halogen, $C_1$-$C_6$-alkyl, $C_1$-$C_4$-haloalkyl, $C_1$-$C_6$-alkoxy, $C_1$-$C_4$-haloalkoxy, $C_1$-$C_3$-alkoxy-$C_1$-$C_4$-alkyl, $C_3$-$C_8$-cycloalkyl, $C_2$-$C_8$-alkenyl, aryl or aryl-$C_1$-$C_{10}$-alkyl, where these aromatic radicals in turn may carry from one to five halogen atoms and from one to three of the following groups: $C_1$-$C_4$-alkyl, $C_1$-$C_4$-haloalkyl, $C_1$-$C_4$-alkoxy, $C_1$-$C_4$-haloalkoxy or $C_1$-$C_4$-alkylthio, is allowed to act on the pests and/or their habitat.

## Revendications

1. Diether hydroquinonique de la formule générale I

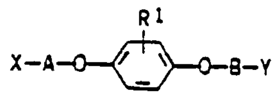

I,

dans laquelle les substituants ont la signification suivante :

$R^1$ hydrogène, halogène ou alkyle en C1-C6;

A,B méthylène, éthylène ou propylène, ces groupes pouvant porter un ou deux restes alkyle en C1-C3;

X,Y un hétéroaromate à 5 chaînons, contenant à côté des atomes de carbone, 1 à 3 hétéroatomes du groupe azote, oxygène et soufre qui peut porter un à trois des groupes suivants : halogène, alkyle en C1-C6, halogenalkyle en C1-C4, alcoxy en C1-C6, halogenalcoxy en C1-C4, alcoxy en C1-C3-alkyle en C1-C4, cycloalkyle en C3-C8, alcényle en C2-C8, aryle ou aryle-alkyle en C1-C10, ces restes aromatiques de leur côté, pouvant porter un à cinq atomes d'halogène et un à trois des groupes suivants : alkyle en C1-C4, halogenalkyle en C1-C4, alcoxy en C1-C4, halogenalcoxy en C1-C4 ou alkylthio en C1-C4;

X et Y n'étant pas mis simultanément pour 3-phénylisoxazol-5-yle quand A et B représentent méthylène et $R^1$ hydrogène, et X et Y n'étant pas mis simultanément pour 3,5-diéthylpyrazol-4-yle ou 1-méthyl-3,5-diéthylpyrazol-4-yle quand A et B représentent propylène et $R^1$ hydrogène.

2. Procédé de préparation des composés I selon la revendication 1, caractérisé par le fait qu'on esterifie, de manière connue en soi, dans un solvant organique inerte, en présence d'une base, un 4-hydroxyphénoléther de la formule générale II

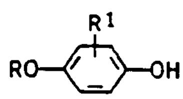

II,

dans laquelle R représente un groupe protecteur inerte, avec un dérivé hétarylalkyle de la formule IIIa ou IIIb

| | |
|---|---|
| Z-A-X | Z-B-Y |
| IIIa | IIIb |

ou Z représente un groupe éliminable nucléofuge, on transforme le dérivé de diéther IVa ou IVb ainsi obtenu

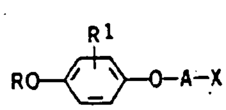

IVa

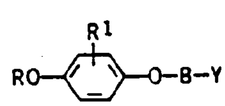

IVb

de manière connue en soi, par séparation du groupe protecteur R, en le monoéther correspondant Va ou Vb

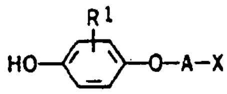

Va

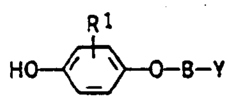

Vb

qui est ensuite estérifié en I de manière connue en soi, avec un dérivé d'hétarylalkyle de formule IIIa ou IIIb.

**3.** Agent de lutte contre les parasites, contenant un diéther hydroquinonique de la formule générale I, selon la revendication 1.

**4.** Agent de lutte contre les parasites, contenant un diéther hydroquinonique de la formule générale I et des additifs inertes.

**5.** Procédé de lutte contre les parasites, caractérisé par le fait qu'on fait agir sur les parasites ou leur biotope une quantité efficace d'un diéther hydroquinonique de la formule Ia

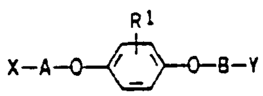

IA

dans laquelle les substituants ont la signification suivante :
$R^1$ hydrogène, halogène ou alkyle en C1-C6;
A,B méthylène, éthylène ou propylène, ces groupes pouvant porter un ou deux restes alkyle en C1-C3;
X,Y un hétéroaromate à 5 chaînons, contenant à côté des atomes de carbone, 1 à 3 hétéroatomes du groupe azote, oxygène et soufre qui peut porter un à trois des groupes suivants : halogène, alkyle en C1-C6, halogenalkyle en C1-C4, alcoxy en C1-C6, halogenalcoxy en C1-C4, alcoxy en C1-C3-alkyle en C1-C4, cycloalkyle en C3-C8, alcényle en C2-C8, aryle ou aryle-alkyle en C1-C10, ces restes aromatiques de leur côté, pouvant porter un à cinq atomes d'halogène et un à trois des groupes suivants : alkyle en C1-C4, halogenalkyle en C1-C4, alcoxy en C1-C4, halogenalcoxy en C1-C4 ou alkylthio en C1-C4.